(19) Europäisches Patentamt
European Patent Office
Office européen des brevets

(11) Numéro de publication: **0 114 128**
**B1**

(12) # FASCICULE DE BREVET EUROPEEN

(45) Date de publication du fascicule du brevet: **22.03.89**

(51) Int. Cl.⁴: **C 07 D 417/14**, C 07 D 403/04, A 61 K 31/425 // C07D205/08

(21) Numéro de dépôt: **84400023.2**

(22) Date de dépôt: **06.01.84**

(54) **Nouveaux dérivés de la 3-amino 2-oxoazétidine comportant en position 1, un radical hétérocyclique azoté, leur procédé de préparation, leur application comme médicaments et les produits intermédiaires nécessaires à leur préparation.**

(30) Priorité: **10.01.83 FR 8300273**

(43) Date de publication de la demande: **25.07.84 Bulletin 84/30**

(45) Mention de la délivrance du brevet: **22.03.89 Bulletin 89/12**

(84) Etats contractants désignés: **AT BE CH DE FR GB IT LI LU NL SE**

(56) Documents cités:
**EP-A- 0 029 183**
**EP-A- 0 053 816**
**EP-A- 0 068 466**
**NL-A- 7 804 148**

**Chemical Abstracts, vol. 89, 1978, 128843g**
**Tetrahedron, vol. 39, no. 15, p. 2600**

(73) Titulaire: **ROUSSEL-UCLAF, 35, boulevard des Invalides, F-75007 Paris (FR)**

(72) Inventeur: **Teutsch, Jean Georges, Résidence Lavoisier Bât. 3, F-93500 Pantin (FR)**
Inventeur: **Klich, Michel, 46-48 rue Victor Hugo, F-93500 Pantin (FR)**
Inventeur: **Chantot, Jean-François, 2, allée Eole, F-77410 Gressy-en-France (FR)**

(74) Mandataire: **Bourgouin, André et al, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 114 128 B1

**Description**

La présente invention concerne de nouveaux dérivés de la 3-amino 2-oxo azétidine comportant en position 1 un radical hétérocyclique azoté, leur procédé de préparation, leur application comme médicaments et les produits intermédiaires nécessaires à leur préparation.

L'invention a pour objet les produits de formule générale (I):

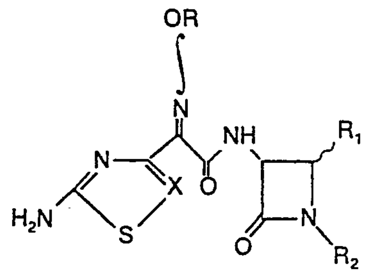

(I)

dans laquelle R représente un atome d'hydrogène, un radical alkyle, alkényle ou alkynyle ayant au plus 12 atomes de carbone, éventuellement substitué par un ou plusieurs radicaux choisis parmi les radicaux carboxy éventuellement salifiés ou estérifiés, les radicaux amino, méthylamino, diméthylamino, diéthylamino, le radical phényle éventuellement substitué par un ou plusieurs radicaux choisis parmi les radicaux hydroxy, méthyl, méthoxy, chloro, bromo, fluoro; les radicaux fluoro, chloro, bromo ou iodo, les radicaux nitrile, $CONH_2$ ou CONH $SO_2R''$ dans lequel R'' représente un radical alkyle ayant de 1 à 4 atomes de carbone, un radical phényle, un radical amino, méthyl ou diméthylamino, un radical amino hétérocyclique choisi parmi les radicaux pipéridino, morpholino, pipérazino ou 4-éthyl 2,3-dioxo 1-pipérazino;

– un radical cycloalkyle ayant de 3 à 8 atomes de carbone ou un radical:

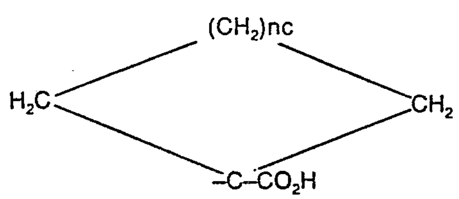

salifié ou estérifié dans lequel nc représente un entier de 0 à 5,

– un radical acétyle, propionyle ou benzoyle, carbamoyle, diméthylamino carbonyle, phényle ou benzyle éventuellement substitué par alkyle, alkoxy ou halogène.

$R_1$ représente un atome d'hydrogène, un radical alkyle, alkényle ou alkynyle ou thioalkyle ayant au plus 12 atomes de carbone, éventuellement substitué par un ou plusieurs radicaux choisis parmi les radicaux azido, alkylthio ayant de 1 à 4 atomes de carbone ou phénylthio, les radicaux carboxy éventuellement salifiés ou estérifiés, les radicaux amino, méthylamino, diméthylamino, diéthylamino, le radical phényl éventuellement substitué par un ou plusieurs radicaux choisis parmi les radicaux hydroxy, halogéno, trifluorométhyle, amino, alkyle ou alkoxy ayant de 1 à 4 atomes de carbone; les radicaux fluoro, chloro, bromo ou iodo, les radicaux nitrile, $CONH_2$ ou CONH $SO_2R''$ dans lequel R'' représente un radical alkyle ayant de 1 à 4 atomes de carbone, un radical phényle, un radical amino, méthyl ou diméthylamino, un radical amino hétérocyclique choisi parmi les radicaux pipéridino, morpholino, pipérazino ou 4-éthyl 2,3-dioxo 1-pipérazino; un radical aryle hétérocyclique choisi parmi les radicaux thiényle, furyle, pyrannyle, thiazolyle, thiadiazolyle, oxazolyle, oxadiazolyle, pyridinyle ou pyrimidinyle; les radicaux acétoxy, propionyloxy, benzoyloxy, acétylamino, benzylcarbonyle, carbamoyloxy, méthylamino carbonyloxy ou diméthylaminocarbonyloxy, acétyle, propionyle, benzoyle,

– un radical carboxy libre estérifié ou salifié,

– un radical phényle éventuellement substitué par un radical alkyle, trifluorométhyle, alkoxy, alkylthio, halogène, hydroxyle, amino, hydroxyalkyle, un radical hétérocyclique choisi parmi les radicaux thiényle, furyle, pyrolyle, thiazolyle, isothiazolyle, oxazolyle, isoxazolyle, imidazolinyle, imidazolyle, triazolyle, tétrazolyle, thiadiazolyle, oxadiazolyle, pyridinyle, pyrimidinyle, pyridazinyle, triazinyle éventuellement substitué par un radical alkyle, carboxy, carboxy alkyle, aminoalkyle ou dialkylaminoalkyle:

– un radical acétyle, propionyle, n-butyryle, benzoyle éventuellement substitué par alkoxy ou hydroxy,

– un radical carbamoyle, méthyl ou diméthylcarbamoyle,

– un radical azido.

$R_2$ représente un hétérocycle azoté éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$, $(CH_2)_nNHSO_3H$, $(CH_2)_nSO_2NH_2$, $(CH_2)_nCO_2H$ dans lesquels n représente un entier de 1 à 4 et comportant au moins un hydrogène acide, X représente un radical CH ou un atome d'azote, les traits ondulés signifient que le radical OR peut se trouver sous la forme syn ou anti et que les produits peuvent se trouver sous la forme cis ou trans ou sous la forme d'un mélange cis-trans, les produits de formule (I) étant sous forme racémique ou optiquement active, ainsi que les sels des produits de formule (I) avec les bases et les acides.

Parmi les valeurs de R, on peut citer:

a) les radicaux méthyle, éthyle, propyle, isopropyle, butyle, sec-butyle, tert-butyle, pentyle, isopentyle, sec-pentyle, tert-pentyle, néo-pentyle, hexyle, isohexyle, sec-hexyle, tert-hexyle, heptyle, octyle, décyle, undécyle, dodécyle;

b) les radicaux vinyle, allyle, 1-propényle, butényle, pentényle, hexényle;

c) les radicaux éthynyle, propargyle, butynyle.

Ces radicaux indiqués ci-dessus aux paragraphes a) à c) peuvent être substitués par un ou plusieurs radicaux tels que les radicaux carboxy éventuellement salifiés ou estérifiés, par exemple

les radicaux alkoxy carbonyle tels que méthoxy carbonyle, éthoxy carbonyle, tert-butoxy carbonyle, tert-amyloxy carbonyle, les radicaux benzyloxy carbonyle éventuellement substitués tels que p-nitro benzyloxy carbonyle, p-méthoxy benzyloxy carbonyle; parmi les radicaux carboxy estérifiés on peut également citer les radicaux carboxy estérifiés par les groupements benzhydryle, phényle, p-nitro phényle; le groupement carboxy peut également être estérifié par un groupement du type

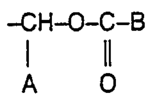

dans lequel A représente un alkyle ayant de 1 à 6 atomes de carbone ou un atome d'hydrogène et B représente un radical alkyle ou alkoxy ayant de 1 à 6 atomes de carbone.

Ces radicaux indiqués ci-dessus en a) à c) peuvent également être substitués par un radical amino, méthylamino, diméthylamino, diéthylamino, phényle éventuellement substitué par un ou plusieurs radicaux choisis parmi les radicaux hydroxy, méthyle, méthoxy, chloro, bromo, fluoro; les radicaux cités en a) à c) peuvent également être substitués par un des halogènes: fluoro, chloro, bromo ou iodo, par un radical nitrile, par un substituant $CONH_2$ ou $CONHSO_2R''$ dans lequel R″ peut représenter un radical alkyle tel que méthyle, éthyle, propyle, isopropyle, butyle, sec-butyle, tert-butyle, un radical aryle tel que phényle, un radical amino tel que amino, méthyl ou diméthylamino ou un radical amino hétérocyclique tel que pipéridino, morpholino ou pipérazino éventuellement substitué tel que 4-éthyl-2,3-dioxo 1-pipérazino.

Parmi les valeurs de R, on peut également citer les groupements carbocycliques tel que cyclopropyle, cyclobutyle, cyclopentyle, cyclohexyle, cycloheptyle et cyclooctyle.

R peut également représenter un radical du type

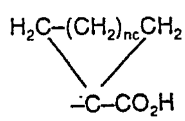

salifié ou estérifié dans lequel nc représente un entier de 0 à 5.

R peut également représenter un radical acyle tel que alkylcarbonyle par exemple acétyle ou propionyle, arylcarbonyle tel que benzoyle; un radical carbamoyle éventuellement substitué comme diméthylamino carbonyle, aryle tel que phényle éventuellement substitué par alkyle, alkoxy ou halogène ou aralkyle tel que benzyle éventuellement substitué de la même façon.

Le radical $R_1$ peut représenter également l'un des radicaux alkyle, alkényle ou alkynyle éventuellement substitué mentionné ci-dessus. $R_1$ peut également représenter un radical thioalkyle tel que thiométhyle ou thioéthyle ainsi que les radicaux thioalkyles dérivés des radicaux alkyles indiqués précédemment en a) pour le substituant R. Le radical thioalkyle que peut représenter $R_1$ peut être substitué par les mêmes radicaux que ceux indiqués ci-dessus pour les valeurs alkyle, alkényle ou alkynyle. Pour le radical thiométhyle, on préfère le substituant carbamoyle $CONH_2$.

On peut citer en plus les radicaux alkyle, alkényle, alkynyle ou thioalkyle substitués par l'un des radicaux suivants: azido, aryle tel que phényle éventuellement substitué par halogéno, trifluorométhyle, amino, hydroxyle, alkyle ou alkoxy ayant de 1 à 4 atomes de carbone, aryle hétérocyclique à 5 ou 6 chaînons tel que thiényle, furyle, pyrannyle, thiazolyle, thiadiazolyle, oxazolyle, oxadiazolyle, pyridinyle, pyrimidinyle.

Les radicaux alkyle, alkényle, alkynyle ou thioalkyle que peut représenter le substituant $R_1$ peuvent également être substitués par les radicaux alkylthio tel que méthylthio, éthylthio, propylthio, isopropylthio, butyl ou tert-butylthio; arylthio tel que phénylthio, par un radical acyle tel que acétyle, propionyle, benzoyle, acyloxy tel que acétoxy, propionyloxy, benzoyloxy, acylamino tel que acétylamino, aralkylcarbonyle tel que benzyl carbonyle; carbamoyloxy; méthylamino carbonyloxy ou diméthylaminocarbonyloxy.

Les valeurs de $R_1$ représentant un carboxy estérifié peuvent être choisies parmi les valeurs indiquées ci-dessus pour le substituant R.

On peut également citer les valeurs suivantes pour les groupements avec lesquels le radical carboxy est estérifié:
méthoxyméthyle, éthoxyméthyle,
isopropyloxyméthyle, α-méthoxyéthyle,
α-éthoxyéthyle, méthylthiométhyle,
éthylthiométhyle, isopropylthiométhyle,
pivaloyloxyméthyle, acétoxyméthyle,
propionyloxyméthyle, butyryloxyméthyle,
isobutyryloxyméthyle, valéryloxyméthyle,
isovaléryloxyméthyle,
tert-butyl carbonyloxyméthyle,
hexadécanoyloxyméthyle, propionyloxyéthyle,
isovaléryloxyéthyle, 1-acétyloxyéthyle,
1-propionyloxyéthyle, 1-butyryloxyéthyle,
1-tert-butylcarbonyloxyéthyle,
1-acétyloxypropyle, 1-hexadécanoyloxyéthyle,
1-propionyloxypropyle,
1-méthoxycarbonyloxyéthyle,
méthoxycarbonyloxyméthyle,
1-acétyloxybutyle, 1-acétyloxyhexyle,
1-acétyloxyheptyle, phtalidyle,
5,6-diméthoxyphtalidyle,
tert-butylcarbonylméthyle,
allyle, 2-chloroallyle, méthoxycarbonylméthyle,
benzyle ou tert-butyle,
méthoxyéthoxyméthyle, diméthylaminoéthyle,
cyanométhyle, tert-butyloxycarbonylméthyle,
2,2-éthylènedioxyéthyle, cyanoéthyle,
2,2-diméthoxyéthyle, 2-chloroéthoxyméthyle,
2-hydroxyéthoxyéthyle, 2,3-époxypropyle,
3-diméthylamino, 2-hydroxypropyle,
2-hydroxyéthyle, 2-méthylaminoéthoxyméthyle,
2-aminoéthoxyméthyle,
3-méthoxy 2,4-thiadiazol-5-yle,
2-tétrahydropyranyle, 2-méthoxyprop-2-yle,

1-hydroxyprop-2-yle, isopropyle,
carbamoylméthyle, chlorométhyle,
2-chloroéthyle, acétyl méthyle,
2-méthylthioéthyle ou thiocyanatométhyle,
2-chloro 1-acétyloxyméthyle,
2-bromo 1-acétyloxyéthyle,
2-fluoro 1-acétyloxyéthyle,
2-méthoxy 1-acétyloxyéthyle,
2-méthyl 1-acétyloxypropyle,
2-acétyloxyprop-2-yle,
1-méthoxyacétyloxyéthyle,
1-acétylcarbonyloxyéthyle,
1-hydroxyacétyloxyéthyle,
1-formylcarbonyloxyéthyle,
1-(2-thiényl) carbonyloxyéthyle,
1-(2-furyl) carbonyloxyéthyle,
1-(5-nitro 2-furyl) carbonyloxyéthyle,
1-(2-pyrrolyl) carbonyloxyéthyle,
1-(propionyloxy)carbonyloxyéthyle,
1-(propyloxycarbonyloxy) éthyle,
1-(isopropyloxycarbonyloxy) éthyle,
1-(méthoxyéthoxycarbonyloxy) éthyle,
1-(allyloxycarbonyloxy) éthyle,
1-(2,3-époxy) propyloxycarbonyloxy éthyle,
1-(2-furyl) méthyloxycarbonyloxy éthyle,
1-(2-fluoro) éthyloxycarbonyloxy éthyle,
1-(méthoxycarbonyloxy) propyle,
(2-méthoxycarbonyloxy) prop-2-yle,
(méthoxycarbonyloxy) chlorométhyle,
1-(méthoxycarbonyloxy) 2-chloroéthyle,
1-(méthoxycarbonyloxy 2-méthoxyéthyle,
1-(méthoxycarbonyloxy) 1-allyle.

Lorsque $R_1$ représente un radical aryle, il peut s'agir d'un radical phényle éventuellement substitué par un radical alkyle $CF_3$, alkoxy, alkylthio, halogéno, hydroxyle, amino, hydroxyalkyle, d'un radical hétérocyclique à 5 ou 6 chaînons contenant de 1 à 4 hétéroatomes tels que N, S ou O. On peut citer par exemple les radicaux thiényle, furyle, pyrolyle, thiazolyle, isothiazolyle, oxazolyle, isoxazolyle, imidazolinyle, imidazolyle, triazolyle, tétrazolyle, thiadiazolyle, oxadiazolyle, pyridinyle, pyrimidinyle, pyridazinyle, triazinyle. Ces radicaux hétérocycliques peuvent éventuellement être substitué par un radical alkyle, carboxy ou carboxyalkyle, aminoalkyle, dialkylaminoalkyle.

Parmi les groupements acyle que peut représenter $R_1$, on peut citer par exemple les radicaux acétyle, propionyle, n-butyryle, benzoyle éventuellement substitués par alkoxy ou hydroxy.

Parmi les groupements carbamoyle substitués, on peut citer les groupements méthyl ou diméthyl carbamoyle.

Parmi les valeurs de $R_2$, on peut citer plus particulièrement les radicaux hétérocycliques à 5 chaînons comprenant de 1 à 4 atomes d'azote tels que les radicaux pyrolyle, pyrazolyle, imidazolyle, triazolyle, tétrazolyle.

Ces radicaux peuvent être substitués par un ou plusieurs des radicaux choisis dans le groupe formé par les radicaux nitro, carboxy, $CF_3$, nitrile, halogène sulfo, alkylsulfo, $(CH_2)_nSO_3H$, $(CH_2)_nNHSO_3H$, $(CH_2)_nSO_2NH_2$, $(CH_2)_nCO_2H$ dans lesquels n représente un entier de 1 à 4.

Parmi les sels des produits de formule (I) avec les bases, on peut citer plus particulièrement les sels de sodium et de potassium. Ces sels peuvent être formés à l'aide de l'hydrogène acide que contient le substituant $R_2$. Comme les radicaux $R_1$ et R peuvent également comporter des fonctions acide salifiables, on peut obtenir des sels multiples.

En plus des sels de sodium et de potassium cités ci-dessus, on peut également citer les sels de lithium, calcium, magnésium, ammonium.

On peut également citer les sels de base organiques telle que la triméthylamine, la diéthylamine, la triéthylamine, la méthylamine, la propylamine, la N,N-diméthyléthanolamine, le tris (hydroxyméthyl) aminométhane, l'éthanolamine, la pyridine, la picoline, la dicyclohexylamine, la N',N'-dibenzyléthylènediamine, la morpholine, la benzylamine, la procaïne, la lysine, l'arginine, l'histidine, la N-méthyl glucamine.

Les produits de formule (I) peuvent se présenter sous forme de sels d'acides organiques ou minéraux puisque ces produits contiennent au moins un radical amino salifiable.

Parmi les acides avec lesquels on peut salifier les groupements amino des produits de formule (I), on peut citer entre autres, les acides acétique, trifluoroacétique, maléïque, tartrique, méthanesulfonique, benzènesulfonique, p-toluènesulfonique, chlorhydrique, bromhydrique, sulfurique, phosphorique. Les produits de formule (I) peuvent également se présenter sous forme de sels internes.

L'invention a plus particulièrement pour objet les produits compris à l'intérieur de la formule (I) précitée et répondant à la formule générale (I'):

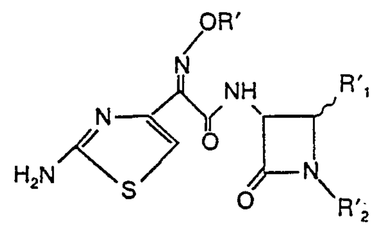

(I')

dans laquelle R' représente un atome d'hydrogène ou un radical alkyle, linéaire ou ramifié ayant de 1 à 6 atomes de carbone, éventuellement substitué par un ou plusieurs des radicaux choisis dans le groupe formé par les radicaux carboxy libre, estérifié ou salifié, amino, mono ou diméthylamino, phényle, halogène, nitrile, CONHSOR'' dans lequel R'' représente un radical alkyle ayant de 1 à 4 atomes de carbone, phényle ou amino, mono ou diméthylamino ou R' représente un radical acétyle, propionyle ou phényl carbonyle ou un radical phényle, ou R' représente un radical cycloalkyle ayant de 3 à 8 atomes de carbone ou un radical:

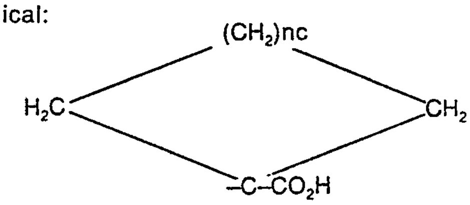

salifié ou estérifié dans lequel nc représente un entier de 0 à 5.

$R'_2$ représente un atome d'hydrogène ou un radical alkyle ayant au plus 12 atomes de carbone éventuellement substitué par un ou plusieurs des radicaux choisis dans le groupe formé par les radicaux halogène, azido, hydroxyle, mercapto, phényle, amino, nitrile, alkylthio ayant de 1 à 4 atomes de carbone ou phénylthio éventuellement oxydés, acétyle propionyle, benzoyle, acétoxy, propionyloxy, benzoyloxy, acétylamino, phényl carbonyle, carbamoyloxy, méthyl ou diméthyl carbamoyloxy ou $R'_1$ représente un radical alkényle ou alkynyle ayant au plus 12 atomes de carbone éventuellement substitué par un radical phényle ou par un ou plusieurs atomes d'halogènes, ou $R'_1$ représente un radical thioalkyle éventuellement substitué par carbamoyle, ou $R'_1$ représente un radical phényle éventuellement substitué par halogène, $CF_3$, amino, hydroxy, alkyle ou alkoxy ou $R'_1$ représente un radical carboxy estérifié, un radical carbamoyle ou un radical azido,

$R'_2$ représente un radical tétrazolyle, triazolyle, imidazolyle, pyrazolyle, ou pyrrolyle, éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$; $(CH_2)_nNHSO_3H$; $(CH_2)_nSO_2NH_2$; $(CH_2)_nCO_2H$ dans lesquels n représente un entier de 1 à 4, les produits ayant l'isomérie syn, le trait ondulé signifie que les produits peuvent se trouver sous la forme cis ou trans, ou sous la forme d'un mélange cis-trans, les produits de formule (I') étant sous forme racémique ou optiquement active ainsi que les sels des produits de formule (I') avec les bases et les acides.

Parmi les hétérocycles que peuvent représenter $R_2$ ou $R'_2$, on peut citer les différents isomères possibles. C'est ainsi que $R_2$ ou $R'_2$ peuvent représenter par exemple un radical pyrrol-2-yle ou pyrrol-3-yle, un radical pyrazol-3-yle ou pyrazol 4-yle, imidazol 2-yle ou imidazol 4-yle, 1,2,3-triazol 4-yle ou 1,2,4-triazol 3-yle.

Parmi les produits de formule (I') précédente, l'invention a plus spécialement pour objet les produits de formule (I') dans laquelle R' représente un atome d'hydrogène, un radical méthyle, phényle, difluorométhyle, 1-méthyl 1-carboxy éthyle, cyano méthyle, carboxy méthyle ou (méthyl sulfonyl) carbamoyl méthyle;

$R'_1$ représente un atome d'hydrogène, un radical méthyle, fluorométhyle, trifluorométhyle, éthoxycarbonyle, carbamoyle;

$R'_2$ représente un radical 1H-tétrazol-5-yle, ou 1,3,4-triazol-2-yle éventuellement substitué par un radical trifluorométhyle ou carboxyméthyle.

On préfère le tétrazolyle et notamment:

– la 3-[[2-(2-aminothiazol-4-yl) 2-méthoxyiminoacétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-carboxyméthoxyimino acétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-(1-carboxy 1-méthyl) éthoxyimino acétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-méthoxyimino acétyl] amino] 4-fluorométhyl 1-(1-H-tétrazol-5-yl) 2-azétidinone cis ou trans, isomère syn, racémique ou optiquement active;

– la 3-[[2-(2-aminothiazol-4-yl) 2-fluorométhoxyimino acétyl] amino] 4-méthyl 1-(1-H-tétrazol-5-yl) 2-azétidinone cis ou trans, isomère syn, racémique ou optiquement active.

L'invention a également pour objet un procédé de préparation des produits de formule générale (I) telle que définie ci-dessus, caractérisé en ce que l'on traite un produit de formule (II):

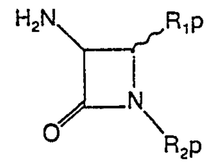

(II)

cis ou trans, racémique ou optiquement actif, formule dans laquelle soit $R_1p$ représente $R_1$, $R_1$ ayant la signification indiquée ci-dessus, soit $R_1p$ représente le substituant $R_1$ dans lequel les fonctions réactives sont protégées et soit $R_2p$ représente $R_2$, $R_2$ ayant la signification précédente soit $R_2p$ représente le substituant $R_2$ dans lequel les fonctions réactives sont protégées, par un produit de formule (III):

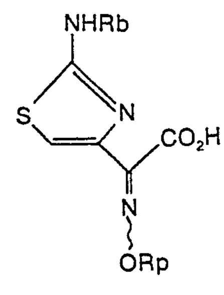

(III)

syn ou anti, dans laquelle Rb représente un atome d'hydrogène ou un groupement protecteur du radical amino et Rp représente un groupement protecteur du radical hydroxyle ou Rp représente R, R ayant la signification précédente ou Rp représente un radical R dans lequel les fonctions réactives sont protégées, pour obtenir un produit de formule (IV):

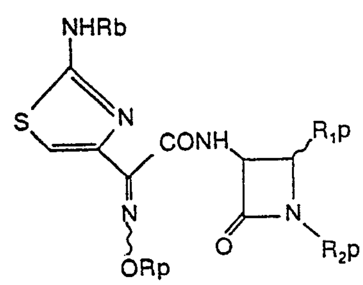

(IV)

syn ou anti, racémique ou optiquement actif, dans lequel Rp, $R_1p$, $R_2p$ et Rb ont la signification précédente, produit que l'on soumet si nécessaire et, si désiré, à l'une quelconque des réactions suivantes, dans un ordre quelconque:

a) coupure par hydrolyse, hydrogénolyse ou par action de la thiourée du ou des groupements protecteurs que peuvent représenter Rb et Rp ou comporter Rp, $R_2p$ et $R_2p$;

b) estérification ou salification des radicaux carboxy ou sulfo que peuvent comporter les radicaux Rp, $R_1p$ et $R_2p$;

c) salification par un acide du ou des radicaux amino;

d) dédoublement de la molécule pour obtenir un produit optiquement actif.

Dans le cas où $R_1p$ comporte un radical hydroxyle ou amino, il peut être avantageux de protéger ces radicaux par des groupements protecteurs éliminables.

Les groupements protecteurs du radical amino peuvent être par exemple un radical alkyle, de préférence tert-butyle ou tert-amyle; ils peuvent également être compris parmi les groupements acyles, aliphatiques, aromatiques ou hétérocycliques et carbamoyle.

On peut également citer les groupements alcanoyle inférieur tels que par exemple formyle, acétyle, propionyle, butyryle, isobutyryle, valéryle, isovaléryle, oxalyle, succinyle, pivaloyle. $R_1p$ peut également comporter un groupe alkoxy ou cycloalkoxycarbonyle inférieur tel que par exemple,
méthoxycarbonyle, éthoxycarbonyle,
propoxycarbonyle,
1-cyclopropyléthoxycarbonyle,
isopropyloxycarbonyle,
butyloxycarbonyle,
tert-butyloxycarbonyle,
pentyloxycarbonyle,
hexyloxycarbonyle,
un groupe benzoyle, toluolyle,
naphtoyle, phtaloyle, mésyle,
phénylacétyle, phénylpropionyle,
un groupe aralcoxycarbonyle,
tel que benzyloxycarbonyle.

Les groupements acyles peuvent être substitués par exemple par un atome de chlore, de brome, d'iode ou de fluor.

On peut citer les radicaux
chloroacétyle, dichloroacétyle,
trichloroacétyle, bromoacétyle ou
trifluoroacétyle.

On peut également utiliser un groupement aralkyle inférieur tel que benzyle,
4-méthoxybenzyle ou phényléthyle,
trityle, 3,4-di-méthoxybenzyle ou
benzhydryle.

On peut également utiliser un groupe haloalkyle tel que trichloroéthyle.

On peut également utiliser un groupement
chlorobenzoyle, para-nitrobenzoyle,
para-tert-butylbenzoyle,
phénoxyacétyle, caprylyle,
n-décanoyle, acryloyle,
trichloroéthoxycarbonyle.

On peut également utiliser un groupement
méthylcarbamoyle,
phénylcarbamoyle,
naphtylcarbamoyle,
ainsi que les thiocarbamoyles correspondants.

On peut également utiliser un groupement allyle, benzyloxyalkyle, alkoxyalkoxyalkyle ou encore ω-phénylsulfonylalkyl.

La liste ci-dessus n'est pas limitative, il est évident que d'autres groupements protecteurs des amines, groupements connus en particulier dans la chimie des peptides, peuvent également être utilisés.

Le groupement de protection du radical hydroxyle peut être choisi dans la liste ci-dessous:

Ce peut être un groupe acyle tel que par exemple
formyle, acétyle, chloroacétyle,
bromoacétyle, dichloroacétyle,
trichloroacétyle, trifluoroacétyle,
méthoxyacétyle, phénoxyacétyle,
benzoyle, benzoylformyle,
p-nitrobenzoyle.
On peut citer également les groupements
éthoxycarbonyle, méthoxycarbonyle,
propoxycarbonyle,
βββ-trichloroéthoxycarbonyle,
benzyloxycarbonyle,
tert-butoxycarbonyle,
1-cyclopropyléthoxycarbonyle,
tétrahydropyrannyle,
tétrahydrothiopyrannyle,
méthoxytétrahydropyrannyle,
trityle, benzyle,
4-méthoxybenzyle,
benzhydryle, trichloroéthyle,
1-méthyle 1-méthoxyéthyle, phtaloyle.

On peut également citer d'autres acyles tels que
propionyle, butyryle, isobutyryle,
valéryle, isovaléryle, oxalyle,
succinyle et pivaloyle.

On peut également citer les radicaux
phénylacétyle, phénylpropionyle, mésyle,
chlorobenzoyle,
para-nitrobenzoyle,
para-tert-butylbenzoyle, caprylyle,
acryloyle, méthylcarbamoyle,
phénylcarbamoyle, naphtylcarbamoyle.

Bien entendu, les valeurs des substituants Rb lorsque celui-ci ne représente pas un atome d'hydrogène, ainsi que les valeurs des groupements protecteurs que peut éventuellement représenter ou comporter Rp, en particulier lorsque Rp comporte une amine, peuvent être prises dans les listes mentionnées ci-dessus. Il en est de même pour les groupes que peut comporter $R_2p$.

Dans un mode préférentiel d'exécution du procédé, on traite le produit de formule (II) par un dérivé fonctionnel d'un produit de formule (III). Ce dérivé fonctionnel peut être par exemple un halogénure, un anhydride symétrique ou mixte, un amide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formés par exemple avec le chlorure de paratoluènesulfonyle. Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

On peut citer également l'azide d'acide ou l'amide d'acide.

L'anhydride peut être formé in situ par action de carbodiimide N,N'-disubstitué, par exemple la N,N'-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

Lorsque Rb représente un atome d'hydrogène, on utilise de préférence un anhydride mixte carboxylique sulfonique.

Selon les valeurs de Rb, $R_1$p, $R_2$p et Rp, les produits de formule (IV) peuvent ou non constituer des produits de formule (I).

Les produits de formule (IV) constituent des produits de formule (I) lorsque Rb représente un atome d'hydrogène, lorsque Rp ne représente pas un groupement protecteur du radical hydroxyle ou ne représente pas un radical R comportant une fonction protégée, enfin lorsque $R_1$p ne représente pas un radical $R_1$ dans lequel une fonction réactive est protégée et que $R_2$ ne représente pas un hétérocycle comportant un groupement protecteur.

Dans les autres cas, l'action sur le produit de formule (IV) d'un ou plusieurs agents d'hydrolyse, d'hydrogénolyse ou de la thiourée a pour but d'éliminer le radical Rb lorsque celui-ci représente un radical protecteur du radical amino, d'éliminer le radical Rp lorsque celui-ci représente un groupement protecteur du radical hydroxyle et d'éliminer les autres groupements protecteurs que peuvent comporter les radicaux Rp, $R_1$p et $R_2$p.

La nature des réactifs à mettre en jeu dans tous ces cas est bien connue de l'homme de métier. Des exemples de telles réactions sont donnés plus loin dans la partie expérimentale.

On donne ci-après une énumération non exhaustive des moyens pouvant être mis en œuvre pour éliminer les différents groupements.

L'élimination du groupe Rb peut être effectuée par hydrolyse, celle-ci étant acide, basique ou utilisant l'hydrazine.

On utilise préférentiellement l'hydrolyse acide pour éliminer les groupements alkoxy et cycloalkoxycarbonyle éventuellement substitués, tels que tert-pentyloxycarbonyle ou tert-butyloxycarbonyle, les groupements aralcoxycarbonyle éventuellement substitués tels que benzyloxycarbonyle, les groupements trityle, diphénylméthyle, tert-butyle ou 4-méthoxybenzyle.

L'acide que l'on utilise de préférence peut être choisi dans le groupe constitué par les acides chlorhydrique, benzène sulfonique ou paratoluène sulfonique, formique ou trifluoroacétique. On peut cependant utiliser d'autres acides minéraux ou organiques.

L'hydrolyse basique est utilisée préférentiellement pour éliminer les groupements acyle tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Le groupement Rb peut également être éliminé par le système zinc-acide acétique (pour les groupements haloalkyle et notamment trichloroéthyle), les groupements diphénylméthyle, benzyloxycarbonyle sont éliminés de préférence par l'hydrogène en présence d'un catalyseur.

Le groupement chloroacétyle est éliminé par action de la thiourée en milieu neutre ou acide selon le type de réaction décrit par MASAKI J.A.C.S., 90, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle. On préfère particulièrement les radicaux trityle et chloroacétyle. Pour le radical $R_2$p, on préfère le groupement benzyle.

L'acide que l'on utilise de préférence est l'acide trifluoroacétique ou l'acide formique.

L'élimination du radical Rp ou des groupements protecteurs que comportent Rp, $R_1$p ou $R_2$p, lorsque celle-ci est nécessaire, est réalisée dans des conditions semblables à celles décrites précédemment pour l'élimination de Rb.

On peut utiliser, entre autres, l'hydrolyse acide pour éliminer les radicaux alkyle ou aralkyle éventuellement substitués.

On utilise préférentiellement un acide choisi dans le groupe formé par les acides chlorhydrique, formique, trifluoroacétique et para-toluène sulfonique.

Les autres valeurs des radicaux Rb ou Rp ou des groupements protecteurs que comportent Rp, $R_1$p ou $R_2$p sont, lorsque cela est désiré, éliminées selon les procédés connus de l'homme de métier.

On opère de préférence dans des conditions modérées, c'est-à-dire, à température ambiante ou en chauffant légèrement.

L'élimination du radical benzyle ou d'un radical benzyloxyalkyle sur le substituant $R_2p$ est effectuée de préférence par hydrogénolyse.

L'élimination du radical allyle sur $R_2p$ est effectuée par exemple par action du complexe triphénylphosphine chlorure de rhodium.

L'élimination du radical alkoxyalkoxyalkyle est effectuée par exemple par action du bromure de zinc ou du chlorure de titane.

L'élimination du radical ω-phénylsulfonylalkyle est effectuée par exemple par une base forte telle qu'un alcoolate alcalin.

Naturellement, on peut, lorsque par exemple Rb, Rp, $R_1p$ ou $R_2p$ sont ou comportent des groupements éliminables appartenant à des types différents, faire agir sur les produits (IV) plusieurs agents envisagés dans les énumérations précédentes.

La salification des produits peut être effectuée selon les méthodes usuelles.

La salification des produits dans lesquels Rp, $R_1p$ ou $R_2p$ comportent une fonction carboxy ou sulfo, peut par exemple être obtenue par action sur un produit sous forme acide ou sur un solvat, par exemple le solvat éthanolique ou sur un hydrate de cet acide, d'une base minérale telle que l'hydroxyde de sodium ou de potassium, le carbonate acide de sodium ou de potassium. On peut également utiliser les sels d'acides minéraux tels que le phosphate tri-sodique. On peut également faire appel à des sels d'acides organiques.

On trouvera une liste de tels sels d'acides organiques par exemple dans le brevet français 2 476 087.

On utilise de préférence comme sels de sodium, l'acétate de sodium, le 2-éthyl héxanoate de sodium ou le diéthyl acétate de sodium.

La salification peut également être obtenue par action d'une base organique ou d'un acide aminé.

L'estérification éventuelle des produits dans lesquels Rp, $R_1p$ ou $R_2p$ comporte une fonction acide est également effectuée dans les conditions classiques.

Le dédoublement éventuel des molécules racémiques de formule (II) ou (IV) peut être effectué selon les méthodes usuelles.

On peut utiliser un acide organique carboxylique ou sulfonique optiquement actif comme les acides tartriques, dibenzoyl tartrique, camphosulfonique ou glutamique, la décomposition du sel ainsi obtenu étant effectuée au moyen d'une base minérale telle que le carbonate acide de sodium ou d'une base organique telle qu'une amine tertiaire, par exemple la triéthylamine.

La présente invention concerne spécialement un procédé tel que décrit ci-dessus, caractérisé en ce que l'on utilise, pour la mise en œuvre du procédé, un produit de formule (II) dans laquelle $R_1p$ représente un atome d'hydrogène, un radical méthyle, fluorométhyle, trifluorométhyle, éthoxycarbonyle ou carbamoyle et $R_2p$ représente un radical 1H-tétrazol-5-yle ou 1,3,4-triazol-2-yle éventuellement substitué par un radical trifluorométhyle ou carboxyméthyle et un produit de formule (III) dans laquelle Rb représente un groupement protecteur du radical amino et Rp représente un radical protecteur du radical hydroxyle, un radical méthyle, phényle, difluorométhyle, 1-méthyl 1-carboxy éthyle, cyano méthyle, carboxyméthyle ou méthylsulfonylcarbamoyl méthyle.

La présente invention a également pour objet un procédé de préparation caractérisé en ce que les produits de formule (II):

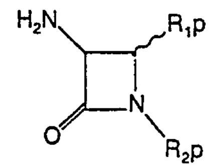

(II)

sont préparés en faisant réagir un produit de formule (V):

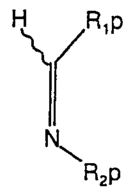

(V)

dans laquelle $R_1p$ et $R_2p$ ont la signification indiquée ci-dessus, en présence d'une base forte, avec un produit de formule (VI):

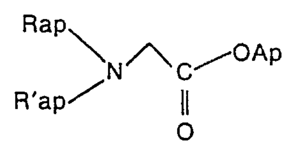

(VI)

dans laquelle Ap représente un atome d'hydrogène ou un groupement ester, Rap et R'ap sont tels que, soit Rap et R'ap représentent chacun un atome d'hydrogène, soit l'un représente un atome d'hydrogène et l'autre représente un groupement protecteur du radical amino, soit Rap et R'ap forment ensemble un radical divalent protecteur du radical amino, pour obtenir un produit de formule (VII):

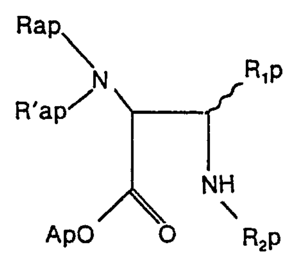

(VII)

Dans laquelle Ap, $R_1p$, $R_2p$, Rap et R'ap conservent la signification précédente et le trait ondulé signifie que le substituant $R_1p$ peut se trouver en position α ou β, produit de formule (VII) que, si désiré, l'on soumet à l'une ou l'autre des réactions suivantes, dans un ordre quelconque:

a) séparation des deux isomères;

b) protection du radical $NH_2$ lorsque Rap et R'ap représentent chacun un atome d'hydrogène, produit de formule (VII), sous forme d'un seul isomère ou d'un mélange d'isomères, que l'on soumet, lorsque Ap représente un groupement ester, à un agent de saponification, puis à un agent de β-lactamisation pour obtenir un produit de formule (VIII):

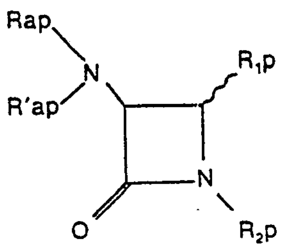
(VIII)

produit que l'on soumet, si nécessaire et si désiré, à l'une quelconque des réactions suivantes, dans un ordre quelconque:

a) séparation des isomères;

b) coupure par hydrolyse, hydrogénolyse ou action de la thiourée de l'un des radicaux Rap et R'ap ou de ces deux radicaux lorsque l'un représente un groupement protecteur, ou les deux représentent ensemble un groupement protecteur pour obtenir un produit de formule (II) attendu.

La base forte que l'on utilise de préférence est le butyl lithium en présence d'une amine secondaire telle que la diisopropylamine. On peut cependant utiliser un alcoolate de métal alcalin tel que le tert-butylate de potassium.

En plus des groupements protecteurs du groupement amino cités précédemment, Rap et R'ap peuvent représenter ensemble un groupement divalent tel que le groupement benzylidène ou le groupement

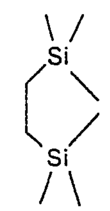

Ap peut représenter un des groupements esters cités précédemment. Parmi ceux-ci, on préfère un groupement alkyle inférieur tel que méthyle ou éthyle.

Si le radical amino du produit de formule (VII) est libre, soit parce que l'on a mis en œuvre le procédé en utilisant un produit de formule (VI) dans laquelle Rap et R'ap représentent chacun un atome d'hydrogène, soit que le groupement protecteur que représentent ces deux substituants ait été clivé lors de la réaction des produits de formules (V) et (VI), on procède éventuellement à une protection de ce radical amino. On utilise alors un des dérivés réactifs connus des groupements protecteurs cités précédemment. Dans un mode préférentiel d'exécution du procédé on protège le radical amino par un radical trityle en utilisant le chlorure de trityle en présence d'une base, préférentiellement une amine telle que la triéthylamine.

La saponification du groupement $-CO_2Ap$ est opérée, lorsque Ap représente un groupement ester, par action d'une base telle que la soude ou la potasse dans un solvant tel que le dioxanne, suivie d'une acidification par exemple par l'acide chlorhydrique.

La réaction de cyclisation qui permet d'obtenir les produits de formule (VIII) est opérée de préférence en présence d'un agent de β-lactamisation qui conduit à préparer un dérivé réactif du carbonyle.

On prépare de préférence l'anhydride mixte carboxylique sulfonique en présence de chlorure de tosyle et d'une base, de préférence le diazabicyclooctane ou la triéthylamine. On pourrait également utiliser la bromotriphénylphosphine.

Les réactions éventuelles de déprotection du radical amino protégé que représente le groupement

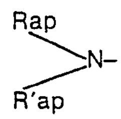

sont réalisées dans les conditions indiquées ci-dessus par exemple l'hydrolyse acide. De la même façon, il peut être préférable de cliver le groupement protecteur que comporte éventuellement le substituant $R_2p$.

La présente invention a également pour objet un procédé de préparation caractérisé en ce que les produits de formule (II) tels que décrits ci-dessus sont préparés en faisant réagir une β-lactone de formule (IX):

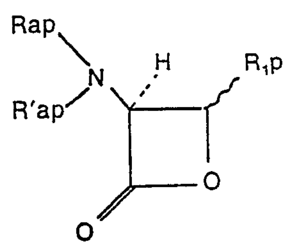
(IX)

dans laquelle $R_1p$, Rap et R'ap ont la signification indiquée ci-dessus, avec un produit de formule (A):

$$H_2N-R_2p \quad (A)$$

dans laquelle $R_2p$ a la signification indiquée ci-dessus, pour obtenir un produit de formule (X):

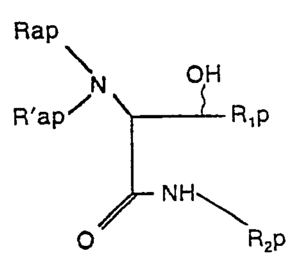
(X)

produit de formule (X) que, si désiré, l'on soumet à l'une ou l'autre des réactions suivantes, dans un ordre quelconque:

a) séparation des isomères dans le cas ou $R_1p$ ne représente pas un atome d'hydrogène;

b) protection du radical $NH_2$ lorsque Rap et R'ap représentent chacun un atome d'hydrogène ou modification du groupement protecteur que représente l'un ou l'autre de Rap et R'ap ou que forment ensemble Rap et R'ap et produit de formule (X) sous forme d'un isomère ou d'un mélange d'isomères que l'on soumet à un réactif de cyclisation pour obtenir un produit de formule (VIII):

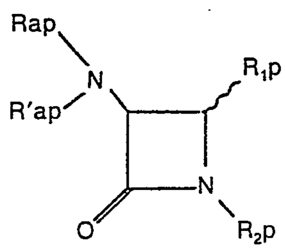
(VIII)

produit de formule (VIII) que l'on peut séparer en ses isomères et que l'on soumet, lorsque Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée pour obtenir un produit de formule (II) attendu.

L'action des produits de formule $H_2N-R_2p$ sur les produits de formule (IX) est effectuée préférentiellement en présence d'un trialkylaluminium tel que le triméthylaluminium. Le réactif A effectivement mis en œuvre est alors un produit de formule:

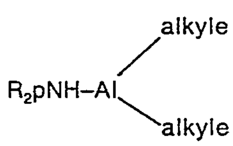

La protection du radical amino ou la modification éventuelle du groupement protecteur sont effectuées dans les conditions usuelles décrites ci-dessus.

Pour la suite de la réaction, on préfère que le radical amino soit protégé par un radical tel que benzyloxycarbonyle.

Le réactif de cyclisation qui permet de passer des produits de formule (X) aux produits de formule (VIII) est de préférence le diazodicarboxylate de diéthyle en présence de triphénylphosphine. On pourrait cependant utiliser une dialkylchloramine ou le tétrachlorure de carbone également en présence de triphénylphosphine ou de tris diméthylaminophosphine. On pourrait enfin utiliser la pyridine disulfure. Egalement, le radical hydroxy du produit X peut être activé par un groupement tel que le mésylate. La cyclisation est alors effectuée en présence d'une base telle que le carbonate acide de sodium ou le carbonate de sodium, (voir Chem. Pharm. Bull. 29, 1063 (1981).

Le traitement final éventuel des produits de formule (VIII) est effectué dans les conditions précédentes.

Comme le dédoublement des produits de formules (II) ou (IV), le dédoublement éventuel des molécules racémiques de formules (VII) ou (X) peut être effectué selon les méthodes usuelles.

On peut utiliser un acide organique carboxylique ou sulfonique optiquement actif comme les acides tartriques, dibenzoyl tartrique, camphosulfonique ou glutamique, la décomposition du sel ainsi obtenue étant effectuée au moyen d'une base minérale telle que le carbonate acide de sodium ou d'une base organiques telle qu'une amine tertiaire, par exemple la triéthylamine.

La présente invention concerne spécialement un procédé tel que décrit ci-dessus, caractérisé en ce que l'on utilise, pour la mise en œuvre du procédé, un produit de formule (IX), dans laquelle $R_1p$ représente un atome d'hydrogène ou un radical méthyle et obtient un produit (II) dans laquelle $R_1p$ représente un atome d'hydrogène ou un radical méthyle.

La présente invention a également pour objet un procédé de préparation caractérisé en ce que les produits de formule (II) tels que décrits ci-dessus sont préparés en faisant réagir un produit de formule

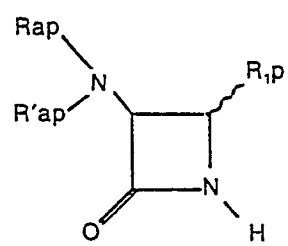
(XI)

dans laquelle $R_1p$, Rap et R'ap sont définis comme précédemment, avec un produit de formule

$$Y-R_2p \quad (XII)$$

dans laquelle $R_2p$ est défini comme précédemment et Y représente un groupement nucléofuge, en présence d'une base, pour obtenir un produit de formule (VIII) tel que défini précédemment, que l'on peut séparer en ses isomères et que l'on soumet, lorsque Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée, pour obtenir un produit de formule (II) attendu.

Le groupement nucléofuge Y est de préférence un atome d'halogène, plus particulièrement un atome de fluor ou de chlore, un groupement $-O-SO_2-CF_3$, $-O-SO_2-\Phi$ ou $-O-SO_2-CH_3$ ou encore un ammonium quaternaire.

La base utilisée peut être un hydrure métallique, un alcoolate alcalin, un amidure alcalin ou

une amine tertiaire. On utilise de préférence un amidure tel que le diisopropyl amidure de sodium ou de lithium ou le bis tri-méthylsilyl amidure de sodium ou de lithium.

La réaction est effectuée de préférence au sein d'un solvant ou d'un mélange de solvants tels que l'éther éthylique, le dioxanne, le tétrahydrofuranne, un alcane, un cycloalcane, le diméthylformamide, le diméthylsulfoxyde, l'hexaméthylphosphotriamide, le toulène, le benzène ou le xylène.

La réaction est effectuée très préférentiellement en utilisant au départ un produit de formule (XI) dont la fonction amino est protégée.

Les groupements Rap et R'ap peuvent être l'un de ceux qui ont été cités précédemment.

Les réactions éventuelles de déprotection du radical amino protégé sont réalisées dans les conditions indiquées précédemment, par exemple par hydrolyse acide.

Si le radical $R_2$p comporte un groupement protecteur, il peut être préférable de le cliver, en utilisant l'une des méthodes citées précédemment.

Ces composés de formule (XII) peuvent encore être préparés par transformation d'un composé de formule (A) telle que défini précédemment, en un dérivé diazoïque correspondant, puis par action sur celui-ci d'un halogénure métallique.

Ces composés peuvent encore être préparés par un procédé selon lequel l'on traite un isocyanate de formule (a)

$$p\text{–}N{=}C{=}O \qquad (a)$$

p étant un groupement protecteur, par l'hydrazine, transforme le composé obtenu (b)

$$p\text{–}NH\text{–}\overset{\overset{\displaystyle O}{\|}}{C}\text{–}NH\text{–}NH_2 \qquad (b)$$

en un azide (c)

$$p\text{–}NH\text{–}\overset{\overset{\displaystyle O}{\|}}{C}\text{–}N_3 \qquad (c)$$

cyclise l'azide par action du pentachlorure de phosphore pour obtenir l'halogénotétrazole attendu que, le cas échéant, l'on soumet à une réaction d'échange au niveau de l'halogène.

Un exemple d'une telle préparation est fourni ci-après dans la partie expérimentale.

Les produits de formule (XII) dans laquelle Y représente un groupement $-O-SO_2-CF_3$, $-O-SO_2-\Phi$ ou $-O-SO_2-CH_3$ peuvent être préparés au départ des dérivés hydroxylés correspondants, par exemple par action d'un chlorure de sulfonyle approprié.

Les produits de formule (XII) dans laquelle Y représente un groupement ammonium quaternaire peuvent être préparés au départ d'un produit de formule (A) telle que définie précédemment, par quaternisation, par exemple à l'aide d'un iodure d'alkyle.

L'invention a également pour objet un procédé de préparation caractérisé en ce que les produits de formule (XI) tels que décrits ci-dessus, de configuration trans, sont préparés en faisant réagir une base sur un produit de formule ($XI_1$):

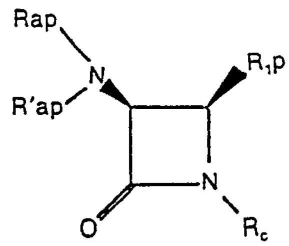

($XI_1$)

de configuration cis, sous forme de l'un ou l'autre des diastéréoisomères ou de leurs mélanges, fourmule ($XI_1$) dans laquelle $R_1$p, Rap et R'ap sont définis comme précédemment et $R_c$ représente un atome d'hydrogène ou un groupement protecteur, pour obtenir un produit de formule ($XI_2$):

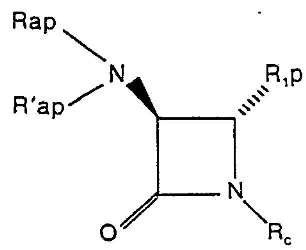

($XI_2$)

de configuration trans, sous forme de l'un ou l'autre des diastéréoisomères ou de leurs mélanges, formule ($XI_2$) dans laquelle $R_1$p, Rap, R'ap et $R_c$ sont définis comme précédemment, que l'on soumet, le cas échéant, à l'action d'un agent de coupure du groupement protecteur $R_c$.

Dans des conditions préférentielles d'exécution du procédé ci-dessus,

– la base utilisée est un alcoolate alcalin ou un amidure alcalin, notamment le tertbutylate de potassium, le diisopropylamidure de sodium ou de lithium ou le bis triméthylsilyl amidure de sodium ou de lithium;

– on opère au sein d'un solvant ou d'un mélange de solvants tels que l'éther éthylique, le dioxanne, le tétrahydrofuranne, un alcane, un cycloalcane, le diméthylformamide, le diméthylsulfoxyde, l'hexaméthyl phosphotriamide, le toluène, le benzène, ou le xylène;

– on utilise au départ un produit de formule ($XI_1$) dans laquelle Rap et R'ap sont définis préférentiellement comme précédemment et $R_c$ représente un groupement protecteur du radical amino, lequel peut être choisi dans la liste de groupements protecteurs d'amines énoncée précédemment et peut contenir un atome de carbone asymétrique; on peut citer, par exemple, le groupement 1-phényléthyle.

L'agent de coupure du groupement protecteur $R_c$ peut être l'un de ceux qui ont été envisagés précédemment. La coupure du groupement protecteur $R_c$ peut conduire, le cas échéant, à la coupure simultanée des groupements Rap et R'ap. Le groupement 1-phényléthyle peut, par

exemple, être éliminé par l'action du persulfate d'ammonium dans l'acétonitrile.

Les produits de formule générale (I) possèdent une très bonne activité antibiotique sur les bactéries gram (−), notamment sur les bactéries coliformes, les klebsiella, les salmonella et les proteus.

Ces produits peuvent notamment être utilisés comme médicaments dans le traitement des colibacilloses et infections associées, dans les infections à proteus, à klebsiella et à salmonella et dans d'autres affections provoquées par des bactéries à gram (−).

La présente invention a donc également pour objet, à titre de médicaments et notamment de médicaments antibiotiques, les produits de formule (I), tels que définis ci-dessus, ainsi que leurs sels pharmaceutiquement acceptables.

L'invention a particulièrement pour objet, à titre de médicaments et notamment de médicaments antibiotiques, les produits de formule (I'):

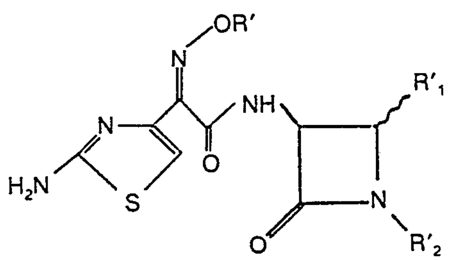

(I')

dans laquelle R' représente un atome d'hydrogène ou un radical alkyle linéaire ou ramifié ayant de 1 à 6 atomes de carbone, éventuellement substitué par un ou plusieurs des radicaux choisis dans le groupe formé par les radicaux carboxy libre, estérifié ou salifié, amino, mono ou dialkylamino, aryle, halogène, nitrile, $CONHSO_2R''$ dans lequel R'' représente un radical alkyle, aryle ou amino éventuellement substitué ou R' représente un radical alkylcarbonyle ou arylcarbonyle ou un radical phényle,

$R'_1$ représente un atome d'hydrogène ou un radical alkyle éventuellement substitué par un ou plusieurs des radicaux choisis dans le groupe formé par les atomes d'halogènes, les radicaux azido, hydroxyle, mercapto, aryle, amino, nitrile, alkylthio ou arylthio éventuellement oxydés, acyle, acyloxy, acylamino, aralkylcarbonyle, carbamoyloxy, alkyl ou dialkylcarbamoyloxy ou $R'_1$ représente un radical alkényle ou alkynyle éventuellement substitué par un radical phényle ou par un ou plusieurs atomes d'halogènes, ou $R'_1$ représente un radical thioalkyle éventuellement substitué par carbamoyle,

ou $R'_1$ représente un radical phényle éventuellement substitué par halogène, $CF_3$, amino, hydroxy, alkyle ou alkoxy ou $R'_1$ représente un radical carboxy estérifié, un radical carbamoyle ou un radical azido,

$R'_2$ représente un radical tétrazolyle, triazolyle, imidazolyle, pyrazolyle ou pyrrolyle éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$; $(CH_2)_nNHSO_3H$; $(CH_2)_nSO_2NH_2$; $(CH_2)_nCO_2H$ dans lesquels n représente un entier de 1 à 4, les produits ayant l'isomérie syn, le trait ondulé signifie que les produits peuvent se trouver sous la forme cis ou trans, ou sous la forme d'un mélange cis-trans, les produits de formule (I') étant sous forme racémique ou optiquement active ainsi que les sels des produits de formule (I') avec les bases et les acides.

L'invention a également plus particulièrement pour objet à titre de médicaments et notamment de médicaments antibiotiques, les produits décrits dans les exemples et spécialement

– la 3[[2-(2-aminothiazol-4-yl) 2-méthoxyiminoacétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-carboxyméthoxyimino acétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-(1-carboxy 1-méthyl) éthoxyimino acétyl] amino] 4-méthyl 1-(1-H-tétrazol-5-yl) 2-azétidinone cis ou trans, isomère syn, racémique ou optiquement active;

– la 3-[[2-(2-aminothiazol-4-yl) 2-méthoxyimino acétyl] amino] 4-fluorométhyl 1-(1-H-tétrazol-5-yl) 2-azétidinone cis ou trans, isomère syn, racémique ou optiquement active;

– la 3-[[2-(2-aminothiazol-4-yl) 2-fluorométhoxyimino acétyl] amino] 4-méthyl 1-(1-H-tétrazol-5-yl) 2-azétidinone cis ou trans, isomère syn, racémique ou optiquement active.

L'invention s'étend aux compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses.

Elles peuvent être solides ou liquides et se présenter sous les formes pharmaceutiques couramment utilisées en médecine humaine comme par exemple, les comprimés, simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous la forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1, ou encore comprise entre 0,500 g et 1 g trois fois par jour, par voie intramusculaire.

Les produits de formule (I) et leur sels peuvent également être utilisés comme désinfectants des instruments chirurgicaux.

L'invention a enfin pour objet, à titre de produits industriels nouveaux, les produits de formule générale (II):

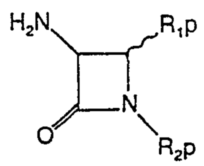

(II)

dans laquelle $R_1p$ et $R_2p$ ont la signification indiquée ci-dessus.

Les produits de formule (V) utilisés au départ du procédé de préparation peuvent être préparés par action d'une amine de formule (A):

$R_2p\ NH_2$ (A)

avec un aldéhyde de formule (B):

$R_1p\ CHO$ (B)

On peut obtenir intermédiairement un produit de formule (C):

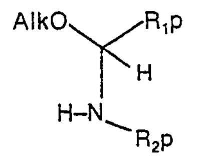

(C)

Alk représentant un radical alkyle correspondant normalement à l'alcanol dans lequel est effectuée la réaction. Les produits de formule (C) sont transformés en produits de formule (II), en général par chauffage, par exemple dans un solvant tel que le xylène. Un exemple d'une telle réaction est décrit dans la partie expérimentale.

Les produits de formule $R_2p\ NH_2$ peuvent être préparés selon les méthodes usuelles. Un exemple d'un tel produit, le 2-benzyl 5-amino tétrazole est décrit dans JACS, 76, 923 (1954).

Les produits de formule (VI) qui sont des dérivés de la glycine peuvent être préparés par les méthodes usuelles.

Les produits de formule (IX) peuvent également être préparés selon les méthodes usuelles. Notamment, lorsque $R_1p$ représente un atome d'hydrogène, le produit de formule (IX) est la lactone de la sérine éventuellement protégée sur la fonction amino. Lorsque $R_1p$ représente un radical méthyle, le produit de formule (IX) est un dérivé de la thréonine. La lactone de la N-trityl L-sérine est décrite dans JACS 81, 6086.

Les produits de formule ($XI_1$) dans laquelle $R_c$ représente un groupement protecteur peuvent être préparés à partir des produits de formule (XI) par des méthodes connues de l'homme de métier. Ils peuvent encore être préparés, comme les produits de formule (XI) eux-mêmes, par un procédé décrit dans le brevet belge n° 894 785.

En plus des produits décrits dans les exemples qui illustrent l'invention sans toutefois la limiter, les produits suivants constituent des produits pouvant être obtenus dans le cadre de la présente invention: les substituants X, R, $R_1$ et $R_2$ sont ceux indiqués dans la formule (I).

| X | R | $R_1$ | $R_2$ |
|---|---|---|---|
| CH | H | H | 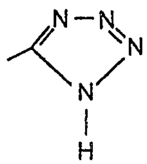 |
| " | " | $CH_3$ | " |
| " | " | $CH_2F$ | " |
| " | " | $CF_3$ | " |
| " | " | $CO_2Et$ | " |
| " | " | $CONH_2$ | " |
| " | " | $CHF_2$ | " |
| " | " | $CF_3$ | " |
| " | " | $CO_2Et$ | " |
| " | " | $CONH_2$ | " |
| " | $CHF_2$ | H | " |

| X | R | $R_1$ | $R_2$ |
|---|---|---|---|
| CH | $CH_2CN$ | $CO_2Et$ | 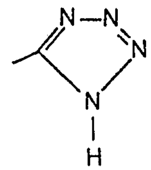 |
| " | " | $CONH_2$ | " |
| " | $CH_2CO_2H$ | H | " |
| " | " | $CHF_2$ | " |
| " | " | $CF_3$ | " |
| " | " | $CO_2Et$ | " |
| " | " | $CO{-}NH_2$ | " |
| " | $-CH_2CONH$ (N-substituted by $H_3C{-}SO_2$) | H | " |
| " | " | $CH_3$ | " |
| " | " | $CF_3$ | " |

| X | R | $R_1$ | $R_2$ |
|---|---|---|---|
| CH | $CH_3$ | H | 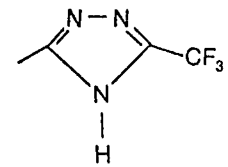 |
| " | " | $CH_2F$ | " |
| " | " | $CF_3$ | " |
| " | " | $CO_2Et$ | " |
| " | " | $CONH_2$ | " |
| " | $CHF_2$ | H | " |
| " | " | $CH_3$ | " |
| " | " | $CF_3$ | " |
| " | " | $CH_2F$ | " |
| " | " | $CO_2Et$ | " |
| " | " | $CONH_2$ | " |

(Fortsetzung)

| X | R | $R_1$ | $R_2$ |
|---|---|---|---|
| CH | $CHF_2$ | $CH_2F$ | 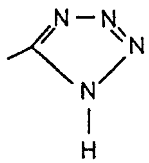 |
| " | " | $CF_3$ | " |
| " | " | $CO_2Et$ | " |
| " | " | $CONH_2$ | " |
| " | 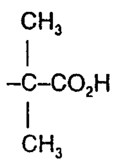 | H | " |
| " | " | $CHF_2$ | " |
| " | " | $CF_3$ | " |
| " | " | $CO_2Et$ | " |
| " | " | $CONH_2$ | " |

| X | R | $R_1$ | $R_2$ |
|---|---|---|---|
| CH | 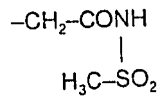 | $CO_2Et$ | 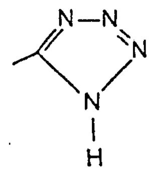 |
| " | " | $CONH_2$ | ″ |
| N | H | H | " |
| " | " | $CH_3$ | " |
| " | " | $CF_3$ | " |
| " | " | $CO_2Et$ | " |
| " | " | $CONH_2$ | " |
| " | $CH_3$ | H | " |
| " | " | $CH_3$ | " |

| X | R | $R_1$ | $R_2$ |
|---|---|---|---|
| CH | 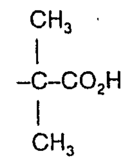 | H | 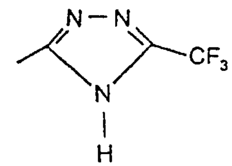 |
| " | " | $CH_3$ | " |
| " | " | $CH_2F$ | " |
| " | " | $CF_3$ | " |
| " | " | $CO_2Et$ | " |
| " | " | $CONH_2$ | " |
| " | φ | H | " |
| " | " | $CH_3$ | " |
| " | " | $CH_2F$ | " |
| " | " | $CF_3$ | " |
| " | " | $CO_2Et$ | " |
| " | " | $CONH_2$ | " |

(Fortsetzung)

| X | R | $R_1$ | $R_2$ |
|---|---|---|---|
| CH | φ | H | 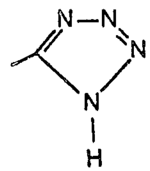 |
| " | " | $CH_3$ | " |
| " | " | $CH_2F$ | " |
| " | " | $CF_3$ | " |
| " | " | $CO_2Et$ | " |
| " | " | $CONH_2$ | " |
| " | $CH_2CN$ | H | " |
| " | " | $CH_3$ | " |
| " | " | $CH_2F$ | " |
| " | " | $CF_3$ | " |
| CH | $CH_2CO_2H$ | $CO_2Et$ | 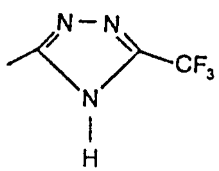 |
| " | " | $CONH_2$ | " |
| | | | " |
| " | $CH_2CONH$ – $H_3CSO_2$ | H | " |

| X | R | $R_1$ | $R_2$ |
|---|---|---|---|
| N | $CH_3$ | $CF_3$ | 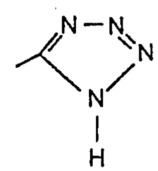 |
| " | " | $CO_2Et$ | " |
| " | " | $CONH_2$ | " |
| CH | H | H | 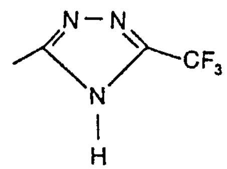 |
| " | " | $CH_3$ | " |
| " | " | $CF_3$ | " |
| " | " | $CO_2Et$ | " |
| " | " | $CONH_2$ | " |
| CH | φ | H | 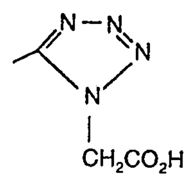 |
| " | " | $CH_3$ | " |
| " | " | $CH_2F$ | " |
| " | " | $CF_3$ | " |

| X | R | $R_1$ | $R_2$ |
|---|---|---|---|
| CH | $CH_2CN$ | H | 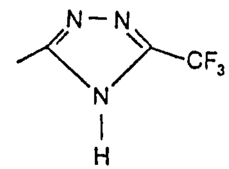 |
| " | " | $CH_3$ | " |
| " | " | $CH_2F$ | " |
| | | $CF_3$ | |
| " | " | $CO_2Et$ | " |
| " | " | $CONH_2$ | " |
| " | $CH_2CO_2H$ | H | " |
| " | " | $CH_3$ | " |
| " | " | $CH_2F$ | " |
| " | " | $CF_3$ | " |
| CH | H | $CH_3$ | 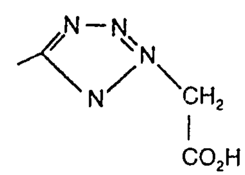 |
| " | CH $F_2$ | $CH_3$ | " |

(Fortsetzung)

| X | R | $R_1$ | $R_2$ |
|---|---|---|---|
| CH | 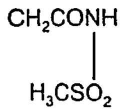 | $CH_3$ | 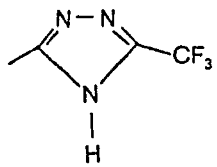 |
| " | " | $CH_2F$ | " |
| " | " | $CF_3$ | " |
| " | " | $CO_2Et$ | " |
| " | " | $CONH_2$ | " |
| " | H | H | 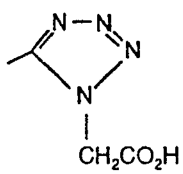 |
| " | " | $CH_3$ | " |
| " | " | $CH_2F$ | " |
| " | " | $CF_3$ | " |
| " | " | $CO_2Et$ | " |
| " | " | $CONH_2$ | " |
| " | $CH_3$ | H | " |

| X | R | $R_1$ | $R_2$ |
|---|---|---|---|
| CH | φ | $CO_2Et$ | 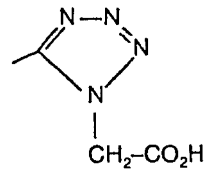 |
| " | " | $CONH_2$ | " |
| " | $CH_2CN$ | H | " |
| " | " | $CH_3$ | " |
| " | " | $CH_2F$ | " |
| " | " | $CF_3$ | " |
| " | " | $CO_2Et$ | " |
| " | " | $CONH_2$ | " |
| " | $CHCO_2H$ | H | " |
| " | " | $CH_3$ | " |
| " | " | $CH_2F$ | " |
| " | " | $CF_3$ | " |

| X | R | $R_1$ | $R_2$ |
|---|---|---|---|
| CH | 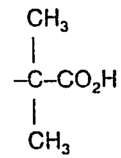 | $CH_3$ | 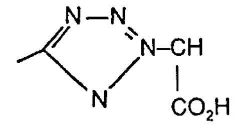 |
| " | φ | " | " |
| " | CN | " | " |
| " | $CH_2CO_2H$ | " | " |
| " | H | $CH_2F$ | " |
| " | $CH_3$ | " | " |
| " | $CH_2F$ | " | " |
| " | 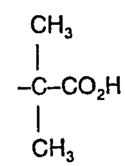 | " | " |
| " | φ | " | " |
| " | CN | " | " |
| " | $CH_2CO_2H$ | " | " |

(Fortsetzung)

| X | R | $R_1$ | $R_2$ | X | R | $R_1$ | $R_2$ | X | R | $R_1$ | $R_2$ |
|---|---|---|---|---|---|---|---|---|---|---|---|
| CH | $CH_3$ | $CH_3$ | 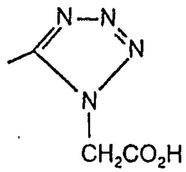 | CH | $CH_2CO_2H$ | $CO_2Et$ | 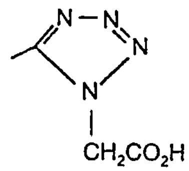 | CH | 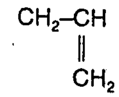 | H | 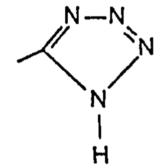 |
| " | " | $CH_2F$ | | " | " | $CONH_2$ | " | " | " | $CH_2F$ | " |
| " | " | $CF_3$ | " | " | 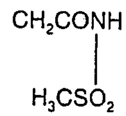 | H | " | " | " | $CF_3$ | " |
| " | " | $CO_2Et$ | " | " | " | $CH_3$ | " | " | " | $CO_2Et$ | " |
| " | " | $CONH_2$ | " | " | " | $CH_2F$ | " | " | 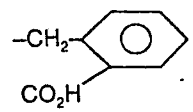 | H | " |
| " | $CHF_2$ | H | " | " | " | $CF_3$ | " | " | " | $CH_3$ | " |
| " | " | $CH_3$ | " | " | " | $CO_2Et$ | " | " | " | $CH_2F$ | " |
| " | " | $CH_2F$ | | " | " | $CONH_2$ | " | " | " | $CF_3$ | " |
| " | " | $CF_3$ | " | " | H | H | 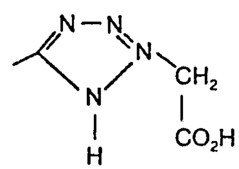 | " | " | $CO_2Et$ | " |
| " | " | $CO_2Et$ | " | " | $CH_3$ | " | " | | | | |
| " | " | $CONH_2$ | " | " | $CHF_2$ | " | " | | | | |
| " | 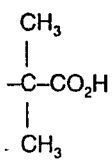 | H | " | " | 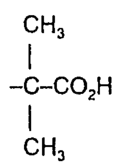 | " | " | | | | |
| " | " | $CH_3$ | " | | | | | | | | |

(Fortsetzung)

| X | R | $R_1$ | $R_2$ |
|---|---|---|---|
| CH | 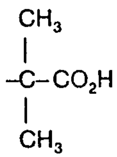 | $CH_2F$ | 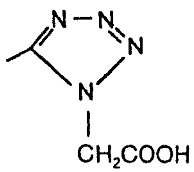 |
| " | " | $CF_3$ | " |
| " | " | $CO_2Et$ | " |
| " | " | $CONH_2$ | " |
| CH | 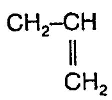 | H | 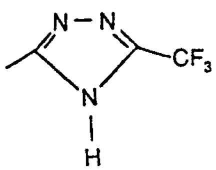 |
| " | " | $CH_3$ | " |
| " | " | $CH_2F$ | " |
| " | " | $CF_3$ | " |
| " | " | $CO_2Et$ | " |
| " | 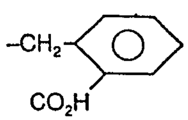 | H | " |
| " | " | $CH_3$ | " |
| " | " | $CH_2F$ | " |

| X | R | $R_1$ | $R_2$ |
|---|---|---|---|
| CH | φ | H | 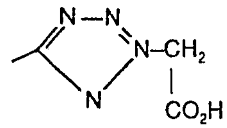 |
| " | CN | " | " |
| " | $CH_2CO_2H$ | " | " |
| CH | 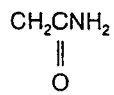 | $CF_3$ | 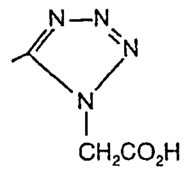 |
| " | " | $CO_2Et$ | " |
| " | H | Et | " |
| " | $CH_3$ | " | " |
| " | $CH_2F$ | " | " |
| " | 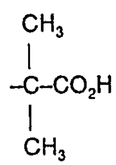 | " | " |
| " | φ | " | " |
| " | $CH_2CN$ | " | " |

| X | R | $R_1$ | $R_2$ |
|---|---|---|---|
| CH | 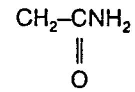 | H | 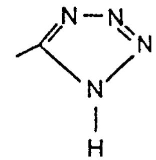 |
| " | " | $CH_3$ | " |
| " | " | $CH_2F$ | " |
| " | " | $CF_3$ | " |
| " | " | $CO_2Et$ | " |
| CH | H | H | 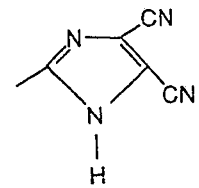 |
| " | $CH_3$ | " | " |
| " | $CHF_2$ | " | " |
| | 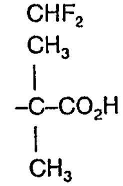 | " | " |
| " | φ | " | " |
| " | $CH_2CN$ | " | " |
| " | $CH_2CO_2H$ | " | " |
| " | 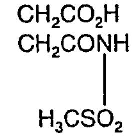 | " | " |

(Fortsetzung)

| X | R | $R_1$ | $R_2$ |
|---|---|---|---|
| CH | 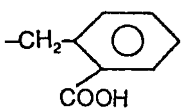 | $CF_3$ | 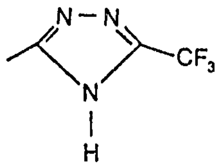 |
| " | " | $-CO_2Et$ | " |
| " | $-CH_2C(=O)NH_2$ | H | " |
| " | " | $CH_3$ | " |
| " | " | $CH_2F$ | " |
| " | " | $CF_3$ | " |
| " | " | $-CO_2Et$ | " |
| " | $CH_2-CH=CH_2$ | H | 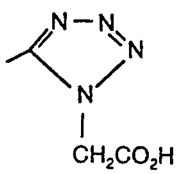 |
| " | " | $CH_3$ | " |
| " | " | $CH_2F$ | " |
| " | " | $CF_3$ | " |
| " | " | $CO_2Et$ | " |

| X | R | $R_1$ | $R_2$ |
|---|---|---|---|
| CH | $CH_2CO_2H$ | Et | 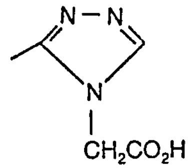 |
| " | $CH_2CONH-H_3CSO_2$ | " | " |
| " | H | " | 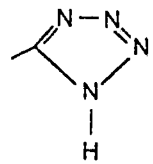 |
| " | $CH_2F$ | " | " |
| " | $-C(CH_3)_2-CO_2H$ | " | " |
| " | φ | " | " |
| " | $CH_2CN$ | " | " |
| " | $CH_2CO_2H$ | " | " |
| " | $CH_2CONH-H_3CSO_2$ | " | " |
| " | $-CH_2-CH=CH_2$ | " | " |
| " | H | $-S-CH_3$ | " |

| X | R | $R_1$ | $R_2$ |
|---|---|---|---|
| CH | $-CH_2-CH=CH_2$ | H | 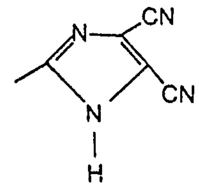 |
| " | H | $CH_3$ | " |
| " | $CH_3$ | " | " |
| " | $CHF_2$ | " | " |
| " | $-C(CH_3)_2-CO_2H$ | " | " |
| " | φ | " | " |
| " | $CH_2CN$ | " | " |
| " | $CH_2CO_2H$ | " | " |
| " | $CH_2-CONH-H_3CSO_2$ | " | " |
| " | $-CH_2-CH=CH_2$ | " | " |
| " | H | H | 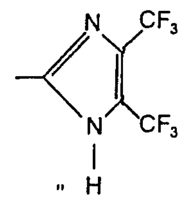 |
| " | $CH_3$ | " | " |

(Fortsetzung)

| X | R | $R_1$ | $R_2$ | X | R | $R_1$ | $R_2$ | X | R | $R_1$ | $R_2$ |
|---|---|---|---|---|---|---|---|---|---|---|---|
| CH | 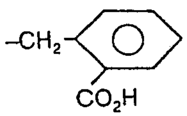 | H | 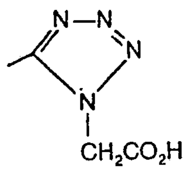 | CH | $CH_3$ | $-S-CH_3$ | 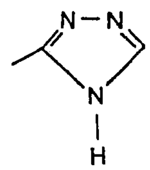 | CH | $CHF_2$ | H | 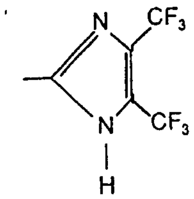 |
| " | " | $CH_3$ | " | " | $CH_2F$ | " | " | " | 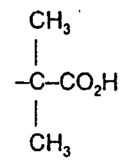 | " | " |
| " | " | $CH_2F$ | " | " | 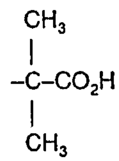 | " | " | " | H | $CH_3$ | " |
| " | " | $CF_3$ | " | " | φ | " | " | " | $CH_3$ | " | " |
| " | " | $CO_2Et$ | " | " | $CH_2CN$ | " | " | " | $CHF_2$ | " | " |
| " | 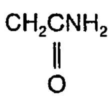 | H | " | " | $CH_2CO_2H$ | " | " | " | 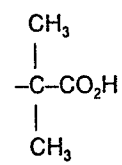 | " | " |
| " | " | $CH_3$ | " | " | 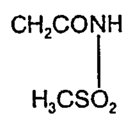 | " | " | " | H | H | 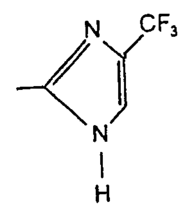 |
| " | " | $CH_2F$ | " | " | 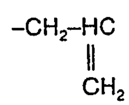 | " | " | " | $CH_3$ | " | " |

| X | R | $R_1$ | $R_2$ |
|---|---|---|---|
| CH | $CHF_2$ | H | 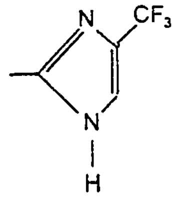 |
| " | 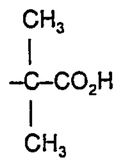 | " | " |
| " | H | $CH_3$ | " |
| " | $CH_3$ | " | " |
| " | $CHF_2$ | " | " |
| " | 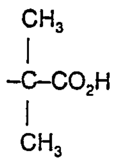 | " | " |
| " | H | $CH_2F$ | " |
| " | $CH_3$ | " | " |
| " | $CHF_2$ | " | " |
| " | 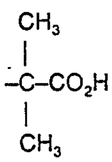 | " | " |
| " | 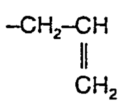 | " | " |
| " | " | $CH_3$ | " |
| " | " | H | " |
| " | 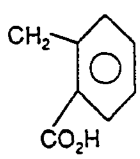 | " | " |
| " | " | $CH_3$ | " |
| ' | 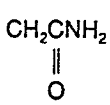 | " | " |
| " | " | H | " |
| | H | 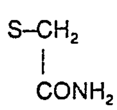 | 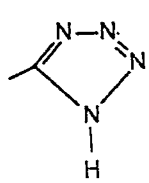 |
| ' | $CH_3$ | " | " |
| " | $CHF_2$ | " | " |

(Fortsetzung)

| X | R | $R_1$ | $R_2$ |
|---|---|---|---|
| CH | 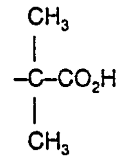 | 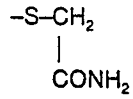 | 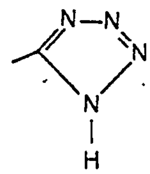 |
| " | φ | " | " |
| " | $CH_2CN$ | " | " |
| " | $CH_2CO_2H$ | " | " |
| " | 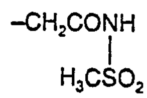 | " | " |
| " | 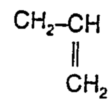 | " | " |
| " | 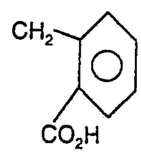 | " | " |
| " | 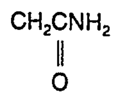 | " | " |

Exemple 1

(3SR,4RS) 3-[[2-(2-aminothiazol-4-yl) 2-méthoxyimino acétyl] amino]-4-méthyl 1-(1H-tétrazol-5-yl)-2-azétidinone isomère syn.

a) Chlorhydrate de (3SR,4RS) 3-amino 4-méthyl 1-(1H-tétrazol-5-yl) 2-azétidinone.

On porte au reflux une solution de 990 mg de (3SR,4RS) chlorhydrate de la 3-amino 4-méthyl 1-[2-(phényl méthyl) 2-H-tétrazol-5-yl] 2-azétidinone dans 30 $cm^3$ d'éthanol puis ajoute 800 mg de palladium sur charbon à 18% dans 5 $cm^3$ d'éthanol.

On fait passer un courant d'hydrogène pendant 15 minutes au reflux puis filtre et évapore sous pression réduite. On obtient 692 mg de produit attendu.

b) anhydride mixte paratoluène sulfonique 2-(2-tritylamino thiazol-4-yl) 2-méthoxyimino acétique isomère syn.

On ajoute 640 mg de chlorure de paratoluène sulfonyle à une solution de 1,5 g d'acide 2-(2-tritylamino thiazol-4-yl) 2-méthoxyimino acétique isomère syn dans 25 $cm^3$ d'acétone et 0,5 $cm^3$ de triéthylamine. On agite une demi-heure à température ambiante.

c) (3SR,4RS) 3-[[2-[2-tritylamino] thiazol-4-yl] 2-méthoxyimino acétyl] amino] 4-méthyl 1-(1H-tétrazol-5-yl) 2-azétidinone isomère syn.

La solution obtenue précédemment est versée dans une solution de 692 mg de chlorhydrate de (3SR,4RS) 3-amino 4-méthyl 1-(1-tétrazol-5-yl) 2-azétidinone dans 30 $cm^3$ d'acétonitrile et 1,5 $cm^3$ de triéthylamine. On agite à température ambiante pendant une heure trente. On ajoute 0,5 $cm^3$ d'acide acétique, essore le précipité, le lave avec 2 fois 2 $cm^3$ d'acétone. Après séchage, on obtient un premier jet de 460 mg de produit attendu. Le filtrat est concentré sous pression réduite jusqu'à 5 $cm^3$ et on le reprend par 50 $cm^3$ d'acétate d'éthyle. On lave avec 10 $cm^3$ d'eau, sèche la phase organique puis concentre sous pression réduite. On reprend le produit par 5 $cm^3$ d'acétate d'éthyle, agite, glace. Après cristallisation d'une heure trente à 0 °C, on essore, lave les cristaux avec 2 fois 3 $cm^3$ d'acétone puis à l'éther. On obtient ainsi un deuxième jet de 555 mg de produit attendu identique au précédent. On rassemble les deux jets soit en tout 1,015 g de produit (F $\simeq$ 200 °C).

d) (3SR,4RS) 3-[[2-(2-aminothiazol-4-yl) 2-méthoxyimino acétyl] amino] 4-méthyl 1-(1H-tétrazol-5-yl) 2-azétidinone isomère syn.

On chauffe à 50 °C pendant une demi-heure les 1,015 g de produit obtenu ci-dessus dans 5 $cm^3$ d'acide formique à 66%. On filtre le triphényl carbinol précipité et le lave avec 2 $cm^3$ d'acide formique à 66% puis avec 2 $cm^3$ d'eau. On dilue le filtrat avec 25 $cm^3$ d'eau et on concentre sous pression réduite jusqu'à 5 $cm^3$ environ. On essore les cristaux formés, les lave à l'eau puis avec 3 fois 2,5 $cm^3$ de méthanol et enfin à l'éther. Le produit est séché à 70 °C pendant 4 h. sous pression réduite. On obtient finalement 471 mg de produit attendu (F > 260 °C) (Rf = 0,25 éluant: acétate d'éthyle, éthanol-eau: 70:20:10).

Analyse: $C_{11} H_{13} N_9 O_3 S$ = 351,34

| | | | |
|---|---|---|---|
| Calculé | C% 37,6 | H% 3,73 | S% 9,12 |
| Trouvé | C% 37,2 | H% 3,7 | S% 8,8 |

le (3SR,4RS) chlorhydrate de la 3-amino 4-méthyl 1-[2-(phényl méthyl) 2-H-tétrazol-5-yl] 2-azétidinone a été préparé comme suit:

a) N-(1-méthoxy éthyl) 2-(phényl méthyl) 2H-tétrazol-5-amine.

On agite 18 h. à température ambiante un mélange de 15 g de 2-benzyl 5-amino tétrazole préparé selon JACS, 76, 923 (1954) dans 300 $cm^3$ de méthanol et 15 $cm^3$ d'aldéhyde acétique pur. On concentre sous pression réduite jusqu'à 25–30 g puis élue sur colonne de 400 g de silice avec de l'éther contenant 0,5% de triéthylamine. On obtient finalement 18,8 g de produit attendu (F $\simeq$ 60 °C) (Rf = 0,6 éluat: éther à 0,5% de triéthylamine).

b) N-éthylidène-2-(phényl méthyl) 2H-tétrazol-5-amine.

On chauffe au reflux 4,78 g de produit obtenu ci-dessus dans 100 $cm^3$ de xylène. On distille à volume constant par apport de xylène pur. On distille ainsi 100 $cm^3$ en une demi-heure. Après une demi-heure on évapore le solvant sous pression réduite et sèche à poids constant sous pression réduite. On obtient ainsi 4,18 g de produit attendu qui cristallise.

c) (2SR,3SR) et (2SR,3RS) 2-amino 3-[2-(phényl méthyl) 2-H-tétrazol-5-yl amino] butanoate de méthyle.

On refroidit à −60 °C une solution sous argon de 10 $cm^3$ de diisopropylamine dans 100 $cm^3$ de tétrahydrofuranne anhydre et ajoute en 5 minutes 40 $cm^3$ de butyllithium à 15% dans l'hexane.

La température remonte à −30 °C. On agite 5 minutes à −30 °C puis revient à −50 °C et ajoute lentement 11,5 g de l'ester méthylique de la N-(benzylidène) glycine, dans 30 $cm^3$ de tétrahydrofuranne. On agite 10 minutes à −50 °C puis ajoute à nouveau 13 g de N-éthylidène 2-(phényl méthyl) 2H-tétrazol-5-amine dans 20 $cm^3$ de tétrahydrofuranne. On laisse remonter à −30 °C et agite à cette température 30 minutes puis verse la solution dans un mélange de 130 $cm^3$ d'acide chlorhydrique 2N et 520 $cm^3$ d'eau. On agite 30 minutes à température ambiante. On lave la solution à l'éther puis ajoute de l'ammoniaque concentrée jusqu'à pH: 8,5–9. On extrait avec du chlorure de méthylène puis sèche. Le filtrat est concentré sous pression réduite. On recueille ainsi 19 g de résine que l'on chromatographie sous pression sur silice en éluant avec un mélange chlorure de méthylène, méthanol, ammoniaque concentrée: 97,5:2,5:0,4. On recueille dans l'ordre 7,22 g de produit trans et 3,9 g de produit cis.

d) (2SR,3RS) 2-[(triphényl méthyl) amino] 3-(2-phényl méthyl) 2H-tétrazol-5-yl amino] butanoate de méthyle.

On porte au reflux pendant 16 heures un mélange de 6 g de produit cis obtenu précédemment et 7 g de chlorure de trityle dans 60 $cm^3$ de tétrahydrofuranne et 4,2 $cm^3$ de triéthylamine puis laisse revenir à température ambiante et filtre. On concentre sous pression réduite puis reprend par 200 $cm^3$ de chlorure de méthylène. On lave à l'eau, sèche et évapore à sec. On chromatographie sur silice en éluant par un mélange hexane, acétate d'éthyle 7:3. On isole ainsi 7,45 g de produit attendu (Rf = 0,25 hexane-acétate d'éthyle 7:3).

e) (2SR,3RS) acide 2-[trityl amino] 3-[2-(phényl méthyl) 2H-tétrazol-5-yl amino] butanoïque.

On porte au reflux pendant 40 h. un mélange de 7,45 g de produit obtenu ci-dessus, 150 $cm^3$ de dioxane, 6 $cm^3$ de soude 1ON et 12 $cm^3$ d'eau puis concentre sous pression réduite. On reprend par 150 $cm^3$ d'eau, 150 $cm^3$ d'acétate d'éthyle et 30 $cm^3$ d'acide chlorhydrique 2N. On décante, extrait à l'acétate d'éthyle puis sèche. On évapore à sec. On reprend les cristaux à l'éther, glace, essore et lave à l'éther, puis sèche à 60 °C pendant

18 heures. On isole ainsi 6,22 g de cristaux blancs (F ≃ 200 °C).

f) (3SR,4RS) 4-méthyl 1-[2-(phényl méthyl) 2H-tétrazol-5-yl]-3-[tritylamino] 2-azétidinone.

On agite 2 minutes à température ambiante une solution de 4,88 g d'acide obtenu ci-dessus dans 50 $cm^3$ de chlorure de méthylène et 2,4 g de diazabicyclooctane puis la refroidit à −40 °C. On ajoute alors en une minute 9,5 $cm^3$ de chlorure de tosyle (1M) dans le chlorure de méthylène. On agite 15 minutes à −40 °C puis laisse remonter en une heure à 0 °C. On lave à l'eau puis sèche et concentre sous pression réduite.

On chromatographie le résidu sur silice en éluant avec un mélange éther isopropylique-chlorure de méthylène 5:95. On obtient ainsi 1,99 g de produit attendu. (Rf = 0,40 éluant éther isopropylique chlorure de méthylène: 5:95).

g) (3SR,4RS) chlorhydrate de la 3-amino 4-méthyl 1-[2-(phényl méthyl) 2H-tétrazol-5-yl] 2-azétidinone.

On agite 15 minutes à température ambiante un mélange de 1,97 g de produit obtenu ci-dessus en f); 20 $cm^3$ de chlorure de méthylène et 1 $cm^3$ d'acide chlorhydrique 8M dans le méthanol. On concentre sous pression réduite, reprend par 5 $cm^3$ de chlorure de méthylène et verse dans 50 $cm^3$ d'éther en agitant fortement. Le produit attendu précipite. On essore, lave à l'éther puis sèche sous pression réduite à 60 °C pendant 2 heures. On obtient 1,02 g de produit attendu (F ≃ 200 °C) (Rf = 0,4 éluant:chlorure de méthylène, méthanol, ammoniaque concentrée: 96:4:0,5%)

Exemple 2

(3SR,4SR) 3-[[2-(2-aminothiazol-4-yl) 2-méthoxyimino acétyl] amino] 4-méthyl 1-(1H-tétrazol-5-yl) 2-azétidinone.

a) chlorhydrate de (3SR,4SR) 3-amino 4-méthyl 1-(1H-tétrazol-5-yl) 2-azétidinone.

On opère comme au stade a) de l'exemple 1 au départ de 495 mg de chlorhydrate de (3SR,4SR) 3-amino 4-méthyl 1-[2-(phényl méthyl) 2-H-tétrazol-5-yl] 2-azétidinone et obtient 361 mg de produit attendu.

b) (3SR,4SR) 3-[[2-[2-tritylamino] thiazol-4-yl] 2-méthoxyimino acétyl] amino] 4-méthyl 1-(1H-tétrazol-5-yl) 2-azétidinone.

On verse une suspension d'anhydride mixte paratoluène sulfonique 2-(2-tritylamino 4-thiazolyl) 2-méthoxyimino acétique isomère syn préparé comme indiqué au stade b) de l'exemple 1 à partir de 450 mg d'acide 2-(2-tritylamino thiazol-4-yl) 2-méthoxyimino acétique isomère syn et 190 mg de chlorure de paratoluène sulfonyle, dans une solution de 209 mg de chlorhydrate de (3SR,4SR) 3-amino 4-méthyl 1-(1H-tétrazol-5-yl) 2-azétidinone dans 25 $cm^3$ d'acétonitrile et 0,45 $cm^3$ de triéthylamine. On agite 16 heures à température ambiante puis ajoute 0,2 $cm^3$ d'acide acétique et concentre sous pression réduite. On reprend par 60 $cm^3$ d'acétate d'éthyle, lave à l'eau puis sèche la phase organique. On évapore à sec, reprend par 3 $cm^3$ d'acétate d'éthyle, agite et laisse cristalliser. Après 2 heures à 0 °C, on essore. Le précipité est lavé avec de l'acétate d'éthyle puis à l'éther. On obtient ainsi 0,21 g de produit attendu (Rf = 0,4 éluant acétate d'éthyle-éthanol-eau 70:20:10).

c) (3SR,4SR) 3-[[2-(2-aminothiazol-4-yl) 2-méthoxyimino acétyl] amino] 4-méthyl 1-(1H-tétrazol-5-yl) 2-azétidinone.

On chauffe à 50 °C pendant 15 minutes 190 mg de produit obtenu ci-dessus dans 1,5 $cm^3$ d'acide formique à 66%. On laisse revenir à température ambiante pendant une demi-heure puis ajoute 2 $cm^3$ d'eau et filtre. On concentre le filtrat sous pression réduite, reprend le résidu par 4 $cm^3$ d'éthanol absolu et concentre sous pression réduite. On répète cette opération 3 fois et reprend le résidu par 2,5 $cm^3$ d'acétone. On observe une dissolution puis une cristallisation. On glace 30 minutes puis essore. Les cristaux sont lavés à l'éther puis séchés sous pression réduite à 40 °C. On obtient ainsi 75 mg de produit attendu (F ≃ 220 °C) (Rf = 0,25 éluant acétate d'éthyle-éthanol-eau 70:20:10).

Le (3SR,4SR) chlorhydrate de la 3-amino 4-méthyl 1-[2-(phényl méthyl) 2H-tétrazol-5-yl] 2-azétidinone a été préparé comme suit:

a) (2SR,3SR) 2-[tritylamino] 3-[2-(phényl méthyl)2-H-tétrazol-5-yl amino] butanoate de méthyle.

On opère comme indiqué au stade d) de la préparation suivant l'exemple 1 au départ de 7 g de produit trans obtenu au stade c) de la préparation de l'exemple 1 et de 8 g de chlorure de trityle. On obtient 9,42 g de produit attendu (F ≃ 160 °C) (Rf = 0,25 éluant hexane-acétate d'éthyle 7:3).

b) acide (2SR,3SR) 3-[2-(phényl méthyl] 2H-tétrazol-5-yl amino] 2-[tritylamino] butanoïque.

On opère comme indiqué au stade e) de la préparation de l'exemple 1 au départ de 9,42 g de produit obtenu ci-dessus. On obtient 8,3 g de produit attendu (F ≃ 200 °C).

c) (3SR,4SR) 4-méthyl 1-[2-(phényl méthyl) 2H-tétrazol-5-yl] 3-[(triphényl méthyl) amino] 2-azétidinone.

On opère comme indiqué au stade f) de la préparation de l'exemple 1 au départ de 8,3 g de produit obtenu ci-dessus. On obtient 3 g de produit attendu.

d) (3SR,4SR) chlorhydrate de la 3-amino 4-méthyl 1-[2-(phényl méthyl) 2H-tétrazol-5-yl] 2-azétidinone.

On opère comme indiqué au stade g) de la préparation de l'exemple 1 au départ de 1,7 g de produit obtenu ci-dessus. On obtient 790 mg de produit attendu.

Exemple 3
(3S) 3-[[2-(2-aminothiazol-4-yl) 2-méthoxyimino acétyl] amino] 1-(1H-tétrazol-5-yl) 2-azétidinone.

a) (3S) 3-[[2-tritylamino thiazol-4-yl) 2-méthoxyimino acétyl] amino] 1-(1H-tétrazol-5-yl) 2-azétidinone.

On opère comme indiqué au stade c) de l'exemple 1 au départ de 660 mg de chlorhydrate de (3S) 3-amino 1-(1H-tétrazol-5-yl) 2-azétidinone et de l'anhydride mixte paratoluène sulfonique 2-(2-tritylamino thiazol-4-yl) 2-méthoxyimino acétique isomère syn préparé comme au stade b) de l'exemple 1, pour obtenir le produit attendu (F $\simeq$ 220 °C). (Rf = 0,4 éluant: acétate d'éthyle-éthanol-eau 70/20/10).

b) (3S) 3-[[2-(2-aminothiazol-4-yl) 2-méthoxyimino acétyl] amino] 1-(1H-tétrazol-5-yl) 2-azétidinone.

On chauffe à 50 °C pendant 15 minutes un mélange de 150 mg de produit obtenu au stade a) dans 2 $cm^3$ d'acide formique à 66%. On laisse revenir à température ambiante, filtre le triphényl carbinol obtenu, le lave avec 2 $cm^3$ d'acide formique à 66%. Le filtrat est concentré sous pression réduite. On reprend avec 10 $cm^3$ d'éthanol absolu, évapore et répète l'opération 3 fois. On reprend le résidu dans 1 $cm^3$ d'acétone, essore, lave à l'éther, sèche sous pression réduite à 60 °C pendant une heure et obtient 48 mg de produit attendu (F > 260 °C) (Rf: 0,1 éluant acétate d'éthyle, éthanol, eau 70/20/10).

Le chlorhydrate de 3-amino 1-(1H-tètrazol-5-yl) 2-azètidinone utilise au départ de l'exemple 3 a été obtenu comme suit:

a) (2S) 2-tritylamino 3-hydroxy N(2-phényl méthyl 2H-tétrazol-5-yl) propionamide.

On porte au reflux pendant 15 minutes un mélange de 25 $cm^3$ de triméthylaluminium 2M dans l'hexane, 3,5 g de 2-phényl méthyl 5-amino 2H-tétrazole et 100 $cm^3$ de tétrahydrofuranne puis ajoute 6,6 g de lactone de la N-trityl L-sérine. On porte 3 heures au reflux, refroidit à 10 °C et ajoute, très lentement et en agitant, 10 $cm^3$ d'éthanol. On évapore à sec et reprend par 300 $cm^3$ de chlorure de méthylène et 200 $cm^3$ d'eau amenée à pH 4 avec de l'acide chlorhydrique. On décante, filtre, lave la phase organique avec 200 $cm^3$ d'acide chlorhydrique 0,05M. On sèche, filtre et évapore à sec les solvants. On reprend par 20 $cm^3$ d'acétate d'éthyle et glace. On essore les cristaux formés, les lave à l'éther et sèche sous pression réduite. On obtient 5,3 g de produit attendu (F = 202–204 °C).

b) (2S) 2-amino 3-hydroxy N-(2-phényl méthyl) 2H-tétrazol-5-yl) propionamide.

On agite à température ambiante pendant 15 minutes un mélange de 3,5 g de produit obtenu ci-dessus, 25 $cm^3$ de chlorure de méthylène et 1,7 $cm^3$ d'acide chlorhydrique 8M dans le méthanol. On essore, lave avec 2 fois 10 $cm^3$ de chlorure de méthylène puis 2 fois 20 $cm^3$ d'éther. On sèche sous pression réduite et obtient 1,94 g de produit attendu.

c) (2S) 2-(phényl méthyl oxy carbonyl amino) 3-hydroxy N-(2-phényl méthyl 2H-tétrazol-5-yl) propionamide.

On agite fortement un mélange de 1,94 g de produit obtenu ci-dessus, 25 $cm^3$ d'eau 2 g de carbonate acide de sodium et 25 $cm^3$ de chlorure de méthylène puis ajoute 2,5 $cm^3$ de chloroformiate de benzyle à 50% dans le toluène. On agite à température ambiante dans le toluène, décante, extrait avec une fois 25 $cm^3$ de chlorure de méthylène. La phase organique est lavée avec 20 $cm^3$ d'acide chlorhydrique 0,05M puis séchée. On évapore le solvant sous pression réduite et obtient 2,45 g de produit attendu.

d) (3S) 1-[2-(phényl méthyl 2H-tétrazol-5-yl) 3-(phényl méthoxy carbonyl amino) 2-azétidinone.

On ajoute à 10 °C, 6 $cm^3$ d'une solution molaire dans le tétrahydrofuranne de diazodicarboxylate de diéthyle à un mélange de 2 g de produit obtenu en c) et 1,75 g de triphényl phosphine dans 50 $cm^3$ de tétrahydrofuranne. On agite 15 minutes à 10 °C, évapore sous pression réduite, chromatographie sur silice avec un mélange chlorure de méthylène-acétate d'éthyle 85:15. On obtient 1,3 g de produit attendu après empâtage à l'éther (F $\simeq$ 150 °C).

Analyse: $C_{19}H_{18}N_6O_3$

| | | | |
|---|---|---|---|
| Calculé | C% 60,3 | H% 4,79 | N% 22,21 |
| Trouvé | C% 60,3 | H% 4,8 | N% 22,0 |

e) chlorhydrate de 3-amino 1-(1H-tétrazol-5-yl) 2-azétidinone.

On porte au reflux un mélange de 1,3 g de produit obtenu ci-dessus dans 80 $cm^3$ d'éthanol absolu puis ajoute 1 $cm^3$ d'acide chlorhydrique 8M dans le méthanol puis 800 mg de palladium sur charbon à 18%. On fait passer un courant d'hydrogène jusqu'à hydrogénation complète. On filtre, évapore à sec et obtient 660 mg de produit.

Exemple 4
(3SR,4RS) 3-[[2-(2-aminothiazol-4-yl) 2-[(difluorométhoxy) imino] acétyl] amino] 4-méthyl 1-(1-H-tétrazol-5-yl) 2-azétidinone, isomère syn.

A) Chlorhydrate de (3SR,4RS) 3-amino 4-méthyl 1-(1-H-tétrazol-5-yl) 2-azétidinone.

On opère comme au stade a de l'exemple 1 en utilisant 890 mg de (3SR,4RS) chlorhydrate de 3-amino 4-méthyl 1-[2-(phénylméthyl) 2-H-tétrazol-5-yl] 2-azétidinone, 20 $cm^3$ d'éthanol à 70° et 750 mg de palladium sur charbon à 18%. Après 15 minutes d'hydrogénation, on obtient le produit attendu que l'on utilise aussitôt.

B) Anhydride mixte paratoluène sulfonique 2-(2-tritylamino thiazol-4-yl) 2-(difluorométhoxy) imino acétique isomère syn.

On opère comme au stade b) de l'exemple 1 en utilisant 325 mg de chlorure de paratoluène sulfonyle et 815 mg d'acide 2-(2-tritylamino thiazol-4-yl) 2-difluorométhoxyimino acétique isomère syn décrit dans le brevet français 2 461 713 dans 25 $cm^3$ d'acétone et 0,25 $cm^3$ de triéthylamine.

C) (3SR,4RS) 3-[[2-[2-tritylamino (thiazol-4-yl) 2-difluorométhoxyimino] acétyl] amino] 4-méthyl 1-(1-H-tétrazol-5-yl) 2-azétidinone isomère syn.

On ajoute la suspension obtenue au stade B) à la solution du produit obtenu au stade A) dans 45 $cm^3$ d'acétonitrile et 0,75 $cm^3$ de triéthylamine. On agite 2 heures à température ambiante, ajoute 0,35 $cm^3$ d'acide acétique et concentre à sec sous pression réduite. On reprend le résidu à l'acétate d'éthyle, ajoute 15 $cm^3$ d'une solution de bicarbonate de soude 1M, essore le précipité, le reprend à l'eau, acidifie à pH 3–4 à l'aide d'acide chlorhydrique 2N, extrait à l'acétate d'éthyle, lave à l'aide d'une solution aqueuse de chlorure de sodium, sèche, concentre à sec sous pression réduite, reprend le résidu à l'acétate d'éthyle, glace, essore, lave les cristaux à l'acétate d'éthyle puis à l'éther, sèche sous pression réduite et obtient 308 mg de produit attendu. F $\simeq$ 220 °C.

D) (3SR,4RS) 2-[[2-(2-aminothiazol-4-yl) 2-(difluorométhoxyimino acétyl] amino] 4-méthyl 1-(1-H-tétrazol-5-yl) 2-azétidinone isomère syn.

On chauffre 15 minutes à 50 °C, 290 mg de produit obtenu au stade précédent dans 2,15 $cm^3$ d'acide formique à 66%. On essore le triphénylcarbinol précipité, le lave avec 2 $cm^3$ d'acide formique à 66% et concentre à sec le filtrat sous pression réduite. On reprend le résidu à l'éthanol et concentre de nouveau sous pression réduite. On renouvelle cette opération puis empâte à l'acétone, essore, lave à l'acétone puis à l'éther et sèche sous pression réduite et obtient 150 mg de produit attendu. F > 260 °C.

Analyse: $C_{11}$ $H_{11}$ $N_9$ $O_9$ $SF_2$ = 387,33

Calculé:

C% 34,11 H% 2,86 F% 9,81 N% 32,55 S% 8,28

Trouvé:

C% 34,1 H% 3,0 F% 9,6 N% 32,1 S% 8,2

Spectre de RMN (DMSO)

1,39–1,45 ppm: $C\underline{H}_3$–CH–

4,6 ppm: $H_4$

5,47 à 5,63 ppm: $H_3$

7,04 ppm: $H_5$ du thiazole

6,4–7,15–7,95 ppm: CH $F_2$

7,37 ppm: $NH_2$

9,7–9,8 ppm: NH–CO

Exemple 5

(3SR,4RS) 3-[[2-(2-aminothiazol-4-yl) 2-[(1-propényloxy) imino] acétyl] amino] 4-méthyl 1-(1-H-tétrazol-5-yl) 2-azétidinone isomère syn.

A) Anhydride mixte paratoluène sulfonique 2-(2-tritylamino thiazol-4-yl) 2-(1-propényloxy) imino acétique isomère syn.

On opère comme au stade b) de l'exemple 1 en utilisant 265 mg de chlorure de paratoluène sulfonyle et 630 mg d'acide 2-(2-tritylamino thiazol-4-yl) 2-(1-propényloxy) imino acétique isomère syn. (décrit dans le brevet français 2 361 893) dans 8 $cm^3$ d'acétone et 0,2 $cm^3$ de triéthylamine.

B) (3SR,4RS) 3-[[2-[2-tritylamino (thiazol-4-yl) 2-(1-propényloxy) imino] acétyl] amino] 4-méthyl 1-(1-H-tétrazol-5-yl) 2-azétidinone isomère syn.

On opère comme au stade c) de l'exemple 1 en utilisant la suspension obtenue au stade A) précédent et le chlorhydrate de (3SR,4RS) 3-amino 4-méthyl 1-(1-H-tétrazol-5-yl) 2-azétidinone préparé selon le stade A) de l'exemple 4. On obtient un premier jet de 400 mg de produit attendu puis on concentre les liqueurs mères, reprend le résidu à l'acétate d'éthyle, extrait avec une solution aqueuse de bicarbonate de soude, décante, acidifie la phase aqueuse, extrait à l'acétate d'éthyle, lave à l'eau, sèche et concentre à sec sous pression réduite. On recueille 140 mg de produit identique au premier jet. F $\simeq$ 220 °C.

C) (3SR,4RS) 3-[[2-[(2-(2-aminothiazol-4-yl) 2-(1-propényloxy) imino] acétyl] amino] 4-méthyl 1-(1-H-tétrazol-5-yl) 2-azétidinone isomère syn.

On chauffe 15 mn à 50 °C 380 mg du produit obtenu au stade B dans 2,8 $cm^3$ d'acide formique à 66%, laisse revenir à température ambiante, élimine le précipité par filtration, concentre à sec le filtrat sous pression réduite, reprend le résidu à l'eau, essore, lave à l'eau, sèche à 50 °C sous pression réduite et recueille 185 mg de produit attendu. F > 260 °C.

Analyse: $C_{13}$ $H_{15}$ $N_9$ $O_3$ S = 377,37

Calculé:

C% 41,37 H% 4,01 N% 33,40 S% 8,50

Trouvé:

C% 41,3 H% 4,0 N% 33,3 S% 8,5

Spectre de RMN (DMSO)

1,38–1,45 ppm: $C\underline{H}_3$–CH

4,58–4,64 ppm: N–$OCH_2$

4,61 ppm: $H_4$

5,14 à 5,42 ppm: =$CH_2$

5,5 à 5,64 ppm: $H_3$

5,77 à 6,14 ppm: $C\underline{H}$=$CH_2$

6,8 ppm: $H_5$ du thiazol

7,24 ppm: $NH_2$

9,4–9,49 ppm: NHCO

Exemple 6

(3SR,4RS) 3-[[2-(2-aminothiazol-4-yl) 2-[(carboxyméthoxy) imino] acétyl] amino] 4-méthyl 1-(1-H-tétrazol-5-yl) 2-azétidinone isomère syn.

A) Anhydride mixte paratoluène sulfonique 2-(2-tritylaminothiazol-4-yl) 2-[(terbutoxycarbonylméthyl) oxy] imino acétique isomère syn.

On opère comme à l'exemple 1 stade b) à partir de 480 mg de chlorure de paratoluène sulfonyle et 1,38 g d'acide 2-(2-tritylamino thiazol-4-yl) 2-[(terbutoxycarbonyl méthyl) oxy] imino acétique isomère syn préparé dans le brevet français 2 445 830 dans 15 $cm^3$ d'acétone et 0,36 $cm^3$ de triéthylamine.

B) (3SR,4RS) 3-[[2-(2-tritylamino thiazol-4-yl) 2-[(terbutoxy carbonyl méthyl) oxy] imino] acétyl] amino 4-méthyl 1-(1-H-tétrazol-5-yl) 2-azétidinone isomère syn.

On opère comme à l'exemple 4 stade C) en utilisant la suspension utilisée au stade A) précédent et le chlorhydrate de (3SR,4RS) 3-amino 4-méthyl 1-(1-H-tétrazol-5-yl) 2-azétidinone préparé à l'exemple 4 stade A). On recueille 850 mg de produit attendu. F ≃ 270 °C.

C) (3SR,4RS) 3-[[2-(2-aminothiazol-4-yl) 2-[(carboxyméthoxy) imino] acétyl] amino] 4-méthyl 1-(1-H-tétrazol-5-yl) 2-azétidinone isomère syn.

On agite 5 minutes à température ambiante une solution de 830 mg de produit obtenu au stade B) précédent dans 2,5 $cm^3$ d'acide trifluoroacétique, concentre à sec sous pression réduite, reprend le résidu avec 5 $cm^3$ d'acide formique à 66%, chauffe 15 minutes à 50 °C, refroidit et élimine le précipité par filtration. Par 3 fois, on concentre à sec le filtrat sous pression réduite en reprenant le résidu à l'éthanol puis à l'acétone. On amorce la cristallisation, glace, essore les cristaux, les lave à l'acétone puis à l'éther, les sèche à 50 °C sous pression réduite et recueille 240 mg de produit attendu. F > 260 °C.

Spectre de RMN (DMSO)
1,39–1,45 ppm: $\underline{CH_3}$–CH
≃ 4,58 ppm:$H_4$
5,48 à 5,63 ppm: $H_3$
4,62 ppm: $OCH_2$–CO
6,81 ppm: $H_5$ du thiazole
7,25 ppm: $NH_2$
9,38–9,48 ppm: NHCO

Exemple 7

(3SR,4RS) 3-[[2-(2-aminothiazol-4-yl) 2-[(1-carboxy 1-méthyléthoxy) imino] acétyl] amino] 4-méthyl 1-(tétrazol-5-yl) 2-azétidinone isomère syn.

A) Anhydride mixte paratoluène sulfonique 2-(2-tritylamino thiazol-4-yl) 2-(terbutoxycarbonyl 1-méthyléthoxy) imino acétique isomère syn.

On opère comme à l'exemple 1 stade b) à partir de 325 mg de chlorure de paratoluène sulfonyle et 970 mg d'acide 2-(2-tritylamino thiazol-4-yl) 2-[(1-terbutoxycarbonyl 1-méthyléthyl) oxy] imino acétique isomère syn préparé dans le brevet français n° 2 421 906 dans 17,5 $cm^3$ d'acétone et 0,25 $cm^3$ de triéthylamine.

B) (3SR,4RS) 3-[[2-(2-tritylamino thiazol-4-yl) 2-(1-terbutoxy carbonyl 1-méthyléthoxy) imino] acétyl] amino 4-méthyl 1-(tétrazol-5-yl) 2-azétidinone isomère syn.

On opère comme à l'exemple 4 stade C) en utilisant la suspension obtenue au stade A) précédent et le chlorhydrate de (3SR,4RS) 3-amino 4-méthyl 1-(1-H-tétrazol-5-yl) 2-azétidinone préparé à l'exemple 4 stade A). On recueille 1,07 g de produit attendu. F ≃ 220 °C.

C) (3SR,4RS) 3-[[2-(2-aminothiazol-4-yl) 2-[(1-carboxy 1-méthyl éthoxy) imino] acétyl] amino] 4-méthyl 1-(tétrazol-5-yl) 2-azétidinone isomère syn.

On opère comme à l'exemple 6 stade C) à partir de 950 mg du produit obtenu au stade B) précédent. On purifie le produit brut par chromatographie sur silice en éluant à l'eau. On concentre à sec, reprend le résidu au méthanol, filtre, élimine le solvant sous pression réduite, cristallise dans l'acétone, sèche à 50 °C sous pression réduite et recueille 157 mg de produit attendu. F > 260 °C.

Spectre de RMN (DMSO)
1,35–1,42 ppm: $\underline{CH_3}$–CH
1,42 ppm: $(CH_3)_2$
4,28 ppm: $H_4$
5,28 à 5,43 ppm: $H_3$
6,75 ppm: $H_5$ du thiazole
7,15 ppm: $NH_2$
11,34–11,44 ppm: NHCO

Exemple 8

(3SR,4RS) 3-[[2-(2-aminothiazol-4-yl) 2-(méthoxyimino) acétyl] amino] 4-éthyl 1-(1-H-tétrazol-5-yl) 2-azétidinone

A) Chlorhydrate de (3SR,4RS) 3-amino 4-éthyl 1-(1-H-tétrazol-5-yl) 2-azétidinone.

On opère comme à l'exemple 1 stade a au départ de 2 g de (3SR,4RS) chlorhydrate de 3-amino 4-éthyl 1-[2-(phénylméthyl) 2H-tétrazol-5-yl] azétidinone mais en hydrogénant 2 fois successivement la solution.

B) (3SR,4RS) 3-[[2-(2-tritylamino thiazol-4-yl) 2-méthoxyimino acétyl] amino] 4-éthyl 1-(1-H-tétrazol-5-yl) 2-azétidinone.

On ajoute 65 $cm^3$ d'acétonitrile et 2,85 $cm^3$ de triéthylamine au produit obtenu au stade A) puis une suspension d'anhydride mixte paratoluène sulfonique 2-(2-tritylamino 4-thiazolyl) 2-méthoxyimino acétique isomère syn préparé comme au stade b) de l'exemple 1 à partir de 2,9 g d'acide 2-(2-tritylamino thiazol-4-yl) 2-méthoxyimino acétique isomère syn et 1,24 g de chlorure de paratoluène sulfonyle. On agite 1 heure 30 minutes à température ambiante, ajoute 0,4 $cm^3$ d'acide acétique et continue la réaction comme au stade b) de l'exemple 2. On obtient 1,86 g de produit brut que l'on recristallise dans l'acétone. On recueille 1 g de produit attendu. F ≃ 260 °C – Rf = 0,27 (éluant: acétate d'éthyle-ethanol-eau 7-2-1).

C) (3SR,4RS) 3-[[2-(2-aminothiazol-4-yl) 2-méthoxyimino) acétyl] amino] 4-éthyl 1-(1-H-tétrazol-5-yl) 2-azétidinone.

On chauffe 15 minutes à 50 °C 1 g de produit obtenu au stade précédent dans 5 $cm^3$ d'acide formique à 66%. On laisse revenir à température ambiante, filtre, concentre le filtrat, ajoute 25 $cm^3$ d'eau, glace, filtre, concentre le filtrat à sec sous pression réduite, reprend le résidu dans 10 $cm^3$ d'acétone, filtre, concentre le filtrat à 3 $cm^3$ et laisse cristalliser. On essore les cristaux, les lave à l'acétone, les sèche sous pression réduite et

obtient 270 mg de produit attendu. F ≃ 210 °C. Rf = 0,15 (éluant: acétate d'éthyle-éthanol-eau 7-2-1).

Spectre de RMN (DMSO)
0,9–0,98–1,05 ppm et 1,67 à 2,22 ppm: Ethyle
3,87 ppm: N–$OHC_3$
4,22 ppm: $H_4$
4,87 à 4,99 ppm: $H_3$
6,74 ppm: $H_5$ du thiazole
7,24 ppm: $NH_2$
9,3–9,4 ppm: NH–CO

Le (3SR,4RS) chlorhydrate de 3-amino 4-éthyl 1-[2-phényl méthyl 2H-tétrazol-5-yl] 2-azétidinone utilisé au départ de l'exemple a été préparé comme suit:

a) N-(1-méthoxypropyl) 2-(phénylméthyl) 2-H-tétrazol-5-amine.

On opère comme au stade a) de la préparation suivant l'exemple 1 au départ de 2 g de 2-benzyl 5-aminotétrazole et de 2 $cm^3$ de propionaldéhyde. On obtient 2,72 g de produit attendu. Rf = 0,57 (éluant: éther).

b) N-propylidène 2-(phénylméthyl) 2-H-tétrazol-5-amine.

On opère comme au stade b) de la préparation de l'exemple 1 au départ de 20 g de produit obtenu au stade a) ci-dessus. On obtient 17,5 g de produit attendu.

c) (2SR,3SR) et (2SR,3RS) 2-amino 2-[2-(phénylméthyl) 2-H-tétrazol-5-yl amino] pentanoate de méthyle.

On opère comme à la préparation de l'exemple 1 stade c) en utilisant le produit obtenu au stade b) précédent. On recueille 14,07 g de produit trans – F ≃ 60 °C – et 6,2 g de produit cis – F ≃ 105 °C –.

d) (2SR,3SR) 2-tritylamino 3-[2-(phénylméthyl) 2-H-tétrazol-5-yl amino] pentanoate de méthyle.

On opère comme indiqué au stade d) de la préparation suivant l'exemple 1 à partir de 21 g de produit trans, préparé comme au stade c) ci-dessus. On obtient 29,8 g de produit attendu. F ≃ 190 °C.

e) (2SR,3SR) acide 2-tritylamino 3-[2-(phénylméthyl) 2-H-tétrazol-5-yl amino] pentanoïque.

On opère comme au stade e) de la préparation suivant l'exemple 1 en utilisant 29,8 g de l'ester préparé ci-dessus. On obtient 26,3 g du produit attendu. F ≃ 175 °C.

f) (3SR,4SR) 4-éthyl 1-[2-(phénylméthyl) 2-H-tétrazol-5-yl 3-(tritylamino)]2-azétidinone.

On opère comme au stade f) de la préparation suivant l'exemple 1 au départ de 5,18 g d'acide préparé ci-dessus. On recueille 2,55 g de produit attendu. (Rf = 0,42 – éluant: chlorure de méthylène-éther isopropylique 95-5).

g) (3SR,4SR) chlorhydrate de 3-amino 4-éthyl 1-[2-(phénylméthyl) 2-H-tétrazol-5-yl] 2-azétidinone.

On opère comme au stade g) de la préparation de l'exemple 1 en utilisant 2,4 g du dérivé tritylé préparé ci-dessus. On obtient 1,15 g de produit attendu. F = 202 °C. (Rf = 0,52 – éluant: acétate d'éthyle-éthanol-eau 7-2-1).

Exemple 9
(3SR,4RS) 3-[[2-(2-aminothiazol-4-yl) 2-méthoxyimino] acétyl amino] 4-éthyl 1-(1-H-tétrazol-5-yl) azétidinone.

A) Chlorhydrate de (3SR,4RS) 3-amino 4-éthyl 1-(1-H-tétrazol-5-yl) 2-azétidinone.

On opère comme à l'exemple 1 stade a) au départ de 1,08 g de (3SR,4RS) chlorhydrate de 3-amino 4-éthyl 1-[2-(phénylméthyl) 2-H-tétrazol-5-yl 2-azétidinone en hydrogénant 2 fois 15 minutes la solution. Après filtration, on concentre à sec en reprenant le résidu 3 fois à l'éthanol et 2 fois à l'acétonitrile.

B) (3SR,4RS) 3-[[2-(tritylamino thiazol-4-yl) 2-méthoxyimino acétyl] amino] 4-éthyl 1-(1-H-tétrazol-5-yl) 2-azétidinone.

On opère comme au stade B) de l'exemple 8 en utilisant le produit obtenu au stade A) précédent et une suspension d'anhydride mixte paratoluène sulfonique 2-(2-tritylamino thiazol-4-yl) 2-méthoxyimino acétique isomère syn préparé comme au stade b) de l'exemple 1 à partir de 1,56 g d'acide 2-(2-tritylamino thiazol-4-yl) 2-méthoxyimino acétique isomère syn et 670 mg de chlorure de paratoluène sulfonyle. On récupère 2 g de produit brut que l'on dissout dans l'acétone et resolidifie par l'éther. On obtient 1,18 g de produit attendu. F = 210 °C.

C) (3SR,4RS) 3-[[2-(2-aminothiazol-4-yl) 2-(méthoxyimino) acétyl] amino] 4-éthyl 1-(1-H-tétrazol-5-yl) 2-azétidinone.

On opère comme au stade d) de l'exemple 1 au départ de 870 mg de produit obtenu au stade B) précédent. Les cristaux sont lavés à l'acétone puis à l'éther. On obtient 370 mg de produit attendu. F ≃ 260 °C. (décomposition) Rf = 0,2 (éluant: acétate d'éthyle-éthanol-eau 7-2-1).

Spectre de RMN (DMSO)
0,88–0,95–1,03 ppm: $CH_3$ de l'éthyle
3,85 ppm: N–$OCH_3$
4,33 ppm: $H_4$
5,47 à 5,63 ppm: $H_3$
6,78 ppm: $H_5$ du thiazole
7,25 ppm: $NH_2$
9,43–9,53 ppm: NH–CO

Le (3SR,4RS) chlorhydrate de 3-amino 4-éthyl 1-[2-(phénylméthyl) 2-H-tétrazol-5-yl] 2-azétidinone utilisé au départ de l'exemple 9 a été préparé comme suit:

a) (2SR,3RS) 2-tritylamino 3-[2-(phénylméthyl) 2-H-tétrazol-5-yl] amino pentanoate de méthyle.

On opère comme indiqué au stade d) de la préparation suivant l'exemple 1 au départ de 14 g de produit cis obtenu comme au stade c) de la préparation suivant l'exemple 8. On obtient 19,87 g de produit attendu. F = 140 °C. Rf = 0,17 (éluant: hexane-acétate d'éthyle 7-3).

b) Acide (2SR,3RS) 2-tritylamino 3-[2-(phénylméthyl) 2-H-tétrazol-5-yl] amino pentanoïque.

On opère comme indiqué au stade c) de la préparation suivant l'exemple 1 au départ de

19,8 g du produit obtenu au stade précédent. On obtient 4,4 g de produit attendu. F ≃ 205 °C. Rf = 0,27 (éluant: acétate d'éthyle-hexane 7-3).

c) (3SR,4RS) 4-éthyl 1-[2-(phénylméthyl) 2-H-tétrazol-5-yl] 3-tritylamino 2-azétidinone.

On opère comme au stade f) de la préparation suivant l'exemple 1 en utilisant 4,75 g d'acide préparé comme ci-dessus. On obtient 1,82 g de produit attendu. Rf = 0,4 (éluant: chlorure de méthylène-éther isopropylique 95-5).

d) (3SR,4SR) chlorhydrate de 3-amino 4-éthyl 1-[2-(phénylméthyl) 2-H-tétrazol-5-yl] 2-azétidinone.

On opère comme au stade g) de la preparation suivant l'exemple 1 à partir de 1,8 g de produit obtenu ci-dessus. On obtient 1,08 g de produit attendu. Rf = 0,66 (éluant: acétate d'éthyle-éthanol-eau 7-2-1).

Exemple 10

Acide (3SR,4RS) 5-[3-[[2-(2-aminothiazol-4-yl) 2-méthoximino acétyl] amino] 4-méthyl 2-oxo 1-azétidinyl] 2-H-tétrazol-2-acétique.

A) (3SR,4RS) 5-[3-[[2-(2-aminothiazol-4-yl) 2-méthoxyimino acétyl] amino] 4-méthyl 2-oxo 1-azétidinyl] 2-H-tétrazol-2-acétate de terbutyle.

On dissout à 50 °C, 2,27 g de (3SR,4RS) 3-[[2-(2-tritylamino) thazol-4-yl] 2-méthoxyimino acétyl] amino] 4-méthyl 1-(1-H-tétrazol-5-yl) 2-azétidinone dans 35 $cm^3$ de diméthylformamide, laisse revenir à température ambiante et ajoute 170 mg d'hydrure de sodium à 55% dans l'huile, agite 15 minutes, ajoute 1 $cm^3$ de bromoacétate de terbutyle et laisse 16 heures sous agitation. On verse le mélange réactionnel dans 250 $cm^3$ d'une solution aqueuse de phosphate monosodique 0,1M, extrait à l'éther, lave à l'eau, sèche et concentre à sec sous pression réduite. On chromatographie le résidu sur silice (éluant: chlorure de méthylène-acétone 9-1) et obtient 1,3 g de produit attendu et 130 mg de dérivé correspondant substitué en 1.

B) Acide (3SR,4RS) 5-[3-[[2-(2-aminothiazol-4-yl) 2-méthoxyimino acétyl] amino] 4-méthyl 2-oxo 1-azétidinyl] 2-H-tétrazol-2-acétique.

On agite 10 minutes 960 mg de produit obtenu au stade A) dans 3 $cm^3$ d'acide trifluoroacétique, concentre à sec sous pression réduite et reprend le résidu dans 6 $cm^3$ d'acide formique à 66%, chauffe 15 minutes à 50 °C et laisse revenir à température ambiante. On essore l'insoluble, concentre le filtrat sous pression réduite jusqu'à cristallisation, ajoute de l'eau, agite 30 minutes, essore, lave à l'eau et sèche sous pression réduite. Après recristallisation dans l'éthanol, on recueille le produit attendu. F ≃ 240 °C. Rf = 0,14 (éluant: acétate d'éthyle-éthanol-eau 7-2-1).

Spectre de RMN (DMSO)

1,38–1,44 ppm: $\underline{CH_3}$–CH

3,89 ppm: $OCH_3$

4,55 ppm: $CH_3$–$\underline{CH}$

5,55 ppm: $H_3$

5,67 ppm: N–$\underline{CH_2}$–COOH

6,83 ppm: $H_5$ du thazole

9,41–9,51 ppm: NH–CO

Exemple 11

(3SR,4SR) 3-[[2-(2-aminothiazol-4-yl) 2-méthoxyimino acétyl] amino] 4-(fluorométhyl) 1-(1-H-tétrazol-5-yl) 2-azétidinone.

A) (3SR,4SR) 3-tritylamino 4-fluorométhyl 2-azétidinone.

On agite 3 heures à température ambiante 2,22 g de chlorhydrate de (3SR,4SR) 3-amino 4-fluorométhyl 2-azétidinone préparé comme dans le brevet belge 894 785, dans 60 $cm^3$ de chlorure de méthylène, 4,2 $cm^3$ de triéthylamine et 4,1 g de chlorure de trityle. On ajoute 100 $cm^3$ de chlorure de méthylène, lave à l'eau, sèche et concentre à sec sous pression réduite. On reprend les cristaux dans l'éther isopropylique, essore, lave à l'éther isopropylique puis au pentane, sèche sous pression réduite à 50 °C et obtient 4,36 g de produit attendu. F = 200°–202 °C (décomposition).

B) (3SR,4SR) 1-(1-benzyl tétrazol-5-yl) 3-tritylamino 4-fluorométhyl 2-azétidinone.

On dissout à 50 °C, 3,6 g de produit obtenu au stade précédent dans 60 $cm^3$ de tétrahydrofuranne puis refroidit la solution à −40 °C. On ajoute en 3 minutes, 15 $cm^3$ d'une solution 0,66M de bis triméthylsilyl amidure de sodium dans le tétrahydrofuranne, agite 5 minutes à −30 °C puis refroidit à −60 °C et obtient une suspension que l'on verse lentement dans une solution de 2 g de 5-fluoro 1-benzyltétrazole dans 900 $cm^3$ de toluène. On agite 30 minutes à température ambiante, ajoute 50 $cm^3$ d'une solution aqueuse de phosphate monosodique 1M, décante, sèche et concentre à sec sous pression réduite. On chromatographie le résidu sur silice (éluant: benzène-acétate d'éthyle 97-3). On isole 2,6 g de produit brut que l'on cristallise dans l'éther. On recueille 2,18 g de produit attendu. Rf = 0,4 (éluant: benzène-acétate d'éthyle 95-5).

C) Chlorhydrate de (3SR,4SR) 1-(1-benzyl-tétrazol-5-yl) 3-amino 4-fluorométhyl 2-azétidinone.

On agite 30 minutes à température ambiante 2,18 g de produit précédent dans 60 $cm^3$ de chlorure de méthylène et 1,3 $cm^3$ d'une solution 5M d'acide chlorhydrique dans une solution méthanol-eau (97-3). On essore le précipité, le lave au chlorure de méthylène puis à l'éther, le sèche et obtient 1,17 g de produit attendu. F ≃ 180°–200 °C (décomposition).

D) Chlorhydrate de (3SR,4SR) 3-amino 4-fluorométhyl 1-(1-H-tétrazol-5-yl) 2-azétidinone.

On hydrogène pendant 30 minutes à 40 °C le mélange de 1,17 g du chlorhydrate obtenu au stade précédent dans 50 $cm^3$ de méthanol additionné de 160 mg de palladium sur charbon à 18%. On filtre, concentre à sec le filtrat sous pression réduite et obtient 836 mg de produit attendu. Rf = 0,1 (éluant: acétate d'éthyle-éthanol-eau 7-2-1).

E) (3SR,4SR) 3-[[2-[tritylamino (thiazol-4-yl) 2-méthoxyimino] acétyl] amino] 4-fluorométhyl 1-(1-H-tétrazol-5-yl) 2-azétidinone.

On dissout 102 mg de produit obtenu au stade précédent dans 3 $cm^3$ diacétonitrile et 0,2 $cm^3$ de triéthylamine; on verse lentement cette solution dans une suspension d'anhydride mixte paratoluène sulfonique 2-(2-tritylamino thiazol-4-yl) 2-méthoxy acétique isomère syn préparé comme au stade b) de l'exemple 1 à partir de 203 mg d'acide 2-(2-tritylamino thiazol-4-yl) 2-méthoxyimino acétique isomère syn et 88 mg de chlorure de paratoluène sulfonyle. On agite 30 minutes, ajoute 0,05 $cm^3$ d'acide acétique, concentre à 2 $cm^3$ sous pression réduite, reprend à l'acétate d'éthyle, lave à l'eau, sèche et concentre à sec. On reprend de nouveau le résidu à l'acétate d'éthyle, glace, essore et sèche les cristaux. On obtient 186 mg de produit attendu. F = 200 °C.

F) (3SR,4SR) 3-[[2-(2-aminothiazol-4-yl) 2-méthoxyimino acétyl] amino] 4-(fluorométhyl) 1-(1-H-tétrazol-5-yl) 2-azétidinone.

On chauffe 15 minutes à 50 °C, 202 mg de produit obtenu comme précédemment dans 4 $cm^3$ d'acide formique à 66%. On glace, essore, et concentre le filtrat sous pression réduite. On reprend à l'eau et essore les cristaux qu'on lave à l'eau, à l'acétone puis à l'éther. On isole 88 mg de produit attendu. F ≃ 250 °C. Rf = 0,2 (éluant: acétate d'éthyle-éthanol-eau 7-2-1).

Spectre de RMN (DMSO)
3,86 ppm: $OCH_3$
de 5,60 à 5,74 ppm: $H_3$
6,78 ppm: $H_5$ du thiazole
7,22 ppm: $NH_2$
9,44–9,53 ppm: NH–CO

Le 5-fluoro 1-benzyltétrazole utilisé au départ de l'exemple 11 a été préparé comme suit:

a) N-benzyl semi-carbazide.

On refroidit à −5 °C ± 2°, 9,7 $cm^3$ d'hydrate d'hydrazine dans 50 $cm^3$ de tétrahydrofuranne et 50 $cm^3$ d'éther sous atmosphère inerte et ajoute 26,6 g d'isocyanate de benzyle dans 100 $cm^3$ d'éther. On agite pendant 16 heures en laissant revenir à température ambiante, essore le précipité, le lave à l'éther, le reprend par 250 $cm^3$ de méthanol, chauffe au reflux, filtre, concentre le filtrat à 50 $cm^3$ sous pression réduite, ajoute de l'acétate d'éthyle en éliminant le méthanol par distillation, concentre à 100 $cm^3$, glace, essore les cristaux, les lave à l'acétate d'éthyle et sèche sous pression réduite. On obtient 25,56 g de produit attendu. F = 115 °C.

b) Azide de l'acide N-(phénylméthyl) carbamique.

On ajoute 8,19 g de nitrite de sodium à une solution de 19,5 g de l'amide obtenu au stade précédent dans 331,5 $cm^3$ d'eau et 58,5 $cm^3$ d'acide chlorhydrique 2N, maintenue à une température inférieure à 25 °C. On agite 1 heure, essore le précipité, le lave à l'eau, le sèche et obtient 18,9 g de produit attendu. F ≃ 85 °C.

c) 5-chloro 1-benzyltétrazole.

On ajoute lentement sous agitation une solution de 23,5 g de pentachlorure de phosphore dans 250 $cm^3$ de trichloroéthylène, dans une suspension de 15,8 g du produit obtenu au stade précédent dans 150 $cm^3$ de trichloroéthylène. On chauffe 1 heure au reflux, refroidit et verse la solution dans une solution aqueuse de bicarbonate de sodium, décante, extrait au chlorure de méthylène, lave à l'aide d'une solution aqueuse de chlorure de sodium, sèche et concentre à sec sous pression réduite. On chromatographie le résidu sur silice (éluant: hexane-acétate d'éthyle 7-3) et recueille 6,85 g de produit attendu.

d) 5-fluoro 1-benzyltétrazole.

On mélange dans 3 litres d'acétonitrile 16 g d'éther couronne (18-6) et 20 g de fluorure de potassium. On distille 500 $cm^3$ de solvant sous courant d'argon et ajoute 7,9 g de produit obtenu comme au stade précédent. On chauffe au reflux pendant 65 heures, filtre et concentre à sec sous pression réduite. On reprend le résidu dans du benzène, concentre à sec, chromatographie le résidu sur silice (éluant: hexane-acétate d'éthyle 7-3) et obtient 5,73 g de produit attendu. Rf = 0,23 (éluant: hexane-acétate d'éthyle 7-3).

Exemple 12

(3S) 3-[[2-(2-aminothiazol-4-yl) 2-méthoxyimino acétyl] amino] 1-(3-trifluorométhyl 1H-1,2,4-triazol-5-yl) 2-azétidinone.

On opère comme au stade a) de l'exemple 3 à partir du chlorhydrate de (3S) 3-amino 1-(1-H-1,2,4-triazol-5-yl) 2-azétidinone et de l'anhydride mixte paratoluène sulfonique 2-(2-tritylamino thiazol-4-yl) 2-méthoxyimino acétique isomère syn, préparé comme au stade b) de l'exemple 1. On poursuit la synthèse comme au stade b) de l'exemple 3 et obtient le produit attendu. Rf = 0,15–0,2 (éluant: chlorure de méthylène-acétone-méthanol 75-17-8).

Spectre de RMN (DMSO)
3,85 ppm: (s) $OCH_3$
3,75 à 4,33 ppm: (m) $H_4$
−5,19 ppm: (m) $H_3$
6,78 ppm: (s) $H_5$ du thiazole
7,22 ppm: (s) $NH_2$
9,31 ppm: (d) NH–CO

Spectre IR (nujol)
1780–1663 $cm^{-1}$: –C=O
1530 $cm^{-1}$: amide secondaire
1588 $cm^{-1}$: système conjugué
1150–1200 $cm^{-1}$: $CF_3$
1025 et 1045 $cm^{-1}$: oxime

Le chlorhydrate de (3S) 3-amino 1-(1-H-1,2,4-triazol-5-yl) 2-azétidinone utilisé au départ de l'exemple 12 a été préparé comme suit:

a) 1-benzyl 3-amino 5-trifluorométhyl 1,2,4-triazole.

On agite à température ambiante 10 g de 2-phénylméthyl hydrazine carboximidamide dans 100 $cm^3$ de méthanol avec 2,7 g de méthylate de sodium puis ajoute 6 $cm^3$ de trifluoroacétate d'éthyle, agite 1 heure puis concentre à sec sous

pression réduite. On reprend le résidu par 250 $cm^3$ d'éther et 100 $cm^3$ d'eau, décante, extrait à l'éther, sèche la phase organique et concentre à sec. On reprend le résidu à l'éther, chromatographie sur silice en éluant à l'éther et isole l7,1 g de produit brut que l'on empâte à l'éther isopropylique, essore et sèche. On obtient 6,25 g de produit attendu. F = 126 °C.

b) Chlorhydrate de (3S) 3-amino 1-(1-H-1,2,4-triazol-5-yl) 2-azétidinone.

On opère comme aux stades a), b), c), d) et e) de la préparation suivant l'exemple 3 au départ du produit obtenu au stade précédent et de lactone de la N-trityl L-sérine.

Exemple 13

(3S) 3-[[2-(2-aminothiazol-4-yl) 2-méthoxyimino acétyl] amino] 1-(4-nitro 3-trifluorométhyl 1H-pyrazol-5-yl) 2-azétidinone.

A) (3S) 3-terbutoxy carbonylamino 1-[1-[2-(phénylthio) éthyl] 3-trifluorométhyl 4-nitro pyrazol-5-yl] 2-azétidinone.

On refroidit à −35 °C, 925 mg de 3-terbutoxy carbonylamino 2-azétidinone préparé selon le brevet français 2 509 299 dans 20 $cm^3$ de tétrahydrofuranne et ajoute 6,25 $cm^3$ d'une solution 0,8M de bis triméthylsilyl amidure de sodium dans le tétrahydrofuranne, agite 15 minutes à −35 °C et ajoute 2,2 g de 1-[2-(phénylthio) éthyl] 3-trifluorométhyl 4-nitro 5-fluoropyrrazole. On laisse revenir à température ambiante, verse dans 100 $cm^3$ d'une solution aqueuse de phosphate monosodique 1M, et extrait à l'éther. On sèche la phase organique, concentre, chromatographie le résidu sur silice (éluant: acétate d'éthyle 75-25) et isole 1,65 g de produit attendu. Rf = 0,22 (éluant hexane-acétate d'éthyle 8-2).

B) (3S) 3-terbutoxycarbonylamino 1-[1-[2-(phénylsulfonyl) éthyl] 3-trifluorométhyl 4-nitropyrrazol-5-yl] 2-azétidinone.

On ajoute sous agitation en 2 fois à 5 minutes d'intervalle, 400 mg d'acide m-chloroperbenzoïque dans une solution de 1,05 g de produit obtenu au stade précédent dans 10 $cm^3$ de chlorure de méthylène. On laisse sous agitation 30 minutes à température ambiante, ajoute 40 $cm^3$ de chlorure de méthylène, lave la phase organique avec une solution aqueuse de carbonate acide de potassium, sèche et concentre à sec. On isole 1,05 g de produit attendu. Rf = 0,45 (éluant: chlorure de méthylène-acétate d'éthyle 9-1).

C) (3S) 3-terbutoxycarbonylamino 1-[4-nitro 3-trifluorométhyl pyrrazol-5-yl] 2-azétidinone.

On ajoute 2,4 $cm^3$ d'une solution 1,45M de terbutylate de potassium dans le tétrahydrofuranne à 1,60 g de produit préparé comme au stade B) ci-dessus dans 80 $cm^3$ d'éther. On agite 15 minutes et ajoute 3,3 $cm^3$ d'acide chlorhydrique N puis 20 $cm^3$ d'eau, décante, extrait à l'éther, sèche la phase organique et concentre à sec sous pression réduite. Après chromatographie sur silice (éluant: chlorure de méthylène-méthanol 96-4), on isole 580 mg de produit brut que l'on recristallise dans un mélange éther isopropylique-éther éthylique 5-3. On obtient 503 mg de produit attendu. F ≃ 200–210 °C (décomposition) Rf = 0,3 (éluant: chlorure de méthylène-méthanol 9-1).

D) Trifluoroacétate de (3S) 3-amino 1-[4-nitro 3-trifluorométhyl pyrrazol-5-yl] 2-azétidinone.

On agite 15 minutes à température ambiante 452 mg de produit obtenu au stade C) précédent dans 5 $cm^3$ d'acide trifluoroacétique et concentre à sec. On reprend le résidu avec 15 $cm^3$ d'acétonitrile et concentre. On répète 4 fois cette opération et évapore le solvant sous pression réduite et recueille 476 mg de produit attendu. Rf = 0,2 (éluant: chlorure de méthylène-méthanol 8-2).

E) (3S) 3-[[2-(2-tritylamino thiazol-4-yl) 2-méthoxyimino acétyl] amino] 1-(4-nitro 3-trifluorométhyl 1H-pyrrazol-5-yl) 2-azétidinone.

On ajoute une solution de 475 mg de produit obtenu précédemment dans 15 $cm^3$ d'acétonitrile et 0,35 $cm^3$ de triéthylamine dans une suspension d'anhydride mixte paratoluène sulfonique 2-(2-tritylamino thiazol-4-yl) 2-méthoxyimino acétique isomère syn préparé comme au stade b) de l'exemple 1 à partir de 600 mg d'acide et 256 mg de chlorure paratoluène sulfonique. Après 1 heure d'agitation à température ambiante, on concentre, reprend au chlorure de méthylène, lave avec une solution aqueuse de phosphate monosodique 1M et d'acide chlorhydrique 0,1N, sèche la phase organique et concentre à sec. Après chromatographie du résidu sur silice (éluant: chlorure de méthylène-méthanol 94-6), on isole 657 mg de produit attendu. Rf = 0,4 (éluant: chlorure de méthylène-méthanol 9-1).

F) (3S) 3-[[2-(2-aminothiazol-4-yl) 2-méthoxyimino acétyl] amino] 1-(4-nitro 3-trifluorométhyl 1H-pyrrazol-5-yl) 2-azétidinone.

On opère comme au stade b) de l'exemple 3 à partir de 610 mg de produit obtenu au stade E) précédent et obtient 257 mg de produit attendu. F ≃ 200–210 °C. Rf = 0,45 (éluant: acétate d'éthyl-éthanol-eau 85-10-5).

Spectre de RMN (DMSO)
3,87 ppm: N–$OCH_3$
4,11 à 4,42 ppm: $H_4$
5,18 ppm: $H_3$
6,77 ppm: $H_5$ du thiazole
7,22 ppm: $NH_2$
9,36–9,44 ppm: NH–CO

Spectre IR (nujol)
1790 (épaulement) 1763 (max)
1750–1680–1655 $cm^{-1}$: C=O
1603–1585–1537–1528 $cm^{-1}$: aromatiques.

Le 1-[2-(phénylthio) éthyl] 3-trifluorométhyl 4-nitro 5-fluoro pyrrazole utilisé au départ de l'exemple 13 a été préparé comme suit:

a) 3-bromo 4-nitro 5-trifluorométhyl pyrrazole.

On chauffe à 80 °C, 14 g de 3-bromo 5-trifluorométhyl pyrrazole dans 100 $cm^3$ d'acide sulfurique pur et ajoute en 15 minutes 10 g de nitrate de potassium. Après 45 minutes d'agitation à 80 °C, on refroidit et verse sur de la glace, extrait au chlorure de méthylène, sèche la phase organique et concentre à sec sous pression réduire à 50 °C. On isole 16,9 g de produit attendu. F = 50–60 °C.

b) 1-[2-(phénylthio) éthyl] 3-trifluorométhyl 4-nitro 5-bromo pyrrazole.

On ajoute 1,95 g d'hydrure de sodium à 55% dans l'huile à 10,4 g de produit obtenu précédemment dans 100 $cm^3$ de diméthyl formamide. Après 15 minutes d'agitation à température ambiante, on ajoute 10 g de 2-phénylthio 1-bromoéthane et porte 16 heures à 80 °C. On refroidit, verse sur 600 $cm^3$ d'eau, extrait à l'éther, sèche la phase organique et concentre sous pression réduite. On reprend à l'hexane, glace, essore les cristaux, les lave à l'hexane, à l'éther isopropylique puis de nouveau à l'hexane. Après recristallisation dans l'éther isopropylique, on isole 6,2 g de produit attendu. Après chromatographie sur silice des liqueurs mères (éluant: hexane-acétate d'éthyle 8-2), on recueille un second jet de 6,1 g de produit identique soit 12,3 g de produit attendu. F ≃ 66–68 °C.

c) 1-[2-(phénylthio) éthyl] 3-trifluorométhyl 4-nitro 5-fluoro pyrrazole.

On porte au reflux sous atmosphère inerte 5 g d'éther couronne (18-6), 1200 $cm^3$ d'acétonitrile et 7 g de fluorure de potassium, distille 200 $cm^3$ de solvant et ajoute 6,2 g de produit obtenu au stade précédent. On chauffe 2 heures au reflux, filtre et concentre à sec. On reprend le résidu au benzène, concentre sous pression réduite et obtient 4,8 g de produit attendu.

Exemple 14

Trifluoroacétate de (3SR,4SR) 3-[[2-(2-aminothiazol-4-yl) 2-[(1-carboxy 1-méthyléthoxy) imino] acétyl] amino] 4-(fluorométhyl) 1-(tétrazol-5-yl) 2-azétidinone.

On opère comme à l'exemple 7 stades E) et F) à partir du chlorhydrate de (3SR,4SR) 3-amino 4-fluorométhyl 1-(1-H-tétrazol-5-yl) 2-azétidinone préparé comme au stade D) de l'exemple 11 et d'anhydride mixte paratoluène sulfonique 2-(2-tritylamino thiazol-4-yl) 2-[(1-carboxy 1-méthyléthoxy) imino] acétique préparé comme à l'exemple 7. On obtient le produit attendu.

Spectre de RMN (DMSO)
1,64 ppm (s): gem-diméthyl
4,69 à 5,06 ppm (m): $H_4$ + $CH_2F$
5,66 ppm (m): $H_3$
6,75 ppm (s): $H_5$ du thiazole
7,36 ppm (m): NH–CO
12,65 ppm: $CF_3$–$CO_2H$

Spectre IR (nujol)
1780–1675 $cm^{-1}$: amide + trifluoroacétate
1640 $cm^{-1}$: $NH_2$
1590 $cm^{-1}$: $CO_2^{\ominus}$
1550 $cm^{-1}$: système conjugué
1200–1143 $cm^{-1}$: $CF_3$

Exemple 15

(3SR,4RS) 3-[[2-(2-aminothiazol-4-yl) 2-méthoxyimino acétyl] amino] 4-fluorométhyl 1-(tétrazol-5-yl) 2-azétidinone.

On opère comme à l'exemple 11, stades A) à E) à partir du chlorhydrate de (3SR,4RS) 3-amino 4-fluorométhyl 2-azétidinone et obtient le produit attendu. Rf = 0,2 (éluant: acétate d'éthyle-éthanol-eau 7-2-1).

Spectre de RMN (DMSO)
3,86 ppm (s): O–$CH_3$
4,56 à 5,22 ppm (m): $CH_2$–F + $H_3$ + $H_4$
7,24 ppm (s): $NH_2$
9,42 ppm (d): NH–CO

Spectre IR (nujol)
1770–1675 $cm^{-1}$: C=O
1528 $cm^{-1}$: amide secondaire
1640 $cm^{-1}$ (déformation): $NH_2$
1611–1592–1528 $cm^{-1}$: système conjugué
1032 $cm^{-1}$: oxime

Le chlorhydrate de (3SR,4RS) 3-amino 4-fluorométhyl 2-azétidinone utilisé au départ de l'exemple 15 a été préparé comme suit:

a) (3RS,4RS,1'SR) 3-amino 4-fluorométhyl 1-(1-phényléthyl) 2-azétidinone.

On ajoute en 3 minutes, 8,6 g d'hydrate d'hydrazine dans 50 $cm^3$ de méthanol à une solution de 61 g de (3RS,4RS,1'SR) 3-phtalimido 4-fluoro 1-(1-phényléthyl) 2-azétidinone 600 $cm^3$ de dioxanne. On agite 1 heure à température ambiante et ajoute 175 $cm^3$ d'acide chlorhydrique N. On évapore le dioxanne, ajoute 400 $cm^3$ de méthanol, agite 2 heures à 40 °C, filtre, concentre à sec le filtrat sous pression réduite, reprend le résidu avec 300 $cm^3$ d'eau et élimine l'insoluble par filtration. On lave le filtrat au chlorure de méthylène, ajoute à la phase aqueuse 87 $cm^3$ de soude 2N et extrait au chlorure de méthylène. On sèche la phase organique, la concentre à sec et recueille 34,3 g de produit attendu. Rf = 0,4 (éluant: chlorure de méthylène-méthanol-ammoniaque 95-5-0,5).

b) (3SR,4RS,1'SR) 3-[(phénylméthylène) amino] 4-fluorométhyl 1-(1-phényléthyl) 2-azétidinone.

On agite 15 minutes à température ambiante sous atmosphère inerte 34,3 g de produit obtenu au stade précédent dans 500 $cm^3$ de chlorure de méthylène avec 16,6 g de benzaldéhyde et 40 g de sulfate de magnésium. On filtre, concentre à sec le filtrat sous pression réduite et récupère 48 g de produit attendu.

c) (3SR,4RS,1'SR) 3-amino 4-fluorométhyl 1-(1-phényléthyl) 2-azétidinone.

On refroidit à −50 °C, 47,7 g de produit obtenu au stade précédent dans 500 $cm^3$ de tétrahydrofuranne et ajoute 185 $cm^3$ d'une solution 0,83M de bis triméthylsilyl amidure de sodium dans le tétrahydrofuranne. On agite 15 minutes à −50 °C et verse la solution dans 500 $cm^3$ d'acide chlorhydrique N. On agite 15 minutes, lave la phase aqueuse à l'éther puis au chlorure de méthylène. On ajoute 20 g de chlorure d'ammonium à la phase aqueuse puis 25 $cm^3$ d'ammoniaque concentré. On extrait au chlorure de méthylène sépare la phase organique, la sèche et la concentre à sec sous pression

réduite. Après chromatographie sur silice (éluant: chlorure de méthylène-méthanol-ammoniaque 97-3-0,5), on isole 19,5 g de produit attendu (isomère trans) de Rf = 0,3 (éluant: chlorure de méthylène-méthanol-ammoniaque 95-5-0,5) et 5 g d'isomère cis correspondant.

d) (3SR,4RS,1'SR) 3-phtalimido 4-fluorométhyl 1-(1-phényléthyl) 2-azétidinone.

On porte 5 heures au reflux 19,3 g de produit obtenu au stade précédent dans 500 $cm^3$ de tétrahydrofuranne avec 20 g de N-éthoxycarbonyl phtalimide et 16 $cm^3$ de triéthylamine. On concentre à sec, reprend le résidu à l'éthanol, glace, essore les cristaux, les lave à l'éthanol puis à l'éther. On isole 19 g de produit puis 6,5 g après chromatographie des eaux mères sur silice (éluant: benzène-acétone 9-1). Rf = 0,3.

e) (3SR,4RS) 3-phtalimido 4-fluorométhyl 2-azétidinone.

On porte au reflux 25 g de produit obtenu au stade précédent dans 400 $cm^3$ d'acétonitrile et 240 $cm^3$ d'eau et ajoute en 15 minutes 40 g de persulfate d'ammonium dans 100 $cm^3$ d'eau. On maintient le reflux 2 heures et 30 minutes, refroidit, sature de chlorure de sodium, décante, extrait la phase aqueuse à l'acétate d'éthyle, sèche les phases organiques et les concentre à sec. Après chromatographie du résidu sur silice (éluant: chlorure de méthylène-acétate d'éthyle 75-25) puis cristallisation dans l'éther, on isole 7,15 g de produit attendu. F = 175–178 °C.

f) Chlorhydrate de (3SR,4RS) 3-amino 4-fluorométhyl 2-azétidinone.

On ajoute en 5 minutes, 1,43 g d'hydrate d'hydrazine dans 10 $cm^3$ de méthanol dans une solution de 7,1 g de produit obtenu au stade précédent dans 150 $cm^3$ de dioxanne. On agite 30 minutes à température ambiante et ajoute 29 $cm^3$ d'acide chlorhydrique N. On concentre sous pression réduite, reprend par 200 $cm^3$ de méthanol et chauffe 1 heure à 40 °C. On évapore le méthanol, reprend par 200 $cm^3$ d'eau, filtre, concentre le filtrat sous pression réduite jusqu'à l'obtention d'un volume de 100 $cm^3$, filtre de nouveau et concentre à sec le filtrat sous pression réduite. On reprend le résidu au méthanol, essore les cristaux, les lave au méthanol puis à l'éther et isole 3,59 g de produit attendu. F $\simeq$ 200 °C (décomposition) Rf = 0,2 (éluant: chlorure de méthylène-méthanol-ammoniaque 9-1-0,1).

Exemple 16

On a réalisé une préparation pour injection de formule:

– (3SR,4RS) 3-[[2-(2-aminothiazol-4-yl) 2-méthoxy imino acétyl] amino] 4-méthyl 1-(1H-tétrazol-5-yl) 2-azétidinone isomère syn 500 mg
– excipient aqueux 5 $cm^3$

Exemple 17

On a réalisé des gélules répondant à la formule:

– (3SR,4RS) 3-[[2-(2-aminothiazol-4-yl) 2]-(carboxyméthoxy) imino] acétyl] amino] 4-méthyl 1-(1H-tétrazol-5-yl) 2-azétidinone isomère syn 250 mg
– excipient q.s.p. une gélule terminée à 400 mg.

Etude pharmacologique des produits de l'invention.

Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarante-huit heures à l'étuve à 37 °C, l'inhibition de la croissance est appréciée par transillumination ce qui permet de déterminer les concentrations minimales inhibitrices (C.M.I.) exprimées en µg/$cm^3$.

Les résultats suivants sont obtenus:

| SOUCHES | Prod. ex. 1 | | Prod. ex. 2 | | Prod. ex. 3 | |
|---|---|---|---|---|---|---|
| | 24 H | 48 H | 24 H | 48 H | 24 H | 48 H |
| Pseudomonas aeruginosa 1771 m | ⩽5 | ⩽5 | ⩽5 | ⩽5 | 5 | 10 |
| Escherichia Coli 1894 | 0,08 | 0,08 | 0,3 | 0,3 | 0,15 | 0,15 |
| Escherichia Coli 078 | 0,3 | 0,3 | 0,6 | 0,6 | 0,3 | 0,3 |
| Escherichia Coli TEM | 0,6 | 1,2 | 10 | 10 | 10 | 10 |
| Escherichia Coli 1507 E | 0,3 | 0,3 | 0,6 | 0,6 | 0,6 | 0,6 |
| Escherichia Coli DCO | 1,2 | 1,2 | 2,5 | 2,5 | 0,6 | 1,2 |
| Escherichia Coli DC2 | 0,6 | 0,6 | 0,6 | 1,2 | 0,6 | 0,6 |
| Salmonella typhimurium MZ 11 | 1,2 | 1,2 | 1,2 | 2,5 | 0,3 | 0,3 |
| Klebsiella pneumonae 52145 | 1,2 | 2,5 | 2,5 | 2,5 | 1,2 | 1,2 |
| Klebsiella Aeruginosa 1522 E | 1,2 | 1,2 | 0,6 | 0,6 | 0,3 | 0,3 |
| Enterobacter cloacae 1321 E | 0,6 | 0,6 | 1,2 | 1,2 | 0,3 | 0,3 |
| Serratia marcesceus 2532 | 20 | 20 | 20 | >40 | 2,5 | 5 |
| Proteus mirabilis A 235 | 0,15 | 0,15 | 0,6 | 1,2 | 0,6 | 0,6 |
| Proteus vulagris A 232 | 0,3 | 0,3 | 1,2 | 2,5 | 0,3 | 0,3 |
| Providencia DU 48 | 0,6 | 0,6 | 1,2 | 1,2 | 0,3 | 0,6 |

| SOUCHES | Prod. ex. 4 | | Prod. ex. 6 | | Prod. ex. 7 | |
|---|---|---|---|---|---|---|
| | 24 H | 48 H | 24 H | 48 H | 24 H | 48 H |
| Pseudomonas aeruginosa 1771 m | | | 2,5 | 5 | 0,6 | 0,6 |
| Escherichia Coli 1894 | ⩽0,04 | ⩽0,04 | 0,6 | 0,6 | 0,6 | 0,6 |
| Escherichia Coli 078 | 0,3 | 0,3 | 0,3 | 0,3 | 0,3 | 0,6 |
| Escherichia Coli TEM | 0,6 | 1,2 | 0,6 | 0,6 | 1,2 | 1,2 |
| Escherichia Coli 1507 E | 0,15 | 0,15 | 0,6 | 0,6 | 1,2 | 1,2 |
| Escherichia Coli DCO | 1,2 | 1,2 | 0,6 | 0,6 | 0,6 | 1,2 |
| Escherichia Coli DC2 | ⩽0,04 | ⩽0,04 | 0,3 | 0,3 | 0,6 | 0,6 |
| Salmonella typhimurium MZ 11 | 1,2 | 1,2 | 0,6 | 0,6 | 0,6 | 0,6 |
| Klebsiella pneumonae 52145 | 5 | 5 | 2,5 | 2,5 | 2,5 | 2,5 |
| Klebsiella Aeruginosa 1522 E | 1,2 | 1,2 | 0,6 | 0,6 | 1,2 | 1,2 |
| Enterobacter cloacae 1321 E | 1,2 | 1,2 | 0,3 | 0,6 | 2,5 | 2,5 |
| Serratia marcesceus 2532 | 20 | 20 | 2,5 | 5 | 1,2 | 1,2 |
| Proteus mirabilis A 235 | 0,3 | 0,3 | 0,15 | 0,15 | 0,15 | 0,15 |
| Proteus vulagris A 232 | 0,3 | 0,3 | 0,08 | 0,15 | 0,08 | 0,08 |
| Providencia DU 48 | 2,5 | 2,5 | 0,6 | 0,6 | 0,6 | 0,6 |

| SOUCHES | Prod. ex. 11 | |
|---|---|---|
| | 24 H | 48 H |
| Pseudomonas aeruginosa 1771 m | | |
| Escherichia Coli 1894 | ⩽0,04 | ⩽0,04 |
| Escherichia Coli 078 | 0,15 | 0,15 |
| Escherichia Coli TEM | 0,6 | 0,6 |
| Escherichia Coli 1507 E | 0,08 | 0,08 |
| Escherichia Coli DCO | 0,6 | 0,6 |
| Escherichia Coli DC2 | 0,15 | 0,15 |
| Salmonella typhimurium MZ 11 | 0,3 | 0,3 |
| Klebsiella pneumonae 52145 | 1,2 | 1,2 |
| Klebsiella Aeruginosa 1522 E | 0,6 | 0,6 |
| Enterobacter cloacae 1321 E | 0,3 | 0,3 |
| Serratia marcesceus 2532 | 10 | 10 |
| Proteus mirabilis A 235 | 0,15 | 0,15 |
| Proteus vulagris A 232 | 0,15 | 0,15 |
| Providencia DU 48 | 1,2 | 1,2 |

**Revendications: Pour les états contractants: BE, SE, CH, DE, FR, GB, IT, LI, LU, NL**

1. Produits de formule générale (I):

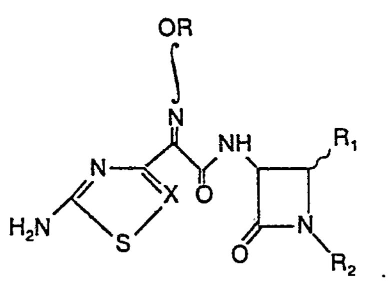

(I)

dans laquelle R représente un atome d'hydrogène, un radical alkyle, alkényle ou alkynyle ayant au plus 12 atomes de carbone, éventuellement substitué par un ou plusieurs radicaux choisis parmi les radicaux carboxy éventuellement salifiés ou estérifiés, les radicaux amino, méthylamino, diméthylamino, diéthylamino, le radical phényle éventuellement substitué par un ou plusieurs radicaux choisis parmi les radicaux hydroxy, méthyl, méthoxy, chloro, bromo, fluoro; les radicaux fluoro, chloro, bromo ou iodo, les radicaux nitrile, $CONH_2$ ou $CONH\ SO_2R''$ dans lequel R'' représente un radical alkyle ayant de 1 à 4 atomes de carbone, un radical phényle, un radical amino, méthyl ou diméthylamino, un radical amino hétérocyclique choisi parmi les radicaux pipéridino, morpholino pipérazino ou 4-éthyl 2,3-dioxo 1-pipérazino;

– un radical cycloalkyle ayant de 3 à 8 atomes de carbone ou un radical:

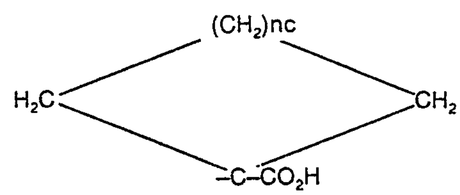

salifié ou estérié dans lequel nc représente un entier de 0 à 5,

– un radical acétyle, propionyle ou benzoyle, carbamoyle, diméthylamino carbonyle, phényle ou benzyle éventuellement substitué par alkyle, alkoxy ou halogène,

$R_1$ représente un atome d'hydrogène, un radical alkyle, alkényle ou alkynyle ou thioalkyle ayant au plus 12 atomes de carbone, éventuellement substitué par un ou plusieurs radicaux choisis parmi les radicaux azido, alkylthio ayant de 1 à 4 atomes de carbone ou phénylthio, les radicaux carboxy éventuellement salifiés ou estérifiés, les radicaux amino, méthylamino, diméthylamino, diéthylamino, le radical phényl éventuellement substitué par un ou plusieurs radicaux choisis parmi les radicaux hydroxy, halogéno, trifluorométhyle, amino, alkyle ou alkoxy ayant de 1 à 4 atomes de carbone; les radicaux fluoro, chloro, bromo ou iodo, les radicaux nitrile, $CONH_2$ ou $CONH\ SO_2R''$

dans lequel R″ représente un radical alkyle ayant de 1 à 4 atomes de carbone, un radical phényle, un radical amino, méthyl ou diméthylamino, un radical amino hétérocyclique choisi parmi les radicaux pipéridino, morpholino, pipérazino ou 4-éthyl 2,3-dioxo 1-pipérazino; un radical aryle hétérocyclique choisi parmi les radicaux thiényle, furyle, pyrannyle, thiazolyle, oxazolye, oxadiazolyle, pyridinyle ou pyrimidinyle; les radicaux acétoxy, propionyloxy, benzoyloxy, acétylamino, benzylcarbonyle, carbamoyloxy, méthylamino carbonyloxy ou diméthylaminocarbonyloxy, acétyle, propionyle, benzoyle,

- un radical carboxy libre estérifié ou salifié,
- un radical phényle éventuellement substitué par un radical alkyle, trifluorométhyle, alkoxy, alkylthio, halogène, hydroxyle, amino, hydroxyalkyle, un radical hétérocyclique choisi parmi les radicaux thiényle, furyle, pyrolyle, thiazolyle, isothiazolyle, oxazolyle, isoxazolyle, imidazolinyle, imidazolyle, triazolyle, tétrazolyle, thiadiazolyle, oxadiazolyle, pyridinyle, pyrimidinyle, pyridazinyle, triazinyle éventuellement substitué par un radical alkyle, carboxy, carboxy alkyle, aminoalkyle ou dialkylaminoalkyle,
- un radical acétyle, propionyle, n-butyryle, benzoyle, éventuellement substitué par alkoxy ou hydroxy,
- un radical carbamoyle, méthyl ou diméthylcarbamoyle,
- un radical azido.

$R_2$ représente un hétérocycle azoté éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$, $(CH_2)_nNHSO_3H$, $(CH_2)_nSO_2NH_2$, $(CH_2)_nCO_2H$, dans lesquels n représente un entier de 1 à 4 et comportant au moins un hydrogène acide, X représente un radical CH ou un atome d'azote, les traits ondulés signifient que le radical OR peut se trouver sous la forme cis ou trans ou sous la forme d'un mélange cis-trans, les produits de formule (I) étant sous forme racémique ou optiquement active, ainsi que les sels des produits de formule (I) avec les bases et les acides.

2. Les produits selon la revendication 1 de formule générale (I′):

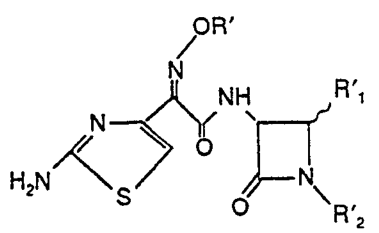

(I′)

dans laquelle R′ représente un atome d'hydrogène ou un radical alkyle, linéaire ou ramifié ayant de 1 à 6 atomes de carbone, éventuellement substitué par un ou plusieurs des radicaux choisis dans le groupe formé par les radicaux carboxy libre, estérifié ou salifié, amino, mono ou diméthylamino, phényle, halogène, nitrile, $CONHSO_2R''$ dans lequel R″ représente un radical alkyle ayant 1 à 4 atomes de carbone, phényle ou amino mono ou diméthylamino ou R′ représente un radical acétyle, propionyle ou phényle carbonyle ou un radical phényle,

ou R′ représente un radical cycloalkyle ayant de 3 à 8 atomes de carbone ou un radical:

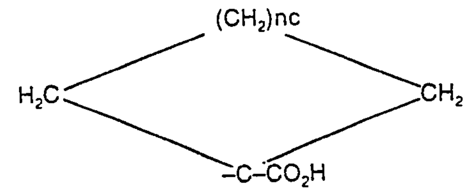

salifié ou estérifié dans lequel nc représente un entier de 0 à 5.

$R'_1$ représente un atome d'hydrogène ou un radical alkyle, ayant au plus 12 atomes de carbone éventuellement substitué par un ou plusieurs des radicaux choisis dans le groupe formé par les radicaux halogène, azido, hydroxyle, mercapto, phényle, amino, nitrile, alkylthio ayant de 1 à 4 atomes de carbone ou phénylthio éventuellement oxydés, acétyle, propionyle, benzoyle, acétoxy, propionyloxy, benzoyloxy, acétylamino, benzylcarbonyle, carbamoyloxy, méthyl ou diméthyl carbamoyloxy ou $R'_1$ représente un radical alkényle ou alkynyle ayant au plus 12 atomes de carbone éventuellement substitué par un radical phényle ou par un ou plusieurs atomes d'halogènes, ou $R'_1$ représente un radical thioalkyle éventuellement substitué par carbamoyle,

ou $R'_1$ représente un radical phényle éventuellement substitué par halogène, $CF_3$, amino, hydroxy, alkyle ou alkoxy ou $R'_1$ représente un radical carboxy estérifié, un radical carbamoyle ou un radical azido,

$R'_2$ représente un radical tétrazolyle, triazolyle, imidazolyle, pyrazolyle, ou pyrrolyle, éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$; $(CH_2)_nNHSO_3H$; $(CH_2)_nSO_2NH_2$; $(CH_2)_nCO_2H$ dans lesquels n représente un entier de 1 à 4, les produits ayant l'isomérie syn, le trait ondulé signifie que les produits peuvent se trouver sous la forme cis ou trans, ou sous la forme d'un mélange cis-trans, les produits de formule (I′) étant sous forme racémique ou optiquement active ainsi que les sels des produits de formule (I′) avec les bases et les acides.

3. Les produits de formule générale (I′) telle que décrite à la revendication 2 dans laquelle R′ représente un atome d'hydrogène, un radical méthyle, phényle, difluorométhyle, 1-méthyl 1-carboxy éthyle, cyano méthyle, carboxy méthyle (méthyl sulfonyl) carbamoyl méthyle ou un radical:

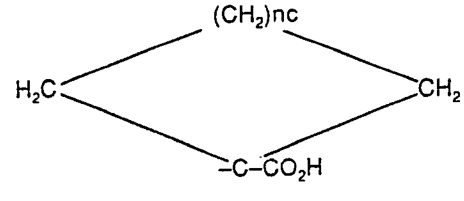

salifié ou estérifié dans lequel nc représente un entier de 0 à 5.

$R'_1$ représente un atome d'hydrogène, un radical méthyle, fluorométhyle, trifluorométhyle, éthoxycarbonyle, carbamoyle;

$R'_2$ représente un radical 1H-tétrazol-5-yle, ou 1,3,4-triazol-2-yle éventuellement substitué par un radical trifluorométhyle ou carboxyméthyle.

4. Les produits de formule générale (I') telle que décrite à la revendication 2 dans laquelle R' représente un radical:

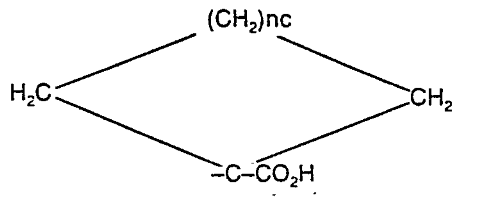

salifié ou estérifié dans lequel nc représente un entier de 0 à 5.

5. L'un quelconque des produits de formule (I) dont les noms suivent:

– la 3-[[2-(2-aminothiazol-4-yl)2-méthoxyimino acétyl]amino] 4-méthyl 1-(1H-tétrazol-5-yl)2-azétidinone cis ou trans, isomère syn, racémique ou optiquement active;

– la 3-[[2-(2-aminothiazol-4-yl)2-carboxy-méthoxy imino acétyl]amino]4-méthyl 1-(1H-tétrazol-5-yl)2-azétidinone cis ou trans, isomère syn, racémique ou optiquement active;

– la 3-[[2-(2-aminothiazol-4-yl) 2-(1-carboxy-1-méthyl) éthoxyimino acétyl]amino] -méthyl 1-(1H-tétrazol-5-yl)2-azétidinone cis ou trans, isomère syn, racémique ou optiquement active;

– la 3-[[2-(2-amino thiazol-4-yl)2-méthoxy imino acétyl]amino] 4-fluorométhyl 1-(1H-tétrazol-5-yl)2-azétidinone cis ou trans, isomère syn, racémique ou optiquement active;

– la 3-[[2-(2-amino thiazol-4-yl)2-fluro-méthoxyiminoacétyl]amino]4-méthyl 1-(1H-tétrazol-5-yl)2-azétidinone cis ou trans, isomère syn, racémique ou optiquement active.

6. Procédé de préparation des produits de formule générale (I) telle que définie à la revendication 1 caractérisé en ce que l'on traite un produit de formule (II):

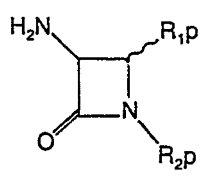

cis ou trans, racémique ou optiquement actif, formule dans laquelle soit $R_1p$ représente $R_1$, $R_1$ ayant la signification indiquée à la revendication 1, soit $R_1p$ représente le substituant $R_1$ dans lequel les fonctions réactives sont protégées et soit $R_2p$ représente $R_2$, $R_2$ ayant la signification indiquée à la revendication 1, soit $R_2p$ représente le substituant $R_2$ dans lequel les fonctions réactives sont protégées, par un produit de formule (III):

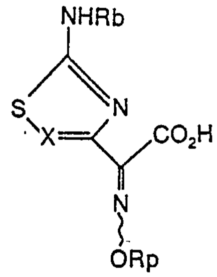

(III)

syn ou anti, dans laquelle Rb représente un atome d'hydrogène ou un groupement protecteur du radical amino et Rp représente un groupement protecteur du radical hydroxyle ou Rp représente R, R ayant la signification indiquée à la revendication 1, ou Rp représente un radical R dans lequel les fonctions réactives sont protégées, pour obtenir un produit de formule (IV):

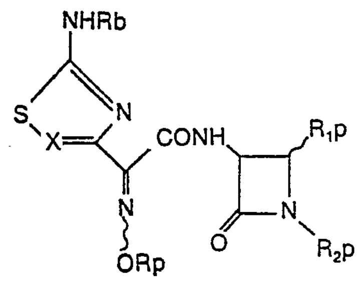

(IV)

syn ou anti, racémique ou optiquement actif, dans lequel X, Rp, $R_1p$, $R_2p$ et Rb ont la signification précédente, produit que l'on soumet, si nécessaire, et, si désiré, à l'une quelconque des réactions suivantes, dans un ordre quelconque:

a) coupure par hydrolyse, hydrogénolyse ou par action de la thiourée du ou des groupements protecteurs que peuvent représenter Rb et Rp ou comporter Rp, $R_1p$ et $R_2p$;

b) estérification ou salification des radicaux carboxy ou sulfo que peuvent comporter les radicaux Rp, $R_1p$ et $R_2p$;

c) salification par un acide du ou des radicaux amino;

d) dédoublement de la molécule pour obtenir un produit optiquement actif.

7. Procédé selon la revendication 6, de préparation des produits de formule (I) telle que définie à la revendication 1, caractérisé en ce que l'on utilise, pour la mise en œuvre du procédé, un produit de formule (II) dans laquelle $R_1p$ représente un atome d'hydrogène, un radical méthyle, fluorométhyle, trifluorométhyle, éthoxycarbonyle ou carbamoyle et $R_2p$ représente un radical 1H-tétrazol-5-yl ou 1,3,4-triazol-2-yl éventuellement substitué par un radical trifluorométhyle ou carboxyméthyle et un produit de formule (III) dans laquelle Rb représente un groupement protecteur du radical amino et Rp représente un radical protecteur du radical hydroxyle, un radical méthyle, phényle, difluorométhyle, 1-méthyl 1-carboxy éthyle, cyanométhyle, carboxyméthyle ou méthylsulfonyl carbamoylméthyle.

8. Procédé selon la revendication 6 de préparation des produits de formule (I) caractérisé en ce que les produits de formule (II):

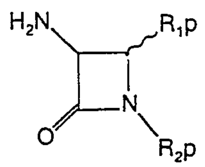

(II)

sont préparés en faisant réagir un produit de formule (V):

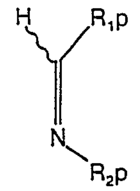

(V)

dans laquelle $R_1p$ et $R_2p$ ont la signification indiquée à la revendication 6, en présence d'une base forte, avec un produit de formule (VI):

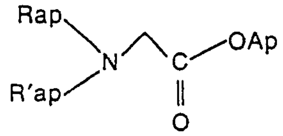

(VI)

dans laquelle Ap représente un atome d'hydrogène ou un groupement ester, Rap et R'ap sont tels que, soit Rap et R'ap représentent chacun un atome d'hydrogène, soit l'un représente un atome d'hydrogène et l'autre représente un groupement protecteur du radical amino, soit Rap et R'ap forment ensemble un radical divalent protecteur du radical amino, pour obtenir un produit de formule (VII):

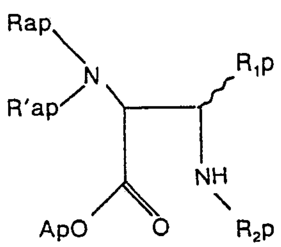

(VII)

dans laquelle Ap, $R_1p$, $R_2p$, Rap et R'ap conservent la signification précédente et le trait ondulé signifie que le substituant $R_1p$ peut se trouver en position α ou β, produit de formule (VII) que, si désiré, l'on soumet à l'une ou l'autre des réactions suivantes, dans un ordre quelconque:

a) séparation des deux isomères;

b) protection du radical $NH_2$ lorsque Rap et R'ap représentent chacun un atome d'hydrogène, produit de formule (VII), sous forme d'un seul isomère ou d'un mélange d'isomères, que l'on soumet, lorsque Ap représente un groupement ester, à un agent de saponification, puis un agent de β-lactamisation, pour obtenir un produit de formule (VIII):

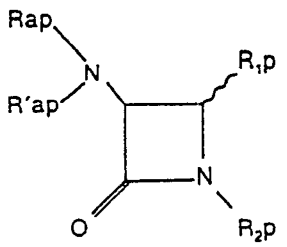

(VIII)

produit que l'on soumet, si nécessaire et, si désiré, à l'une quelconque des réactions suivantes, dans un ordre quelconque:

a) séparation des isomères;

b) coupure par hydrolyse, hydrogénolyse ou action de la thiourée de l'un des radicaux Rap et R'ap ou de ces deux radicaux lorsque l'un représente un groupement protecteur ou les deux représentent ensemble un groupement protecteur, pour obtenir un produit de formule (II) attendu.

9. Procédé selon la revendicatin 6 de préparation des produits de formule (I) caractérisé en ce que les produits de formule (II):

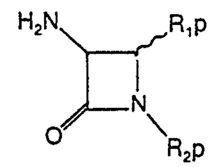

(II)

sont préparés en faisant réagir une β-lactone de formule (IX):

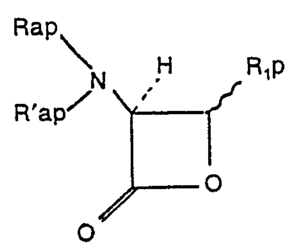

(IX)

dans laquelle $R_1p$, Rap et R'ap ont la signification indiquée à la revendication 8, avec un produit de formule (A):

$H_2N-R_2p$ (A)

dans laquelle $R_2p$ a la signification indiquée à la revendication 8, pour obtenir un produit de formule (X):

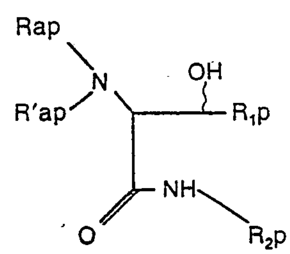

(X)

produit de formule (X) que, si désiré, l'on soumet à l'une ou l'autre des réactions suivantes, dans un ordre quelconque:

a) séparation des isomères dans le cas où $R_1p$ ne représente pas un atome d'hydrogène;

b) protection du radical $NH_2$ lorsque Rap et R'ap représentent chacun un atome d'hydrogène ou modification du groupement protecteur que représente l'un ou l'autre de Rap et R'ap ou que forment ensemble Rap et R'ap et produit de formule (X) sous forme d'un isomère ou d'un mélange d'isomères que l'on soumet à un réactif de cyclisation pour obtenir un produit de formule (VIII):

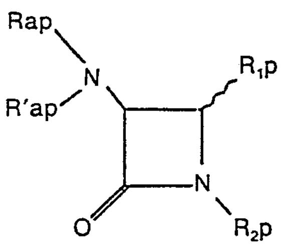

(VIII)

produit de formule (VIII) que l'ont peut séparer en ses isomères et que l'on soumet, lorsque Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée pour obtenir un produit de formule (II) attendu.

10. Procédé selon la revendication 9, caractérisé en ce que pour préparer un produit de formule (II) dans laquelle le substituant $R_1p$ représente un atome d'hydrogène ou un radical méthyle, l'on met en œuvre le procédé décrit à la revendication 8 en partant d'un produit de formule (IX) dans laquelle $R_1p$ représente un atome d'hydrogène ou un radical méthyle.

11. Procédé selon la revendication 6 de préparation des produits de formule (I), caractérisé en ce que les produits de formule (II):

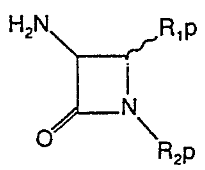

(II)

sont préparés en faisant réagir un produit de formule:

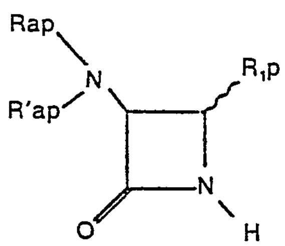

(XI)

dans laquelle $R_1p$, Rap et R'ap sont définis comme précédemment, avec un produit de formule:

$$Y\text{–}R_2p \qquad (XII)$$

dans laquelle $R_2p$ est définis comme précédemment et Y représente un groupement nucléofuge en présence d'une base, pour obtenir un produit de formule (VIII) tel que définis précédemment, que l'on peut séparer en ses isomères et que l'on soumet, lorsque l'un de Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée, pour obtenir un produit de formule (II) attendu.

12. Procédé selon la revendication 11, caractérisé en ce que les produits de formule (XI) de configuration trans, sont préparés en faisant réagir une base sur un produit de formule ($XI_1$):

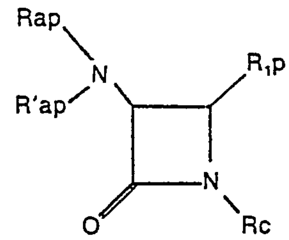

($XI_1$)

de configuration cis, dans laquelle $R_1p$, Rap et R'ap sont définis comme précédemment et Rc représente un atome d'hydrogène ou un groupement protecteur, pour obtenir un produit de formule ($XI_2$):

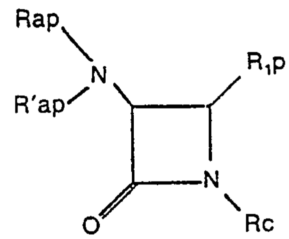

($XI_2$)

de configuration trans, dans laquelle $R_1p$, Rap, R'ap et Rc sont définis comme précédemment, que l'on soumet, le cas échéant, à l'action d'un agent de coupure du groupement protecteur Rc.

13. A titre de médicaments, les produits répondant à la formule (I) telle que définie à la revendication 1 et leurs sels pharmaceutiquement acceptables.

14. A titre de médicaments, les produits répondant à la formule (I') telle que définie aux revendications 2 à 4 et leurs sels pharmaceutiquement acceptables.

15. A titre de médicaments, les produits définis à la revendication 5.

16. Compositions pharmaceutiques contenant, à titre de principe actif, au moins un médicament selon l'une des revendications 13 à 15.

17. A titre de produits industriels nouveaux, les produits de formule générale (II):

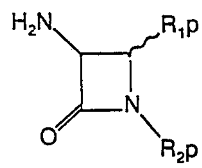

(II)

dans laquelle $R_1p$ et $R_2p$ ont la signification indiquée à la revendication 6.

18. A titre de proudits industriels nouveaux, les produits de formule générale (II) telle que décrite à la revendication 17 dans laquelle $R_1p$ représente un atome d'hydrogène ou un radical alkyle ayant au plus 12 atomes de carbone éventuellement substitué par un ou plusieurs radicaux choisis dans le groupe formé par les radicaux, halogène,

azido, hydroxyle, mercapto, phényle, amino, nitrile, alkylthio ayant de 1 à 4 atomes de carbone ou phénylthio éventuellement oxydés, acétyle, propionyle, benzoyle, acétoxy, propionyloxy, benzoyloxy, acétylamino, benzylcarbonyle, carbamoyloxy, méthyl ou diméthylcarbamoyloxy et $R_2p$ représente un radical tétrazolyle éventuellement protégé.

19. A titre de produits industriels nouveaux, les produits de formule générale (II) telle que décrite à la revendication 17 dans laquelle $R_1p$ représente un radical fluorométhyle et $R_2p$ représente un radical tétrazolyle éventuellement protégé.

**Revendications: Pour l'état contractant: AT**

1. Procédé pour préparer les produits de formule générale (I):

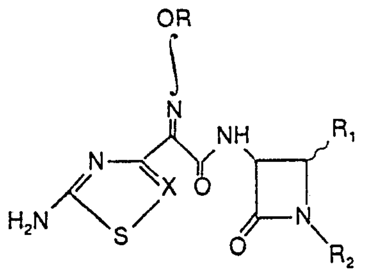

(I)

dans laquelle R représente un atome d'hydrogène, un radical alkyle, alkényle ou alkynyle ayant au plus 12 atomes de carbone, éventuellement substitué par un ou plusieurs radicaux choisis parmi les radicaux carboxy éventuellement salifiés ou estérifiés, les radicaux amino, méthylamino, diméthylamino, diéthylamino, le radical phényle éventuellement substitué par un ou plusieurs radicaux choisis parmi les radicaux hydroxy, méthyl, méthoxy, chloro, bromo, fluoro; les radicaux fluoro, chloro, bromo ou iodo, les radicaux nitrile, $CONH_2$ ou CONH $SO_2R''$ dans lequel R'' représente un radical alkyle ayant de 1 à 4 atomes de carbone, un radical phényle, un radical amino, méthyl ou diméthylamino, un radical amino hétérocyclique choisi parmi les radicaux pipéridino, morpholino pipérazino ou 4-éthyl 2,3-dioxo 1-pipérazino;

– un radical cycloalkyle ayant de 3 à 8 atomes de carbone ou un radical:

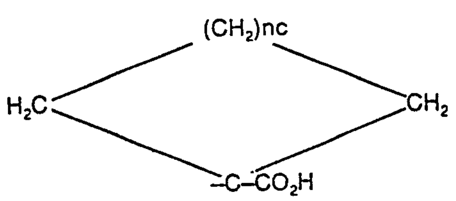

salifié ou estérié dans lequel nc représente un entier de 0 à 5,

– un radical acétyle, propionyle ou benzoyle, carbamoyle, diméthylamino carbonyle, phényle ou benzyle éventuellement substitué par alkyle, alkoxy ou halogène.

$R_1$ représente un atome d'hydrogène, un radical alkyle, alkényle ou alkynyle ou thioalkyle ayant au plus 12 atomes de carbone, éventuellement substitué par un ou plusieurs radicaux choisis parmi les radicaux azido, alkylthio ayant de 1 à 4 atomes de carbone ou phénylthio, les radicaux carboxy éventuellement salifiés ou estérifiés, les radicaux amino, méthylamino, diméthylamino, diéthylamino, le radical phényl éventuellement substitué par un ou plusieurs radicaux choisis parmi les radicaux hydroxy, halogéno, trifluorométhyle, amino, alkyle ou alkoxy ayant de 1 à 4 atomes de carbone; les radicaux fluoro, chloro, bromo ou iodo, les radicaux nitrile, $CONH_2$ ou CONH $SO_2R''$ dans lequel R'' représente un radical alkyle ayant de 1 à 4 atomes de carbone, un radical phényle, un radical amino, méthyl ou diméthylamino, un radical amino hétérocyclique choisi parmi les radicaux pipéridino, morpholino, pipérazino ou 4-éthyl 2,3-dioxo 1-pipérazino; un radical aryle hétérocyclique choisi parmi les radicaux thiényle, furyle, pyrannyle, thiazolyle, thiadiazolyle, oxazolyle, oxadiazolyle, pyridinyle ou pyrimidinyle; les radicaux acétoxy, propionyloxy, benzoyloxy, acétylamino, benzylcarbonyle, carbamoyloxy, méthylamino carbonyloxy ou diméthylaminocarbonyloxy, acétyle, propionyle, benzoyle:

– un radical carboxy libre estérifié ou salifié,

– un radical phényle éventuellement substitué par un radical alkyle, trifluorométhyle, alkoxy, alkylthio, halogène, hydroxyle, amino, hydroxyalkyle, un radical hétérocyclique choisi parmi les radicaux thiényle, furyle, pyrolyle, thiazolyle, isothiazolyle, oxazolyle, isoxazolyle, imidazolinyle, imidazolyle, triazolyle, tétrazolyle, thiadiazolyle, oxadiazolyle, pyridinyle, pyrimidinyle, pyridazinyle, triazinyle éventuellement substitué par un radical alkyle, carboxy, carboxy alkyle, aminoalkyle ou diaminoalkyle:

– un radical acétyle, propionyle, n-butyryle, benzoyle, éventuellement substitué par alkoxy ou hydroxy,

– un radical carbamoyle, méthyl ou diméthylcarbamoyle,

– un radical azido.

$R_2$ représente un hétérocycle azoté éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$, $(CH_2)_nNHSO_3H$, $(CH_2)_nSO_2NH_2$, $(CH_2)_nCO_2H$, dans lesquels n représente un entier de 1 à 4 et comportant au moins un hydrogène acide, X représente un radical CH ou un atome d'azote, les traits ondulés signifient que le radical OR peut se trouver sous la forme syn ou anti et que les produits peuvent se trouver sous la forme cis ou trans ou sous la forme d'un mélange cis-trans, les produits de formule (I) étant sous forme racémique ou optiquement active, ainsi que les sels des produits de formule (I) avec les bases et les acides, caractérisé en ce que l'on traite un produit de formule (II):

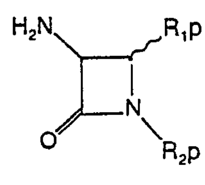

(II)

cis ou trans, racémique ou optiquement actif, formule dans laquelle soit $R_1p$ représente $R_1$, $R_1$ ayant la signification indiquée précédemment, soit $R_1p$ représente le substituant $R_1$ dans lequel les fonctions réactives sont protégées et soit $R_2p$ représente $R_2$, $R_2$ ayant la signification indiquée précédemment, soit $R_2p$ représente le substituant $R_2$ dans lequel les fonctions réactives sont protégées, par un produit de formule (III):

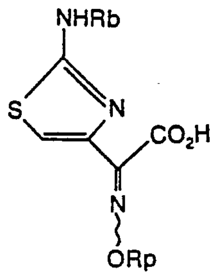

(III)

syn ou anti, dans laquelle Rb représente un atome d'hydrogène ou un groupement protecteur du radical amino et Rp représente un groupement protecteur du radical hydroxyle ou Rp représente R, R ayant la signification indiquée à la revendication 1, ou Rp représente un radical R dans lequel les fonctions réactives sont protégées, pour obtenir un produit de formule (IV):

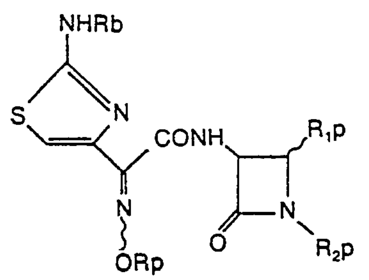

(IV)

syn ou anti, racémique ou optiquement actif, dans lequel Rp, $R_1p$, $R_2p$ et Rb ont la signification précédente, produit que l'on soumet, si nécessaire, et, si désiré, à l'une quelconque des réactions suivantes, dans un ordre quelconque:

a) coupure par hydrolyse, hydrogénolyse ou par action de la thiourée du ou des groupements protecteurs que peuvent représenter Rb et Rp ou comporter Rp, $R_1p$ et $R_2p$;

b) estérification ou salification des radicaux carboxy ou sulfo que peuvent comporter les radicaux Rp, $R_1p$ et $R_2p$;

c) salification par un acide du ou des radicaux amino;

d) dédoublement de la molécule pour obtenir un produit optiquement actif.

2. Procédé selon la revendication 5, de préparation des produits de formule (I) telle que définie à la revendication 1, répondant à la formule ($I_a$):

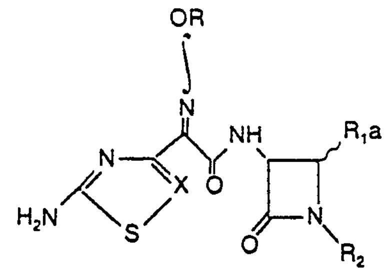

($I_a$)

dans laquelle R, $R_2$ et X ont la signification indiquée à la revendication 1 et $R_1a$ a la signification indiquée à la revendication 1 pour $R_1$ à l'exception d'azido, caractérisé en ce que l'on utilise au départ un produit de formule (II) dans laquelle $R_1p$ à les valeurs indiquées à la revendication 1, à l'exception d'un radical azido.

3. Procédé selon la revendication 2, caractérisé en ce que l'on utilise au départ des composés de formule (II) et (III) choisis de manière telle que l'on prépare les composés de formule générale (I'):

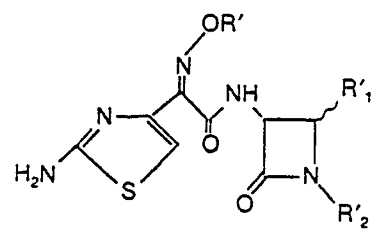

(I')

dans laquelle R' représente un atome d'hydrogène ou un radical alkyle, linéaire ou ramifié ayant de 1 à 6 atomes de carbone, éventuellement substitué par un ou plusieurs des radicaux choisis dans le groupe formé par les radicaux carboxy libre, estérifié ou salifié, amino, mono ou diméthylamino, phényle, halogène, nitrile, $CONHSO_2R''$ dans lequel R'' représente un radical alkyle ayant 1 à 4 atomes de carbone, phényle ou amino mono ou diméthylamino ou R' représente un radical acétyle, propionyle ou phényle carbonyle ou un radical phényle,

ou R' représente un radical cycloalkyle ayant de 3 à 8 atomes de carbone ou un radical:

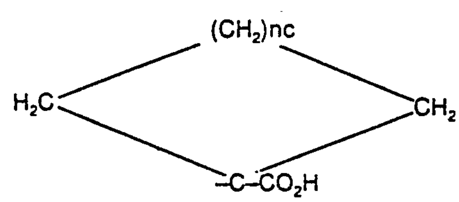

salifié ou estérifié dans lequel nc représente un entier de 0 à 5.

$R'_1$ représente un atome d'hydrogène ou un radical alkyle, ayant au plus 12 atomes de carbone éventuellement substitué par un ou plusieurs des radicaux choisis dans le groupe formé par les radicaux halogène, azido, hydroxyle, mercapto, phényle, amino, nitrile, alkylthio ayant de 1 à 4 atomes de carbone ou phénylthio éventuellement oxydés, acétyl propionyle, benzoyle, acétoxy, propionyloxy, benzoyloxy, acétylamino, benzylcarbonyle, carbamoyloxy, méthyl ou diméthyl carbamoyloxy ou $R'_1$ représente un radical alkényle ou alkynyle ayant au plus 12 atomes de carbone éventuellement substitué par un radical phényle ou par un ou plusieurs atomes d'halogènes, ou $R'_1$ représente un radical thioalkyle éventuellement substitué par carbamoyle,

ou $R'_2$ représente un radical phényle éventuellement substitué par halogène, $CF_3$, amino, hydroxy, alkyle ou alkoxy ou $R'_1$ représente un radical carboxy estérifié, un radical carbamoyle ou un radical azido,

$R'_2$ représente un radical tétrazolyle, triazolyle, imidazolyle, pyrazolyle, ou pyrrolyle, éventuellement substitué par un ou plusieurs des radicaux choisis dans le groupe formé par les radicaux nitro, carboxy, $CF_3$, nitrile, halogène, sulfo, alkylsulfo, $(CH_2)_nSO_3H$; $(CH_2)_nNHSO_3H$; $(CH_2)_nSO_2NH_2$; $(CH_2)_nCO_2H$ dans lesquels n représente un entier de 1 à 4, les produits ayant l'isomérie syn, le trait ondulé signifie que les produits peuvent se trouver sous la forme cis ou trans, ou sous la forme d'un mélange cis-trans, les produits de formule (I') étant sous forme racémique ou optiquement active ainsi que les sels des produits de formule (I') avec les bases et les acides.

4. Procédé selon la revendication 3, caractérisé en ce que l'on utilise au départ des composés de formule (II) et (III) choisis de manière telle que l'on prépare les composés de formule générale (I') dans laquelle R' représente un atome d'hydrogène, un radical méthyle, phényle, difluorométhyle, 1-méthyl 1-carboxyéthyle, cyanométhyle, carboxyméthyle (méthylsulfonyl) carbamoylméthyle; ou un radical

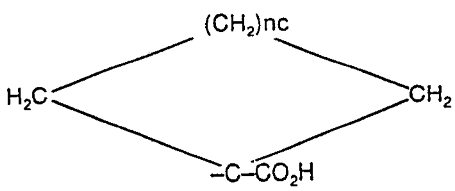

salifié ou estérifié dans lequel nc représente un entier de 0 à 5.

$R'_1$ représente un atome d'hydrogène, un radical méthyle, fluorométhyle, trifluorométhyle, éthoxycarbonyle, carbamoyle;

$R'_2$ représente un radical 1H-tétrazol-5-yle, ou 1,3,4-triazol-2-yle éventuellement substitué par un radical trifluorométhyle ou carboxyméthyle.

5. Procédé selon la revendication 2, de préparation des produits de formule (I) telle que définie à la revendication 1, caractérisé en ce que l'on utilise, pour la mise en œuvre de procédé, un produit de formule (II) dans laquelle $R_1p$ représente un atome d'hydrogène, un radical méthyle, fluorométhyle, trifluorométhyle, éthoxycarbonyle ou carbamoyle et $R_2p$ représente un radical 1H-tétrazol 5-yl ou 1,3,4-triazol 2-yl éventuellement substitué par un radical trifluorométhyle ou carboxyméthyle et un produit de formule (III) dans laquelle Rb représente un groupement protecteur du radical amino et Rp représente un radical protecteur du radical hydroxyle, un radical méthyle, phényle, difluorométhyle, 1-méthyl 1-carboxy éthyle, cyanométhyle, carboxyméthyle (méthylsulfonyl) carbamoylméthyle ou un radical:

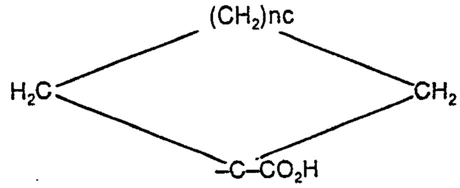

salifié ou estérifié dans lequel nc représente un entier de 0 à 5.

6. Procédé selon la revendication 3 pour la préparation des produits de formule (I') dans laquelle R' représente un radical:

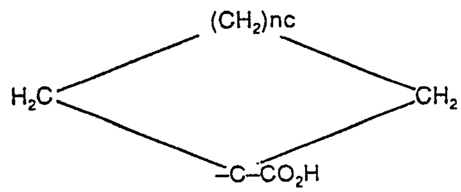

estérifié ou salifié dans lequel nc représente un entier de 0 à 5 et $R'_1$ représente un radical fluorométhyle.

7. Procédé selon la revendication 1, caractérisé en ce que l'on utilise au départ des composés de formule (II) et (III) choisis de manière telle que l'on prépare les composés de formule (I) dont les noms suivent:

– la 3-[[2-(2-aminothiazol-4-yl)2-méthoxyimino acétyl]amino] 4-méthyl 1-(1H-tétrazol-5-yl)2-azétidinone cis ou trans, isomère syn, racémique ou optiquement active;

– la 3-[[2-(2-aminothiazol-4-yl)2-carboxy-méthoxy imino acétyl]amino]4-méthyl 1-(1H-tétrazol-5-yl)2-azétidinone cis ou trans, isomère syn, racémique ou optiquement active;

– la 3-[[2-(2-aminothiazol-4-yl) 2-(1-carboxy-1-méthyl) éthoxyimino acétyl]amino] 4-méthyl 1-(1H-tétrazol-5-yl)2-azétidinone cis ou trans, isomère syn, racémique ou optiquement active;

– la 3-[[2-(2-amino thiazol-4-yl)2-méthoxy imino acétyl]amino]4-fluorométhyl 1-(1H-tétrazol-5-yl)2-azétidinone cis ou trans, isomère syn, racémique ou optiquement active;

– la 3-[[2-(2-amino thiazol-4-yl)2-fluoro-méthoxyiminoacétyl]amino]4-méthyl 1-(1H-tétrazol-5-yl)2-azétidinone cis ou trans, isomère syn, racémique ou optiquement active.

8. Procédé selon la revendication 1 de préparation des produits de formule (I) caractérisé en ce que les produits de formule (II):

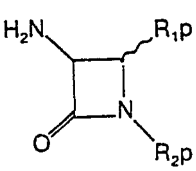

(II)

sont préparés en faisant réagir un produit de formule (V):

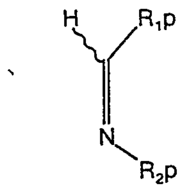

(V)

dans laquelle $R_1p$ et $R_2p$ ont la signification indiquée à la revendication 1, en présence d'une base forte, avec un produit de formule (VI):

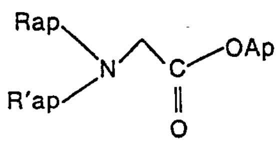

(VI)

dans laquelle Ap représente un atome d'hydrogène ou un groupement ester, Rap et R'ap sont tels que, soit Rap et R'ap représentent chacun un atome d'hydrogène, soit l'un représente un atome d'hydrogène et l'autre représente un groupement protecteur du radical amino, soit Rap et R'ap forment ensemble un radical divalent protecteur du radical amino, pour obtenir un produit de formule (VII):

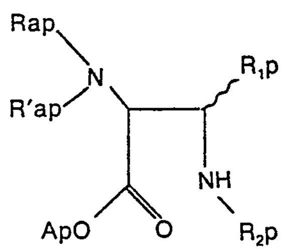

(VII)

dans laquelle Ap, $R_1p$, $R_2p$, Rap et R'ap conservent la signification précédente et le trait ondulé signifie que le substituant $R_1p$ peut se trouver en position α ou β, produit de formule (VII) que, si désiré, l'on soumet à l'une ou l'autre des réactions suivantes, dans un ordre quelconque:

a) séparation des deux isomères;

b) protection du radical $NH_2$ lorsque Rap et R'ap représentent chacun un atome d'hydrogène, produit de formule (VII), sous forme d'un seul isomère ou d'un mélange d'isomères, que l'on soumet, lorsque Ap représente un groupement ester, à un agent de saponification, puis un agent de β-lactamisation, pour obtenir un produit de formule (VIII):

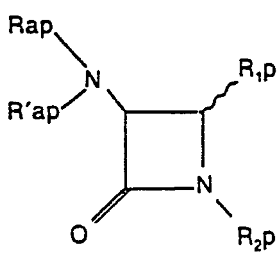

(VIII)

produit que l'on soumet, si nécessaire et, si désiré, à l'une quelconque des réactions suivantes, dans un ordre quelconque:

a) séparation des isomères;

b) coupure par hydrolyse, hydrogénolyse ou action de la thiourée de l'un des radicaux Rap et R'ap ou de ces deux radicaux lorsque l'un représente un groupement protecteur ou les deux représentent ensemble un groupement protecteur, pour obtenir un produit de formule (II) attendu.

9. Procédé selon la revendication 1 de préparation des produits de formule (I) caractérisé en ce que les produits de formule (II):

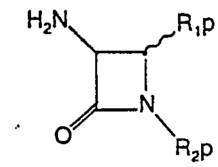

(II)

sont préparés en faisant réagir une β-lactone de formule (IX):

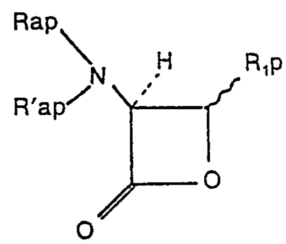

(IX)

dans laquelle $R_1p$, Rap et R'ap ont la signification indiquée à la revendication 7, avec un produit de formule (A):

$H_2N{-}R_2p$ (A)

dans laquelle $R_2p$ a la signification indiquée à la revendication 8, pour obtenir un produit de formule (X):

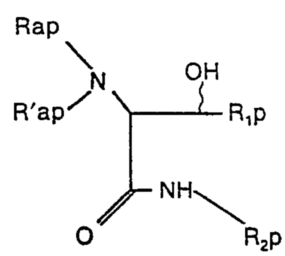

(X)

produit de formule (X) que, si désiré, l'on soumet à l'une ou l'autre des réactions suivantes, dans un ordre quelconque:

a) séparation des isomères dans le cas où $R_1p$ ne représente pas un atome d'hydrogène;

b) protection du radical $NH_2$ lorsque Rap et R'ap représentent chacun un atome d'hydrogène ou modification du groupement protecteur que représente l'un ou l'autre de Rap et R'ap ou que forment ensemble Rap et R'ap et produit de formule (X) sous forme d'un isomère ou d'un mélange d'isomères que l'on soumet à un réactif de cyclisation pour obtenir un produit de formule (VIII):

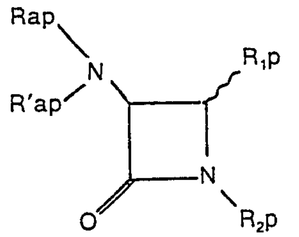

(VIII)

produit de formule (VIII) que l'on peut séparer en ses isomères et que l'on soumet, lorsque Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée pour obtenir un produit de formule (II) attendu.

10. Procédé selon la revendication 9, caractérisé en ce que pour préparer un produit de formule (II) dans laquelle le substituant $R_1p$ représente un atome d'hydrogène ou un radical méthyle, l'on met en œuvre le procédé décrit à la revendication 8 en partant d'un produit de formule (IX) dans laquelle $R_1p$ représente un atome d'hydrogène ou un radical méthyle.

11. Procédé selon la revendication 1 de préparation des produits de formule (I), caractérisé en ce que les produits de formule (II):

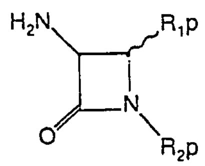

(II)

sont préparés en faisant réagir un produit de formule:

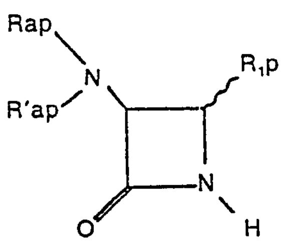

(XI)

dans laquelle $R_1p$, Rap et R'ap sont définis comme précédemment, avec un produit de formule:

$$Y\text{–}R_2p \qquad (XII)$$

dans laquelle $R_2p$ est définis comme précédemment et Y représente un groupement nucléofuge en présence d'une base, pour obtenir un produit de formule (VIII) tel que définis précédemment, que l'on peut séparer en ses isomères et que l'on soumet, lorsque l'un de Rap ou R'ap représente un groupement protecteur du radical amino ou lorsque Rap et R'ap représentent ensemble un radical divalent protecteur du radical amino, à l'action d'un réactif de coupure par hydrolyse, hydrogénolyse ou à l'action de la thiourée, pour obtenir un produit de formule (II) attendu.

12. Procédé selon la revendication 11, caractérisé en ce que les produits de formule (XI) de configuration trans, sont préparés en faisant réagir une base sur un produit de formule ($XI_1$):

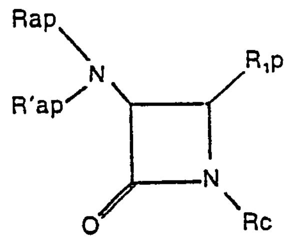

($XI_1$)

de configuration cis, dans laquelle $R_1p$, Rap et R'ap sont définis comme précédemment et Rc représente un atome d'hydrogène ou un groupement protecteur, pour obtenir un produit de formule ($XI_2$):

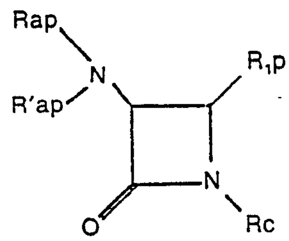

($XI_2$)

de configuration trans, dans laquelle $R_1p$, Rap, R'ap et Rc sont définis comme précédemment, que l'on soumet, le cas échéant, à l'action d'un agent de coupure du groupement protecteur Rc.

**Claims: For the contracting states: BE, SE, CH, DE, FR, GB, IT, LI, LU, NL**

1. Products of general formula (I):

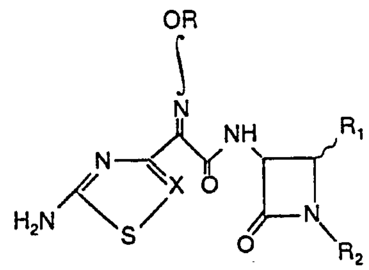

(I)

in which R represents a hydrogen atom, an alkyl, alkenyl or alkynyl radical having at the most 12 carbon atoms, optionally substituted by one or more radicals chosen from carboxy radicals, optionally salified or esterified; amino, methylamino, dimethylamino and diethylamino radicals; the phenyl radical optionally substituted by one or more radicals chosen from the following radicals: hydroxy, methyl, methoxy, chloro, bromo, fluoro; fluoro, chloro, bromo or iodo radicals; nitrile, $CONH_2$ or CONH $SO_2R''$ radicals in which R'' represents an alkyl radical having from 1 to 4 carbon atoms; a phenyl radical, an amino, methyl or dimethylamino radical; a heterocyclic amino radical chosen from piperidino, morpholino piperazino or 4-ethyl-2,3-dioxo-1-piperazino radicals;

– a cycloalkyl radical having from 3 to 8 carbon atoms or a radical:

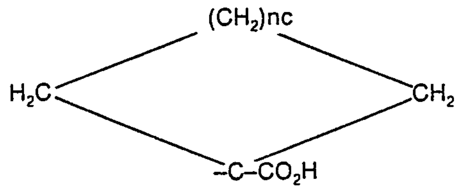

salified or esterified, in which nc represents a whole number from 0 to 5,

– one of the following radicals: acetyl, propionyl or benzoyl, carbamoyl, dimethylamino carbonyl, phenyl or benzyl optionally substituted by alkyl, alkoxy or halogen;

$R_1$ represents a hydrogen atom, an alkyl, alkenyl, alkynyl or thioalkyl radical having a the most 12 carbon atoms, optionally substituted by one or more radicals chosen from azido or alkylthio radicals having from 1 to 4 carbon atoms or a phenylthio radical, optionally salified or esterified carboxy radicals, amino, methylamino, dimethylamino or diethylamino radicals, the phenyl radical, optionally substituted by one or more radicals chosen from hydroxy, halogeno, trifluoromethyl, amino, alkyl or alkoxy radicals having from 1 to 4 carbon atoms; fluoro, chloro, bromo or iodo radicals, nitrile, $CONH_2$ or CONH $SO_2R''$ radicals in which R'' represents an alkyl radical having from 1 to 4 carbon atoms, a phenyl radical, an amino, methyl or dimethylamino radical, a heterocyclic amino radical chosen from piperidino, morpholino, piperazino or 4-ethyl-2,3-dioxo-1-piperazino radicals; a heterocyclic aryl radical chosen from the following radicals: thienyl, furyl, pyrannyl, thiazolyl, thiadiazolyl, oxazolyl, oxadiazolyl, pyridinyl or pyrimidinyl; and the following radicals: acetoxy, propionyloxy, benzoyloxy, acetylamino, benzylcarbonyl, carbamoyloxy, methylaminocarbonyloxy or dimethylaminocarbonyloxy, acetyl, propionyl, and benzoyl,

– a free, esterified or salified carboxy radical,

– a phenyl radical optionally substituted by one of the following radicals: alkyl, trifluoromethyl, alkoxy, alkylthio, halogen, hydroxyl, amino or hydroxyalkyl, a heterocyclic radical chosen from the following radicals: thienyl, furyl, pyrrolyl, thiazolyl, isothiazolyl, oxazolyl, isoxazolyl, imidazolinyl, imidazolyl, triazolyl, tetrazolyl, thiadiazolyl, oxadiazolyl, pyridinyl, pyrimidinyl, pyridazinyl, triazinyl optionally substituted by an alkyl, carboxy, carboxy alkyl, aminoalkyl or dialkylaminoalkyl radical,

– an acetyl, propionyl, n-butyryl or benzoyl radical, optionally substituted by alkoxy or hydroxy,

– a carbamoyl, methyl or dimethylcarbamoyl radical,

– a azido radical.

$R_2$ represents a nitrogen-containing heterocycle optionally substituted by one or more of the radicals chosen from the group formed by the following radicals: nitro, carboxy, $CF_3$, nitrile, halogen, sulpho, alkylsulpho, $(CH_2)_nSO_3H$, $(CH_2)_nNHSO_3H$, $(CH_2)_nSO_2NH_2$, $(CH_2)_nCO_2H$, in which n represents a whole number from 1 to 4 and containing at least one acid hydrogen, X represents a CH radical or a nitrogen atom, the wavy lines indicate that the OR radical can exist in cis or trans form or in the form of a cis-trans mixture, the products of formula (I) being in racemic or optically active form, as well as the salts of the products of formula (I) with the bases and acids.

2. The products according to claim 1 of general formula (I'):

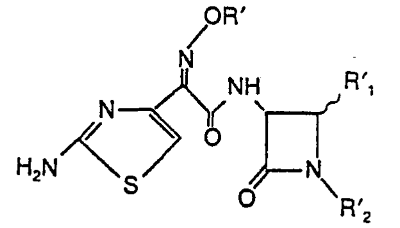

(I')

in which R' represents a hydrogen atom or a linear or branched alkyl radical having from 1 to 6 carbon atoms, optionally substituted by one or more of the radicals chosen from the group formed by the following radicals: free, esterified or salified carboxy radical, amino, mono- or dimethylamino, phenyl, halogen, nitrile, $CONHSO_2R''$ in which R'' represents an alkyl radical having 1 to 4 carbon atoms, phenyl or amino mono or dimethylamino, or R' represents an acetyl, propionyl or phenyl carbonyl radical or a phenyl radical, or R' represents a cycloalkyl radical having 3 to 8 carbon atoms or a radical:

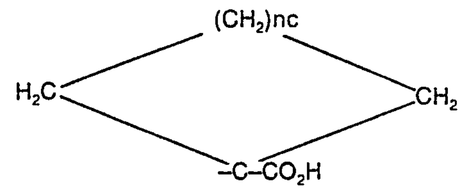

salified or esterified, in which nc represents a whole number from 0 to 5.

R' represents a hydrogen atom or an alkyl radical having at the most 12 carbon atoms, optionally substituted by one or more radicals chosen from the group formed by the following radicals: halogen, azido, hydroxyl, mercapto, phenyl, amino, nitrile, alkylthio having 1 to 4 carbon atoms or optionally oxidized phenylthio, acetyl, propionyl, benzoyl, acetoxy, propionyloxy, benzoyloxy, acetylamino, benzylcarbonyl, carbamoyloxy, methyl or dimethyl carbamoyloxy; or $R'_1$ represents an alkenyl or alkynyl radical having at the most 12 carbon atoms, optionally substituted by a phenyl radical or by one or more halogen atoms, or $R'_1$ represents a thioalkyl radical optionally substituted by carbamoyl; or $R'_1$ represents a phenyl radical optionally substituted by halogen, a $CF_3$, amino, hydroxy, alkyl or alkoxy radical; or $R'_1$ represents an esterified carboxy radical, a carbamoyl radical or an azido radical;

$R'_2$ represents one of the following radicals: tetrazolyl, triazolyl, imidazolyl, pyrazolyl or pyrrolyl, optionally substituted by one or more of the radicals chosen from the group formed by the following radicals: nitro, carboxy, $CF_3$, nitrile,

halogen, sulpho, alkylsulpho, $(CH_2)_nSO_3H$, $(CH_2)_nNHSO_3H$, $(CH_2)_nSO_2NH_2$ or $(CH_2)_nCO_2H$, in which n represents a whole number from 1 to 4, the products having syn isomerism; the wavy line indicates that the products can exist in cis or trans form, or in the form of a cis-trans mixture, the products of formula (I') being in racemic or optically active form, as well as the salts of the products of formula (I') with the bases and acids.

3. The products of general formula (I') as described in claims 2 in which R' represents a hydrogen atom, one of the following radicals: methyl, phenyl, difluoromethyl, 1-methyl-1-carboxy ethyl, cyano methyl, carboxy methyl (methyl sulphonyl) carbamoyl methyl or a radical:

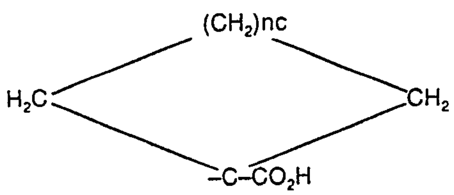

salified or esterified, in which nc represents a whole number from 0 to 5;

$R'_1$ represents a hydrogen atom, a methyl, fluoromethyl, trifluoromethyl, ethoxycarbonyl or carbamoyl radical;

$R'_2$ represents a 1H-tetrazol-5-yl radical, or 1,3,4-triazol-2-yl radical, optionally substituted by a trifluoromethyl or carboxymethyl radical.

4. The products of general formula (I') as described in claim 2 in which R' represents a radical:

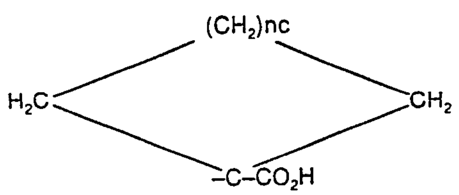

salified or esterified, in which nc represents a whole number from 0 to 5.

5. Any one of the products of formula (I) of which the names follow:

– cis or trans, syn isomer, racemic or optically active
3-[[2-(2-aminothiazol-4-yl)2-methoxyimino acetyl]amino]4-methyl 1-(1H-tetrazol-5-yl)2-azetidinone;

– cis or trans, syn isomer, racemic or optically active
3-[[2-(2-aminothiazol-4-yl)2-carboxymethoxy imino acetyl]amino]4-methyl 1-(1H-tetrazol-5-yl)2-azetidinone;

– cis or trans, syn isomer, racemic or optically active
3-[[2-(2-aminothiazol-4-yl)-2-(1-carboxy-1-methyl) ethoxyimino acetyl]amino]4-methyl 1-(1H-tetrazol-5-yl)2-azetidinone;

– cis or trans, syn isomer, racemic or optically active
3-[[2-(2-aminothiazol-4-yl)2-methoxyimino-acetyl]amino]4-fluoromethyl-1-(1H-tetrazol-5-yl)2-azetidinone;

– cis or trans, syn isomer, racemic or optically active
3-[[2-(2-aminothiazol-4-yl)2-fluoromethoxy-iminoacetyl]amino]4-methyl 1-(1H-tetrazol-5-yl)2-azetidinone.

6. Preparation process for products of general formula (I) as defined in claim 1, characterized in that a product of formula (II):

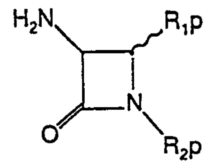

(II)

cis or trans, racemic or optically active, in which formula either $R_1p$ represents $R_1$, $R_1$ having the significance indicated in claim 1, or $R_1p$ represents the substituent $R_1$ in which the reactive functions are protected and either $R_2p$ represents $R_2$, $R_2$ having the significance indicated in claim 1, or $R_2p$ represents the substituent $R_2$ in which the reactive functions are protected, is treated by a product of formula (III):

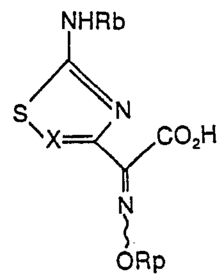

(III)

syn or anti, in which Rb represents a hydrogen atom or a protector group of the amino radical, and Rp represents a protector group of the hydroxyl radical, or Rp represents R, R having the significance indicated in claim 1, or Rp represents a radical R in which the reactive functions are protected, so as to obtain a product of formula (IV):

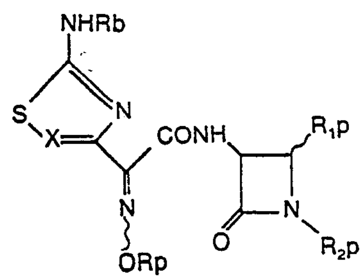

(IV)

syn or anti, racemic or optically active, in which X, Rp, $R_1p$, $R_2p$ and Rb have the previous significance, which product is submitted, if necessary and if desired, to any one of the following reactions, in any order:

a) cleavage by hydrolysis, hydrogenolysis or by the action of the thiourea of the protector group or groups which can be represented by Rb and Rp or which can be contained in Rp, $R_1p$ and $R_2p$;

b) esterification or salification of the carboxy or sulpho radicals which can be contained in the radicals Rp, $R_1p$ and $R_2p$;

c) salification by an acid of the amino radical or radicals;

d) resolution of the molecule so as to obtain an optically active product.

7. Process according to claim 6, for the preparation of products of formula (I) as defined in claim 1, characterized in that, for the operation of the process, a product of formula (II) is used in which $R_1p$ represents a hydrogen atom, a methyl, fluoromethyl, trifluoromethyl, ethoxycarbonyl or carbamoyl radical, and $R_2p$ represents a 1H-tetrazol-5-yl or 1,3,4-triazol-2-yl radical optionally substituted by a trifluoromethyl or carboxymethyl radical, and product of formula (III) in which Rb represents a protector group of the amino radical and Rp represents a protector radical of the hydroxyl radical, or one of the following radicals: methyl, phenyl, difluoromethyl, 1-methyl 1-carboxy ethyl, cyanomethyl, carboxymethyl or methylsulphonyl carbamoylmethyl.

8. Process according to claim 6 for the preparation of products of formula (I), characterized in that the products of formula (II):

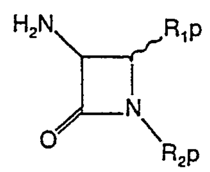

(II)

are prepared by reacting a product of formula (V):

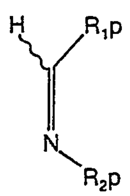

(V)

in which $R_1p$ and $R_2p$ have the significance indicated in claim 6, in the presence of a strong base, with a product of formula (VI):

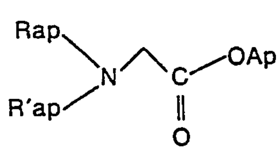

(VI)

in which Ap represents a hydrogen atom or an ester group, Rap and R'ap are such that either Rap and R'ap each represents a hydrogen atom, or one represents a hydrogen atom and the other represents a protector group of the amino radical, or Rap and R'ap together form a divalent protector radical of the amino radical, so as to obtain a product of formula (VII):

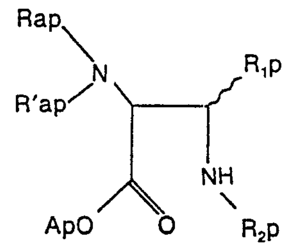

(VII)

in which Ap, $R_1p$, $R_2p$, Rap and R'ap keep the previous significance and the wavy line indicates that the substituent $R_1p$ can exist in alpha or beta position, which product of formula (VII) is submitted, if desired, to one or other of the following reactions, in any order:

a) separation of the two isomers;

b) protection of the $NH_2$ radical when Rap and R'ap each represents a hydrogen atom, which product of formula (VII), in the form of a single isomer or a mixture of isomers, is submitted, when Ap represents an ester group, to a saponification agent, then to a beta-lactamization agent, so as to obtain a product of formula (VIII):

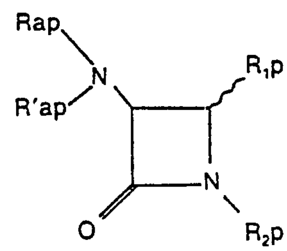

(VIII)

which product is submitted, if necessary and if desired, to any one of the following reactions, in any order:

a) separation of the isomers;

b) cleavage by hydrolysis, hydrogenolysis or by the action of the thiourea of one of the radicals Rap and R'ap or of these two radicals when one represents a protector group or both represent together a protector group, so as to obtain an expected product of formula (II).

9. Process according to claim 6 for the preparation of products of formula (I) characterized in that the products of formula (II):

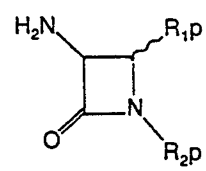

(II)

are prepared by reacting a beta-lactone of formula (IX):

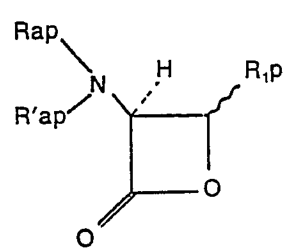

(IX)

in which $R_1p$, Rap and R'ap have the significance indicated in claim 8, with a product of formula (A):

$$H_2N\text{–}R_2p \qquad (A)$$

in which $R_2p$ has the significance indicated in claim 8, so as to obtain a product of formula (X):

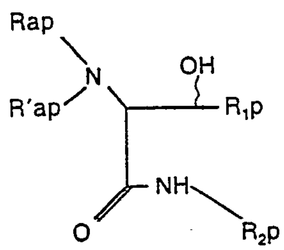

(X)

which product of formula (X) is, if desired, submitted to one or other of the following reactions, in any order:

a) separation of the isomers when $R_1p$ does not represent a hydrogen atom;

b) protection of the $NH_2$ radical when Rap and R'ap each represents a hydrogen atom or modification of the protector group represented by one or other of Rap and R'ap or which is formed by Rap and R'ap together, and which product of formula (X), in the form of an isomer or of a mixture of isomers, is submitted to a cyclization reagent so as to obtain a product of formula (VIII):

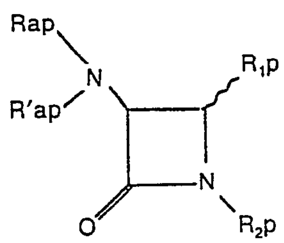

(VIII)

which product of formula (VIII) can be separated into its isomers and which is submitted, when Rap or R'ap represents a protector group of the amino radical or when Rap and R'ap together represent a divalent protector radical of the amino radical, to the action of a cleavage reagent by hydrolysis, hydrogenolysis or to the action of the thiourea so as to obtain an expected product of formula (II).

10. Process according to claim 9, characterized in that in order to prepare a product of formula (II) in which the substituent $R_1p$ represents a hydrogen atom or a methyl radical, the process described in claim 8 is put into operation, starting with a product of formula (IX) in which $R_1p$ represents a hydrogen atom or a methyl radical.

11. Process according to claim 6 for the preparation of products of formula (I), characterized in that the products of formula (II):

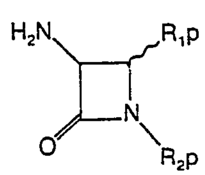

(II)

are prepared by reacting a product of formula:

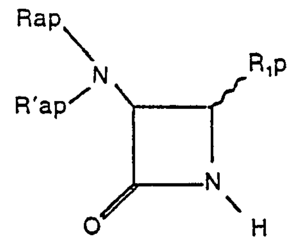

(XI)

in which $R_1p$, Rap and R'ap are defined as previously, with a product of formula:

$$Y\text{–}R_2p \qquad (XII)$$

in which $R_2p$ is defined as previously and Y represents a nucleofugal group in the presence of a base, so as to obtain a product of formula (VIII) as defined previously, which can be separated into its isomers and which is submitted, when either Rap or R'ap represents a protector group of the amino radical or when Rap and R'ap together represent a divalent protector radical of the amino radical, to the action of a cleavage reagent by hydrolysis or hydrogenolysis or to the action of the thiourea, so as to obtain an expected product of formula (II).

12. Process according to claim 11, characterized in that the products of formula (XI) of trans configuration, are prepared by reacting a base on a product of formula ($XI_1$):

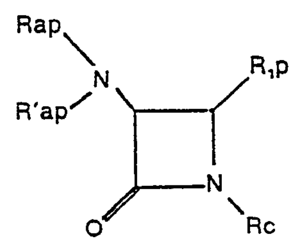

($XI_1$)

of cis configuration, in which $R_1p$, Rap and R'ap are defined as previously and Rc represents a hydrogen atom or a protector group, so as to obtain a product of formula ($XI_2$):

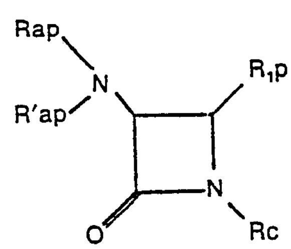

($XI_2$)

of trans configuration, in which $R_1p$, Rap, R'ap and Rc are defined as previously, which is submitted, if appropriate, to the action of a cleavage agent of the Rc protector group.

13. As medicaments, the products corresponding to the formula (I) as defined in claim 1 and their pharmaceutically acceptable salts.

14. As medicaments, the products corresponding to the formula (I') as defined in claims 2 to 4 and their pharmaceutically acceptable salts.

15. As medicaments, the products defined in claim 5.

16. Pharmaceutical compositions containing, as active principle, at least one medicament according to one of claims 13 to 15.

17. As new industrial products, the products of general formula (II):

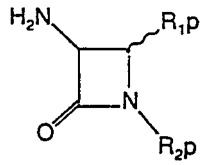

(II)

in which $R_1p$ and $R_2p$ have the significance indicated in claim 6.

18. As new industrial products, the products of general formula (II) as described in claim 17 in which $R_1p$ represents a hydrogen atom or an alkyl radical having at the most 12 carbon atoms, optionally substituted by one or more radicals chosen from the group formed by the following radicals: halogen, azido, hydroxyl, mercapto, phenyl, amino, nitrile, alkylthio having from 1 to 4 carbon atoms or optionally oxidized phenylthio; acetyl, propionyl, benzoyl, acetoxy, propionyloxy, benzoyloxy, acetylamino, benzylcarbonyl, carbamoyloxy, methyl or dimethylcarbamoyloxy, and $R_2p$ represents an optionally protected tetrazolyl radical.

19. As new industrial products, the products of general formula (II) as described in claim 17 in which $R_1p$ represents a fluoromethyl radical and $R_2p$ represents an optionally protected tetrazolyl radical.

**Claims: For the contracting state AT**

1. Process for preparing the products of general formula (I):

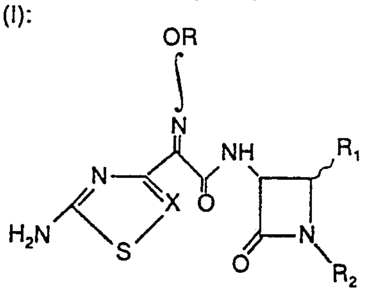

(I)

in which R represents a hydrogen atom, an alkyl, alkenyl or alkynyl radical having at the most 12 carbon atoms, optionally substituted by one or more radicals chosen from carboxy radicals, optionally salified or esterified; amino, methylamino, dimethylamino and diethylamino radicals; the phenyl radical optionally substituted by one or more radicals chosen from the following radicals: hydroxy, methyl, methoxy, chloro, bromo, fluoro; fluoro, chloro, bromo or iodo radicals; nitrile, $CONH_2$ or CONH $SO_2R''$ radicals in which R'' represents an alkyl radical having from 1 to 4 carbon atoms; a phenyl radical; an amino, methyl or dimethylamino radical; a heterocyclic amino radical chosen from piperidino, morpholino piperazino or 4-ethyl-2,3-dioxo-1-piperazino radicals;

– a cycloalkyl radical having from 3 to 8 carbon atoms or a radical:

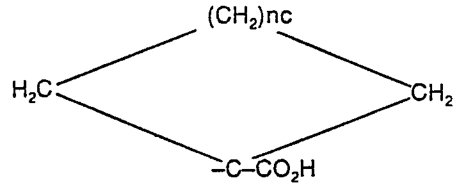

salified or esterified, in which nc represents a whole number from 0 to 5,

– one of the following radicals: acetyl, propionyl or benzoyl, carbamoyl, dimethylamino carbonyl, phenyl or benzyl optionally substituted by alkyl, alkoxy or halogen;

$R_1$ represents a hydrogen atom, an alkyl, alkenyl, alkynyl or thioalkyl radical having at the most 12 carbon atoms, optionally substituted by one or more radicals chosen from azido or alkylthio radicals having from 1 to 4 carbon atoms or a phenylthio radical, optionally salified or esterified carboxy radicals, amino, methylamino, dimethylamino or diethylamino radicals, the phenyl radical, optionally substituted by one or more radicals chosen from hydroxy, halogeno, trifluoromethyl, amino, alkyl or alkoxy radicals having from 1 to 4 carbon atoms; fluoro, chloro, bromo or iodo radicals, nitrile, $CONH_2$ or CONH $SO_2R''$ radicals in which R'' represents an alkyl radical having from 1 to 4 carbon atoms, a phenyl radical, an amino, methyl or dimethylamino radical, a heterocyclic amino radical chosen from piperidino, morpholino, piperazino or 4-ethyl-2,3-dioxo-1-piperazino radicals; a heterocyclic aryl radical chosen from the following radicals: thienyl, furyl, pyrannyl, thiazolyl, thiadiazolyl, oxazolyl, oxadiazolyl, pyridinyl or pyrimidinyl; and the following radicals: acetoxy, propionyloxy, benzoyloxy, acetylamino, benzylcarbonyl, carbamoyloxy, methylaminocarbonyloxy or dimethylaminocarbonyloxy, acetyl, propionyl, and benzoyl,

– a free, esterified or salified carboxy radical,

– a phenyl radical optionally substituted by one of the following radicals: alkyl, trifluoromethyl, alkoxy, alkylthio, halogen, hydroxyl, amino or hydroxyalkyl, a heterocyclic radical chosen from the following radicals: thienyl, furyl, pyrolyl, thiazolyl, isothiazolyl, oxazolyl, isoxazolyl, imidazolinyl, imidazolyl, triazolyl, tetrazolyl, thiadiazolyl, oxadiazolyl, pyridinyl, pyrimidinyl, pyridazinyl, triazinyl optionally substituted by an alkyl, carboxy, carboxy alkyl, aminoalkyl or dialkylaminoalkyl radical,

– an acetyl, propionyl, n-butyryl or benzoyl radical, optionally substituted by alkoxy or hydroxy,

– a carbamoyl, methyl or dimethylcarbamoyl radical,

– an azido radical.

$R_2$ represents a nitrogen-containing heterocycle optionally substituted by one or more of the radicals chosen from the group formed by the following radicals: nitro, carboxy, $CF_3$, nitrile, halogen, sulpho, alkylsulpho, $(CH_2)_nSO_3H$, $(CH_2)_nNHSO_3H$, $(CH_2)_nSO_2NH_2$, $(CH_2)_nCO_2H$, in which n represents a

whole number from 1 to 4 and containing at least one acid hydrogen, X represents a CH radical or a nitrogen atom, the wavy lines indicate that the OR radical can exist in cis or trans form or in the form of a cis-trans mixture, the products of formula (I) being in racemic or optically active form, as well as the salts of the products of formula (I) with the acids and bases, characterized in that a product of formula (II):

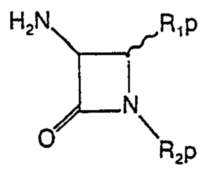

(II)

cis or trans, racemic or optically active, in which formula either $R_1p$ represents $R_1$, $R_1$ having the previously indicated significance, or $R_1p$ represents the substituent $R_1$ in which the reactive functions are protected and either $R_2p$ represents $R_2$, $R_2$ having the previously indicated significance, or $R_2p$ represents the substituent $R_2$ in which the reactive functions are protected, is treated with a product of formula (III):

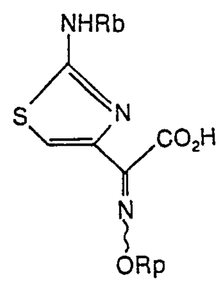

(III)

syn or anti, in which Rb represents a hydrogen atom or a protector group of the amino radical and Rp represents a protector group of the hydroxyl radical or Rp represents R, R having the significance indicated in claim 1, or Rp represents a radical R in which the reactive functions are protected, so as to obtain a product of formula (IV):

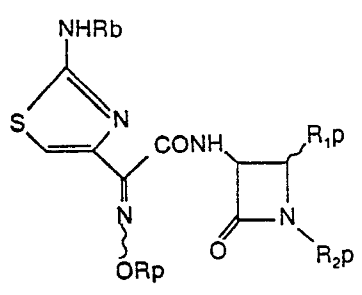

(IV)

syn or anti, racemic or optically active, in which Rp, $R_1p$, $R_2p$ and Rb have the previous significance, which product is submitted, if necessary and if desired, to any one of the following reactions, in any order:

a) cleavage by hydrolysis, hydrogenolysis or by the action of the thiourea of the protector group or groups which can be represented by Rb and Rp or can be contained in Rp, $R_1p$ and $R_2p$;

b) esterification or salification of the carboxy or sulpho radicals which can be contained in the radicals Rp, $R_1p$ and $R_2p$;

c) salification by an acid of the amino radical or radicals;

d) resolution of the molecule so as to obtain an optically active product.

2. Process according to claim 5, for the preparation of products of formula (I) as defined in claim 1, corresponding to the formula ($I_a$):

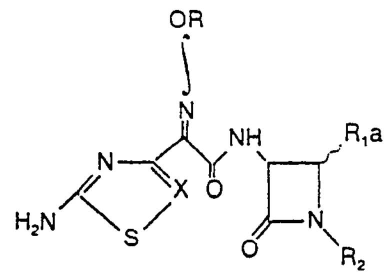

($I_a$)

in which R, $R_2$ and X have the significance indicated in claim 1 and $R_1a$ has the significance indicated in claim 1 for $R_1$ with the exception of azido, characterized in that at the start a product of formula (II) is used in which $R_1p$ has the values indicated in claim 1, with the exception of an azido radical.

3. Process according to claim 2, characterized in that at the start compounds of formula (II) and (III) are used chosen in such a way that compounds of general formula (I′) are prepared:

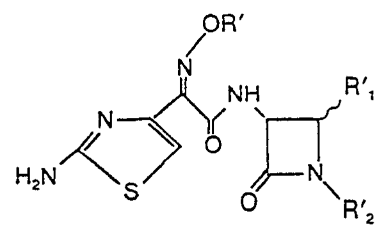

(I′)

in which R′ represents a hydrogen atom or a linear or branched alkyl radical having 1 to 6 carbon atoms, optionally substituted by one or more radicals chosen from the group formed by the following radicals: free, esterified or salified carboxy, amino, mono- or dimethylamino, phenyl, halogen, nitrile, $CONHSO_2R''$ in which R″ represents an alkyl radical having 1 to 4 carbon atoms, phenyl or amino mono or dimethylamino, or R′ represents an acetyl, propionyl or phenyl carbonyl radical, or a phenyl radical, or R′ represents a cycloalkyl radical having 3 to 8 carbon atoms or a radical:

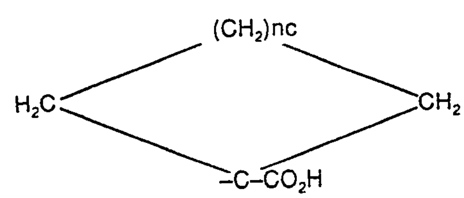

salified or esterified, in which nc represents a whole number from 0 to 5,

$R'_1$ represents a hydrogen atom or an alkyl radical having at the most 12 carbon atoms, optionally substituted by one or more radicals chosen from the group formed by the following radicals: halogen, azido, hydroxyl, mercapto, phenyl, amino,

nitrile, alkylthio having 1 to 4 carbon atoms or optionally oxidized phenylthio, acetyl, propionyl, benzoyl, acetoxy, propionyloxy, benzoyloxy, acetylamino, benzylcarbonyl, carbamoyloxy, methyl or dimethyl carbamoyloxy; or $R'_1$ represents an alkenyl or alkynyl radical having at the most 12 carbon atoms, optionally substituted by a phenyl radical or by one or more halogen atoms, or $R'_1$ represents a thioalkyl radical optionally substituted by carbamoyl; or $R'_1$ represents a phenyl radical optionally substituted by halogen, a $CF_3$, amino, hydroxy, alkyl or alkoxy radical; or $R'_1$ represents an esterified carboxy radical, a carbamoyl radical or an azido radical;

$R'_2$ represents one of the following radicals: tetrazolyl, triazolyl, imidazolyl, pyrazolyl or pyrrolyl, optionally substituted by one or more of the radicals chosen from the group formed by the following radicals: nitro, carboxy, $CF_3$, nitrile, halogen, sulpho, alkylsulpho, $(CH_2)_nSO_3H$, $(CH_2)_nNHSO_3H$, $(CH_2)_nSO_2NH_2$ or $(CH_2)_nCO_2H$, in which n represents a whole number from 1 to 4, the products having syn isomerism; the wavy line indicates that the products can exist in cis or trans form, or in the form of a cis-trans mixture, the products of formula (I') being in racemic or optically active form, as well as the salts of the products of formula (I') with the bases and acids.

4. Process according to claim 3, characterized in that at the start compounds of formula (II) and (III) are used, chosen in such a way that compounds of general formula (I') are prepared in which R' represents a hydrogen atom, one of the following radicals: methyl, phenyl, difluoromethyl, 1-methyl 1-carboxyethyl, cyanomethyl, carboxymethyl, (methylsulphonyl) carbamoylmethyl; or a radical:

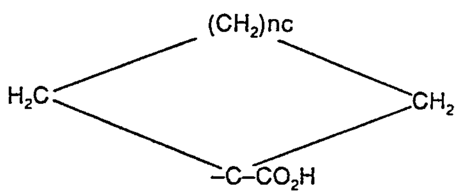

salified or esterified, in which nc represents a whole number from 0 to 5,

$R'_1$ represents a hydrogen atom, or a methyl, fluoromethyl, trifluoromethyl, ethoxycarbonyl or carbamoyl radical;

$R'_2$ represents a 1H-tetrazol-5-yl or 1,3,4-triazol-2-yl radical, optionally substituted by a trifluoromethyl or carboxymethyl radical.

5. Process according to claim 2, for the preparation of products of formula (I) as defined in claim 1, characterized in that, for putting the process into operation, a product of formula (II) is used, in which $R_1p$ represents a hydrogen atom, or a methyl, fluoromethyl, trifluoromethyl, ethoxycarbonyl or carbamoyl radical, and $R_2p$ represents a 1H-tetrazol-5-yl or 1,3,4-triazol-2-yl radical optionally substituted by a trifluoromethyl or carboxymethyl radical, and a product of formula (III) is used in which Rb represents a protector group of the amino radical and Rp represents a protector radical of the hydroxyl radical, one of the following radicals: methyl, phenyl, difluoromethyl, 1-methyl 1-carboxy ethyl, cyanomethyl, carboxymethyl (methylsulphonyl) carbamoylmethyl or a radical:

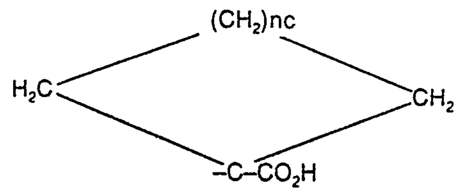

salified or esterified, in which nc represents a whole number from 0 to 5.

6. Process according to claim 3 for the preparation of products of formula (I') in which R' represents a radical:

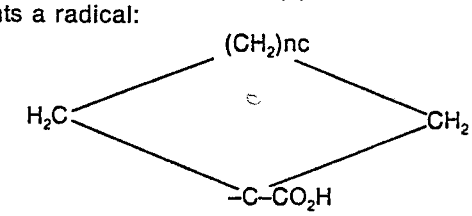

esterified or salified, in which nc represents a whole number from 0 to 5 and $R'_1$ represents a fluoromethyl radical.

7. Process according to claim 1, characterized in that at the start compounds of formula (II) and (III) are used, chosen in such a way that the compounds of formula (I) are prepared of which the names follow:

– cis or trans, syn isomer, racemic or optically active
3-[[2-(2-aminothiazol-4-yl)2-methoxyimino acetyl]amino]4-methyl 1-(1H-tetrazol-5-yl)2-azetidinone;

– cis or trans, syn isomer, racemic or optically active
3-[[2-(2-aminothiazol-4-yl)2-carboxymethoxy imino acetyl]amino]4-methyl 1-(1H tetrazol-5-yl)2-azetidinone;

– cis or trans, syn isomer, racemic or optically active
3-[[2-(2-aminothiazol-4-yl)-2-(1-carboxy-1-methyl) ethoxyimino acetyl]amino]4-methyl 1-(1H-tetrazol-5-yl)2-azetidinone;

– cis or trans, syn isomer, racemic or optically active
3-[[2-(2-aminothiazol-4-yl)2-methoxyimino-acetyl]amino]4-fluoromethyl-1-(1H-tetrazol-5-yl)2-azetidinone;

– cis or trans, syn isomer, racemic or optically active
3-[[2-(2-aminothiazol-4-yl)2-fluoromethoxy-iminoacetyl]amino]4-methyl 1-(1H-tetrazol-5-yl)2-azetidinone.

8. Process according to claim 1 for the preparation of products of formula (I) characterized in that the products of formula (II):

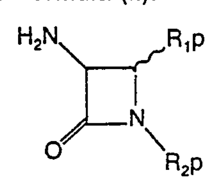

(II)

are prepared by reacting a product of formula (V):

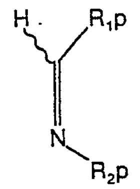

(V)

in which $R_1p$ and $R_2p$ have the significance indicated in claim 1, in the presence of a strong base, with a product of formula (VI):

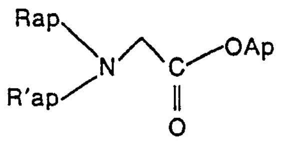

(VI)

in which Ap represents a hydrogen atom or an ester group, Rap and R'ap are such that either Rap and R'ap each represents a hydrogen atom, or one represents a hydrogen atom and the other represents a protector group of the amino radical, or Rap and R'ap together form a divalent protector radical of the amino radical, so as to obtain a product of formula (VII):

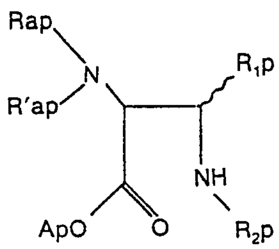

(VII)

in which Ap, $R_1p$, $R_2p$, Rap and R'ap keep the previous significance and the wavy line indicates that the substituent $R_1p$ can exist in alpha or beta position, which product of formula (VII) is, if desired, submitted to one or other of the following reactions, in any order:

a) separation of the two isomers;

b) protection of the $NH_2$ radical when Rap and R'ap each represents a hydrogen atom, which product of formula (VII), in the form of a single isomer or a mixture of isomers, is submitted, when Ap represents an ester group, to a saponification agent, then to a beta-lactamization agent, so as to obtain a product of formula (VIII):

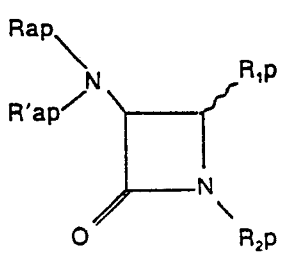

(VIII)

which product is submitted, if necessary and if desired, to any one of the following reactions, in any order:

a) separation of the isomers;

b) cleavage by hydrolysis, hydrogenolysis or by the action of the thiourea of one of the radicals Rap and R'ap or of these two radicals when one represents a protector group or both together represent a protector group, so as to obtain an expected product of formula (II).

9. Process according to claim 1 for the preparation of products of formula (I) characterized in that the products of formula (II):

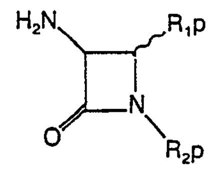

(II)

are prepared by reacting a beta-lactone of formula (IX):

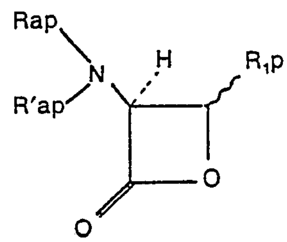

(IX)

in which $R_1p$, Rap and R'ap have the significance indicated in claim 7, with a product of formula (A):

$H_2N-R_2p$ (A)

in which $R_2p$ have the significance indicated in claim 8, so as to obtain a product of formula (X):

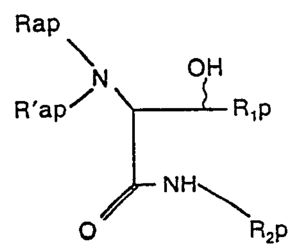

(X)

which product of formula (X), if desired, is submitted to one or other of the following reactions, in any order:

a) separation of the isomers when $R_1p$ does not represent a hydrogen atom;

b) protection of the $NH_2$ radical when Rap and R'ap each represents a hydrogen atom or modification of the protector group which is represented by one or other of Rap and R'ap or which is formed by Rap and R'ap together, and which product of formula (X) in the form of an isomer or a mixture of isomers is submitted to a cyclization reagent so as to obtain a product of formula (VIII):

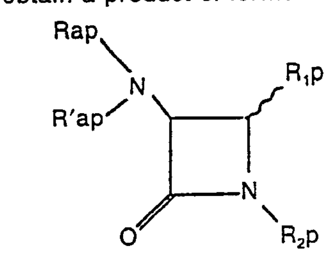

(VIII)

which product of formula (VIII) can be separated into its isomers and which is submitted, when Rap or R'ap represents a protector group of the amino radical or when Rap and R'ap together represent a divalent protector radical of the amino radical, to the action of a cleavage reagent by hydrolysis, hydrogenolysis or to the action of the thiourea so as to obtain an expected product of formula (II).

10. Process according to claim 9, characterized that in order to prepare a product of formula (II) in which the substituent $R_1p$ represents a hydrogen atom or a methyl radical, the process described in claim 8 is put into operation, starting with a product of formula (IX) in which $R_1p$ represents a hydrogen atom or a methyl radical.

11. Process according to claim 1 for the preparation of products of formula (I), characterized in that the products of formula (II):

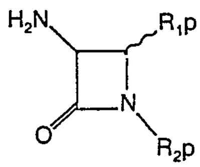

(II)

are prepared by reacting a product of formula:

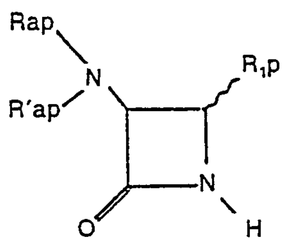

(XI)

in which $R_1p$, Rap and R'ap are defined as previously, with a product of formula:

$$Y{-}R_2p \qquad \text{(XII)}$$

in which $R_2p$ is defined as previously and Y represents a nucleofugal group in the presence of a base, so as to obtain a product of formula (VIII) as defined previously, which can be separated into its isomers and which is submitted, when either Rap or R'ap represents a protector group of the amino radical or when Rap and R'ap together represent a divalent protector radical of the amino radical, to the action of a cleavage reagent by hydrolysis or hydrogenolysis or to the action of the thiourea, so as to obtain an expected product of formula (II).

12. Process according to claim 11, characterized in that the products of formula (XI) of trans configuration, are prepared by reacting a base on a product of formula $(XI_1)$:

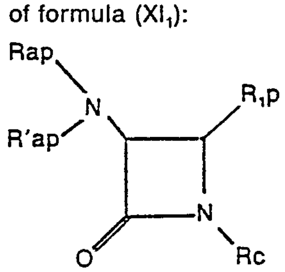

$(XI_1)$

of cis configuration, in which $R_1p$, Rap and R'ap are defined as previously and Rc represents a hydrogen atom or a protector group, so as to obtain a product of formula $(XI_2)$:

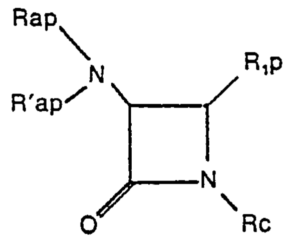

$(XI_2)$

of trans configuration, in which $R_1p$, Rap, R'ap and Rc are defined as previously, which is submitted, if appropriate, to the action of a cleavage agent of the Rc protector group.

**Patentansprüche für die Vertragsstaaten: BE, SE, CH, DE, FR, GB, IT, LI, LU, NL**

1. Produkte der allgemeinen Formel (I)

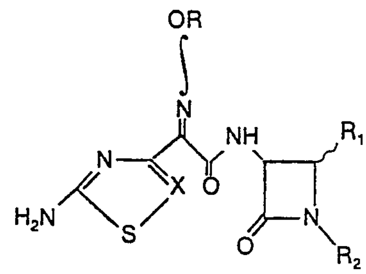

(I)

worin

R ein Wasserstoffatom, einen Alkyl-, Alkenyl- oder Alkinylrest mit höchstens 12 Kohlenstoffatomen, gegebenenfalls substituiert durch einen oder mehrere Reste, ausgewählt unter den gegebenenfalls der Salzbildung oder der Veresterung unterzogenen Carboxyresten, den Amino-, Methylamino-, Dimethylamino- und Diethylaminogruppen, dem Phenylrest, der gegebenenfalls substituiert ist durch einen oder mehrere Reste, ausgewählt unter den Hydroxy-, Methyl-, Methoxy-, Chlor-, Brom- und Fluorgruppen; den Fluor-, Chlor-, Brom- oder Jodresten, den Nitril-, $CONH_2$- oder CONH $SO_2R''$-Resten, worin R'' für einen Alkylrest mit 1 bis 4 Kohlenstoffatomen steht, einem Phenylrest, einer Amino-, Methyl- oder Dimethylaminogruppe, einer heterocyclischen Aminogruppe, ausgewählt unter den Piperidino-, Morpholino-, Piperazino- oder 4-Ethyl-2,3-dioxo-1-piperazinoresten;

einen Cycloalkylrest mit 3 bis 8 Kohlenstoffatomen oder einen Rest

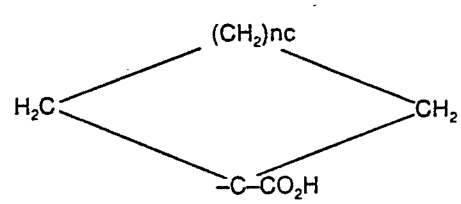

der der Salzbildung unterzogen oder verestert ist, worin nc eine ganze Zahl von 0 bis 5 darstellt,

einen Acetyl-, Propionyl- oder Benzoyl-, Carbamoyl-, Dimethylaminocarbonyl-, Phenyl- oder Benzylrest, gegebenenfalls substituiert durch Alkyl, Alkoxy oder Halogen, bedeutet;

$R_1$ ein Wasserstoffatom, einen Alkyl-, Alkenyl- oder Alkinyl- oder Thioalkylrest mit höchstens 12 Kohlenstoffatomen, gegebenenfalls substituiert durch einen oder mehrere Reste, ausgewählt unter den Azido-, Alkylthio- mit 1 bis 4 Kohlenstoffatomen oder Phenylthioresten, den gegebenenfalls der Salzbildung unterzogenen oder veresterten Carboxyresten, den Amino-, Methylamino-, Dimethylamino-, Diethylaminogruppen, dem Phenylrest, der gegebenenfalls substituiert ist durch einen oder mehrere Reste, ausgewählt unter den Hydroxy-, Halogen-, Trifluormethyl-, Amino-, Alkyl- oder Alkoxyresten mit 1 bis 4 Kohlenstoffatomen; den Fluor-, Chlor-, Brom- oder Jodresten, den Nitril-, $CONH_2$- oder CONH $SO_2R''$-Gruppen, worin R'' für einen Alkylrest mit 1 bis 4 Kohlenstoffatomen, einen Phenylrest, eine Amino-, Methyl- oder Dimethylaminogruppe, eine heterocyclische Aminogruppe, ausgewählt unter den Piperidino-, Morpholino-, Piperazino- oder 4-Ethyl-2,3-dioxo-1-piperazinoresten, steht; einem heterocyclischen Arylrest, ausgewählt unter den Thienyl-, Furyl-, Pyranyl-, Thiazolyl-, Thiadiazolyl-, Oxazolyl-, Oxadiazolyl-, Pyridinyl- oder Pyrimidinylresten; den Acetoxy-, Propionyloxy-, Benzoyloxy-, Acetylamino-, Benzylcarbonyl-, Carbamoyloxy-, Methylaminocarbonyloxy- oder Dimethylaminocarbonyloxy-, Acetyl-, Propionyl- oder Benzoylresten,

eine freie, veresterte oder der Salzbildung unterzogene Carboxygruppe,

eine Phenylgruppe, gegebenenfalls substituiert durch einen Alkyl-, Trifluormethyl-, Alkoxy-, Alkylthio-, Halogen-, Hydroxyl-, Amino-, Hydroxyalkylrest, einen heterocyclischen Rest, ausgewählt unter den Thienyl-, Furyl-, Pyrrolyl-, Thiazolyl-, Isothiazolyl-, Oxazolyl-, Isoxazolyl-, Imidazolinyl-, Imidazolyl-, Triazolyl-, Tetrazolyl-, Thiadiazolyl-, Oxadiazolyl-, Pyridinyl-, Pyrimidinyl-, Pyridazinyl-, Triazinylresten, gegebenenfalls substituiert durch einen Alkyl-, Carboxy-, Carboxyalkyl-, Aminoalkyl- oder Dialkylaminoalkylrest;

einen Acetyl-, Propionyl-, n-Butyryl- oder Benzoylrest, gegebenenfalls substituiert durch Alkoxy oder Hydroxy;

einen Carbamoyl-, Methyl- oder Dimethylcarbamoylrest;

eine Azidogruppe

darstellt;

$R_2$ einen Stickstoff enthaltenden, heterocyclischen Rest, gegebenenfalls substituiert durch einen oder mehrere Reste, ausgewählt unter den Nitro-, Carboxy-, $CF_3$-, Nitril-, Halogen-, Sulfo-, Alkylsulfo-, $(CH_2)_nSO_3H$-, $(CH_2)_nNHSO_3H$-, $(CH_2)_nSO_2NH_2$-, $(CH_2)_nCO_2H$-Gruppen, worin n für eine ganze Zahl von 1 bis 4 steht, und zumindest ein saures Wasserstoffatom enthaltend, darstellt,

X für einen Rest CH oder ein Stickstoffatom steht,

die gewellten Linien anzeigen, daß der Rest OR in der cis- oder trans-Form oder in der Form eines cis-trans-Gemisches vorliegen kann, wobei die Produkte der Formel (I) in racemischer oder optisch aktiver Form vorliegen, sowie die Salze der Produkte der Formel (I) mit Basen und Säuren.

2. Produkte gemäß Anspruch 1 der allgemeinen Formel (I')

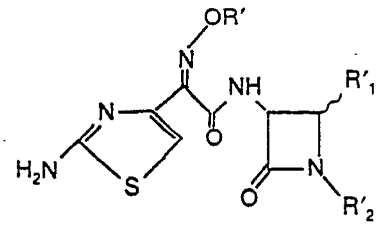

(I')

worin

R' ein Wasserstoffatom oder einen linearen oder verzweigten Alkylrest mit 1 bis 6 Kohlenstoffatomen, gegebenenfalls substituiert durch einen oder mehrere Reste, ausgewählt unter den freien, veresterten oder der Salzbildung unterzogenen Carboxygruppen, den Amino-, Mono- oder Dimethylamino-, Phenyl-, Halogen-, Nitril-, CONH-$SO_2R''$-Gruppen, worin R'' für einen Alkylrest mit 1 bis 4 Kohlenstoffatomen, Phenyl oder Amino, Mono- oder Dimethylamino steht, bedeutet oder R' einen Acetyl-, Propionyl- oder Phenylcarbonyl- oder einen Phenylrest wiedergibt oder R' einen Cycloalkylrest mit 3 bis 8 Kohlenstoffatomen oder einen Rest

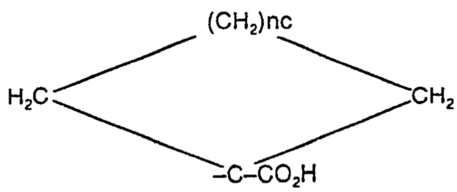

der der Salzbildung oder Veresterung unterzogen ist, worin nc eine ganze Zahl von 0 bis 5 darstellt, bedeutet;

$R'_1$ ein Wasserstoffatom oder einen Alkylrest mit höchstens 12 Kohlenstoffatomen, gegebenenfalls substituiert durch einen oder mehrere Reste, ausgewählt unter den Halogen-, Azido-, Hydroxyl-, Mercapto-, Phenyl-, Amino-, Nitril-, Alkylthio- mit 1 bis 4 Kohlenstoffatomen oder Phenylthioresten, die gegebenenfalls oxydiert sind, den Acetyl-, Propionyl-, Benzoyl-, Acetoxy-, Propionyloxy-, Benzoyloxy-, Acetylamino-, Benzylcarbonyl-, Carbamoyloxy-, Methyl- oder Dimethyl-carbamoyloxy-Resten, bedeutet, oder $R'_1$ einen Alkenyl- oder Alkinylrest mit höchstens 12 Kohlenstoffatomen, gegebenenfalls substituiert durch einen Phenylrest oder durch ein oder mehrere Halogenatome, darstellt, oder $R'_1$ für einen Thioalkylrest; gegebenenfalls substituiert durch Carbamoyl, steht, oder $R'_1$ einen Phenylrest, gegebenenfalls substituiert durch Halogen, $CF_3$, Amino, Hydroxy, Alkyl oder Alkoxy, wiedergibt, oder $R'_1$ für einen veresterten Carboxyrest, eine Carbamoylgruppe oder eine Azidogruppe steht;

$R'_2$ einen Tetrazolyl-, Triazolyl-, Imidazolyl-, Pyrazolyl-, oder Pyrrolylrest, gegebenenfalls substituiert durch einen oder mehrere Reste, ausgewählt unter den Nitro-, Carboxy-, $CF_3$-, Nitril-, Halogen-, Sulfo-, Alkylsulfo-, $(CH_2)_nSO_3H$-, $(CH_2)_nNHSO_3H$-, $(CH_2)_nSO_2NH_2$- und $(CH_2)_nCO_2H$-Gruppen, worin n für eine ganze Zahl von 1 bis 4 steht, bedeutet, wobei die Produkte die syn-Isomerie besitzen,

die gewellte Linie anzeigt, daß die Produkte sich in der cis- oder trans-Form oder in Form eines cis-trans-Gemisches befinden können, wobei die Produkte der Formel (I') in racemischer oder optisch aktiver Form vorliegen können, ebenso wie die Salze der Produkte der Formel (I') mit Basen und Säuren.

3. Produkte der allgemeinen Formel (I') gemäß Anspruch 2, worin R' für ein Wasserstoffatom, einen Methyl-, Phenyl-, Difluormethyl-, 1-Methyl-1-carboxyethyl-, Cyanomethyl-, Carboxymethyl-, (Methylsulfonyl)-carbamoylmethyl- oder einen Rest

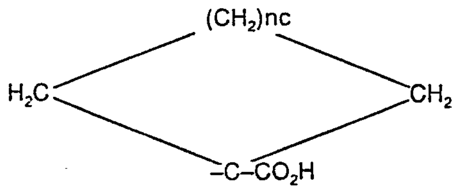

in Form des Salzes oder in veresterter Form, worin nc eine ganze Zahl von 0 bis 5 darstellt, steht,

R'₁ ein Wasserstoffatom, einen Methyl-, Fluormethyl-, Trifluormethyl-, Ethoxycarbonyl-, Carbamoylrest bedeutet;

R'₂ einen 1H-Tetrazol-5-yl oder 1,3,4-Triazol-2-yl-Rest, der gegebenenfalls durch einen Trifluormethyl- oder Carboxymethylrest substituiert ist, bedeutet.

4. Produkte der allgemeinen Formel (I') gemäß Anspruch 2, worin R' für einen Rest

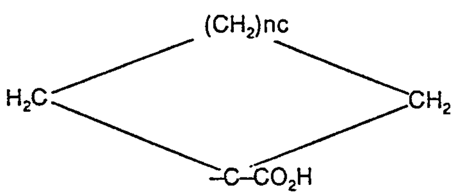

in Form des Salzes oder in veresterter Form, worin nc eine ganze Zahl von 0 bis 5 bedeutet, steht.

5. Eines der Produkte der Formel (I) mit den folgenden Bezeichnungen:

3-[[2-(2-Aminothiazol-4-yl)-2-methoxyiminoacetyl]-amino]-4-methyl-1-(1H-tetrazol-5-yl)-2-azetidinon in der cis- oder trans-, syn-isomeren, racemischen oder optisch aktiven Form;

3-[[2-(2-Aminothiazol-4-yl)-2-carboxymethoxyiminoacetyl]-amino]-4-methyl-1-(1H-tetrazol-5-yl)-2-azetidinon in cis- oder trans-, syn-isomerer, racemischer oder optisch altiver Form;

3[[2-(2-Aminothiazol-4-yl)-2-(1-carboxy-1-methyl)-ethoxyiminoacetyl]-amino]-4-methyl-1-(1H-tetrazol-5-yl)-2-azetidinon in cis- oder trans-, syn-isomerer, racemischer oder optisch aktiver Form;

3-[[2-(2-Aminothiazol-4-yl)-2-methoxyiminoacetyl]-amino]-4-fluormethyl-1-(1H-tetrazol-5-yl)-2-azetidinon in cis- oder trans-, syn-isomerer, racemischer oder optisch aktiver Form;

3-[[2-(2-Aminothiazol-4-yl)-2-fluormethoxyiminoacetyl]-amino]-4-methyl-1-(1H-tetrazol-5-yl)-2-azetidinon in cis- oder trans-, syn-isomerer, racemischer oder optisch aktiver Form.

6. Verfahren zur Herstellung der Produkte der allgemeinen Formel (I) gemäß Anspruch 1, dadurch gekennzeichnet, daß man ein Produkt der Formel (II)

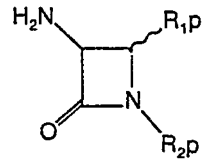
(II)

in der cis- oder trans-, racemischen oder optisch aktiven Form, worin entweder $R_1p$ die Bedeutung von $R_1$ besitzt, wobei $R_1$ die in Anspruch 1 gegebene Bedeutung hat, oder $R_1p$ den Substituenten $R_1$ darstellt, in dem die reaktiven Funktionen geschützt sind, und entweder $R_2p$ die Bedeutung von $R_2$ aufweist, wobei $R_2$ wie in Anspruch 1 definiert ist, oder $R_2p$ den Substituenten $R_2$ wiedergibt, in dem die reaktiven Funktionen geschützt sind, mit einem Produkt der Formel (III)

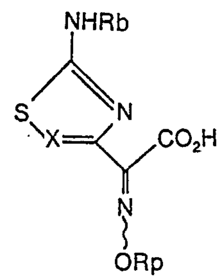
(III)

in der syn- oder anti-Form, wobei Rb ein Wasserstoffatom oder eine Schutzgruppe des Aminorestes darstellt und Rp eine Schutzgruppe für die Hydroxylgruppe wiedergibt, oder Rp die Bedeutung von R besitzt, wobei R die in Anspruch 1 angegebene Bedeutung hat, oder Rp einen Rest R wiedergibt, in dem die reaktiven Funktionen geschützt sind, behandelt, um ein Produkt der Formel (IV)

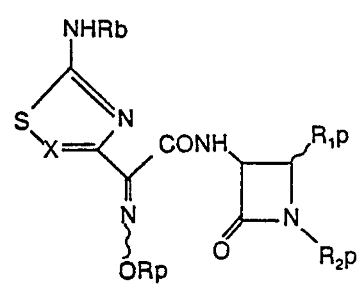
(IV)

in der syn- oder anti-, racemischen oder optisch aktiven Form zu erhalten, worin Rp, $R_1p$, $R_2p$ und Rb die vorstehende Bedeutung besitzen, welches man erforderlichenfalls und falls erwünscht irgendeiner der folgenden Reaktionen in beliebiger Reihenfolge unterzieht:

(a) Abspaltung durch Hydrolyse, Hydrogenolyse oder durch Einwirkung von Thioharnstoff der Schutzgruppe(n), die Rb und Rp darstellen können oder Rp, $R_1p$ und $R_2p$ enthalten können;

(b) Veresterung oder Salzbildung der Carboxy- oder Sulfogruppen, die die Reste Rp, $R_1p$ und $R_2p$ aufweisen können;

(c) Salzbildung mit einer Säure des Aminorestes oder der Aminoreste;

(d) Auftrennung des Moleküls, um ein optisch aktives Produkt zu erhalten.

7. Verfahren gemäß Anspruch 6 zur Herstellung der Produkte der Formel (I) gemäß Anspruch 1, dadurch gekennzeichnet, daß man für die Durchführung des Verfahrens ein Produkt der Formel (II) einsetzt, in dem $R_1p$ für ein Wasserstoffatom, einen Methyl-, Fluormethyl-, Trifluormethyl-, Ethoxycarbonyl- oder Carbamoylrest steht und $R_2p$ einen 1H-Tetrazol-5-yl- oder 1,3,4-Triazol-2-yl-Rest, der gegebenenfalls durch einen Trifluormethyl- oder Carboxymethylrest substituiert ist, bedeutet, und ein Produkt der Formel (III), in dem Rb für eine Schutzgruppe der Aminogruppe steht und Rp für eine Schutzgruppe der Hydroxylgruppe, einen Methyl-, Phenyl-, Difluormethyl-, 1-Methyl-1-carboxyethyl-, Cyanomethyl-, Carboxymethyl- oder Methylsulfonylcarbamoylmethylrest steht, einsetzt.

8. Verfahren gemäß Anspruch 6 zur Herstellung der Produkte der Formel (I), dadurch gekennzeichnet, daß die Produkte der Formel (II)

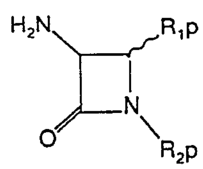
(II)

hergestellt werden, indem man ein Produkt der Formel (V)

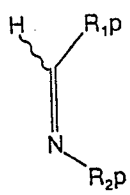
(V)

worin $R_1p$ und $R_2p$ die in Anspruch 6 gegebene Bedeutung haben, in Gegenwart einer starken Base mit einem Produkt der Formel (VI)

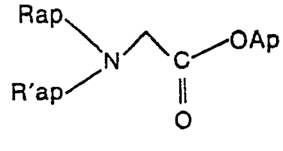
(VI)

worin Ap für ein Wasserstoffatom oder eine Estergruppe steht, Rap und R'ap derart sind, daß entweder Rap und R'ap jeweils ein Wasserstoffatom bedeuten oder einer der beiden Reste ein Wasserstoffatom und der andere eine Schutzgruppe für die Aminogruppe darstellt oder Rap und R'ap gemeinsam eine zweiwertige Schutzgruppe für die Aminogruppe bilden, umsetzt, um ein Produkt der Formel (VII)

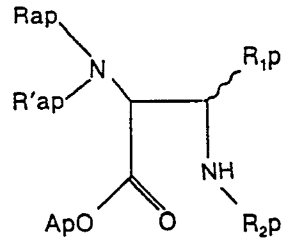
(VII)

zu erhalten, in dem Ap, $R_1p$, $R_2p$, Rap und R'ap die vorstehende Bedeutung besitzen und die gewellte Linie anzeigt, daß sich der Substituent $R_1p$ in α- oder β-Stellung befinden kann, das Produkt der Formel (VII) gewünschtenfalls der einen oder anderen der folgenden Reaktionen in beliebiger Reihenfolge unterzieht:

(a) der Trennung der beiden Isomeren;

(b) dem Schutz der $NH_2$-Gruppe, wenn Rap und R'ap jeweils ein Wasserstoffatom wiedergeben, das Produkt der Formel (VII) in Form eines einzigen Isomeren oder in Form eines Gemisches von Isomeren, wenn Ap eine Estergruppe bedeutet, einem Verseifungsmittel unterzieht, hiernach einem Mittel für die β-Lactamisierung, um ein Produkt der Formel (VIII)

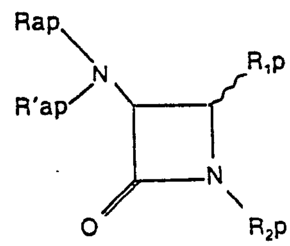
(VIII)

zu erhalten, das man erforderlichenfalls und wenn erwünscht einer der folgenden Reaktionen in beliebiger Reihenfolge unterzieht:

(a) der Trennung der Isomeren;

(b) der Abspaltung durch Hydrolyse, Hydrogenolyse oder durch Einwirken von Thioharnstoff eines der Reste Rap und R'ap oder dieser beiden Reste, wenn einer eine Schutzgruppe wiedergibt oder die beiden gemeinsam eine Schutzgruppe darstellen, um das gewünschte Produkt der Formel (II) zu erhalten.

9. Verfahren gemäß Anspruch 6 zur Herstellung von Produkten der Formel (I), dadurch gekennzeichnet, daß man die Produkte der Formel (II)

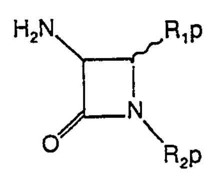
(II)

herstellt, indem man ein β-Lacton der Formel (IX)

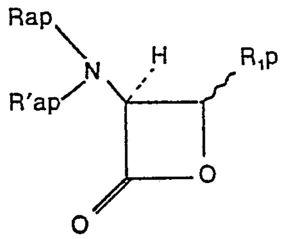

(IX)

worin $R_1p$, Rap und R'ap wie in Anspruch 8 definiert sind, mit einem Produkt der Formel (A):

$H_2N{-}R_2p$ (A)

worin $R_2p$ wie in Anspruch 8 definiert ist, umsetzt, um ein Produkt der Formel (X)

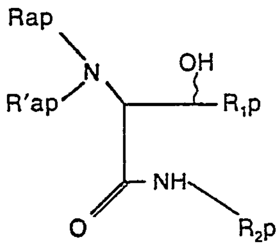

(X)

zu erhalten, das Produkt der Formel (X) gewünschtenfalls einer der folgenden Reaktionen in beliebiger Reihenfolge unterzieht:

(a) der Trennung der Isomeren, wenn $R_1p$ kein Wasserstoffatom bedeutet;

(b) dem Schutz der $NH_2$-Gruppe, wenn Rap und R'ap jeweils für ein Wasserstoffatom stehen, oder die Modifizierung der Schutzgruppe, für die einer der Reste Rap und R'ap steht oder die gemeinsam durch Rap und R'ap gebildet wird, und das Produkt der Formel (X) in Form eines Isomeren oder eines Gemisches von Isomeren einem Cyclisierungsreagens unterzieht, um zu einem Produkt der Formel (VIII)

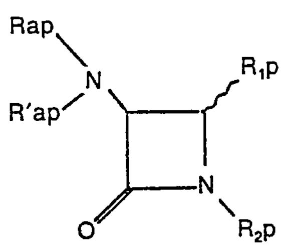

(VIII)

zu gelangen, das Produkt der Formel (VIII), das man in seine Isomeren trennen kann und das man, wenn Rap oder R'ap für eine Schutzgruppe der Aminogruppe steht oder wenn Rap und R'ap gemeinsam eine zweiwertige Schutzgruppe für die Aminogruppe wiedergeben, der Einwirkung eines Abspaltungsreagens durch Hydrolyse, Hydrogenolyse oder Einwirkung von Thioharnstoff unterzieht, um zu einem erwarteten Produkt der Formel (II) zu gelangen.

10. Verfahren gemäß Anspruch 9, dadurch gekennzeichnet, daß man zur Herstellung eines Produkts der Formel (II), in dem der Substituent $R_1p$ für ein Wasserstoffatom oder einen Methylrest steht, das Verfahren gemäß Anspruch 8 durchführt, indem man von einem Produkt der Formel (IX) ausgeht, worin $R_1p$ für ein Wasserstoffatom oder einen Methylrest steht.

11. Verfahren gemäß Anspruch 6 zur Herstellung der Produkte der Formel (I), dadurch gekennzeichnet, daß man die Produkte der Formel (II) herstellt,

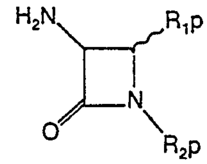

(II)

indem man ein Produkt der Formel

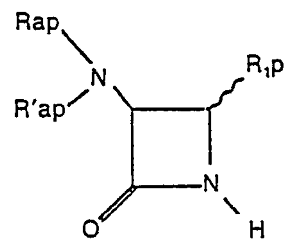

(XI)

worin $R_1p$, Rap und R'ap wie vorstehend definiert sind, mit einem Produkt der Formel

$Y{-}R_2p$ (XII)

in dem $R_2p$ wie vorstehend definiert ist und Y eine aus dem Kern austretende Gruppe wiedergibt, in Gegenwart einer Base umsetzt, um zu einem vorstehend definierten Produkt der Formel (VIII) zu gelangen, das man in seine Isomeren auftrennen kann und das man, wenn Rap und R'ap für eine Schutzgruppe der Aminogruppe steht oder wenn Rap und R'ap gemeinsam eine zweiwertige Schutzgruppe für die Aminogruppe wiedergeben, der Einwirkung eines Reagens für die Spaltung durch Hydrolyse, Hydrogenolyse oder der Einwirkung von Thioharnstoff unterzieht, um zu einem erwarteten Produkt der Formel (II) zu gelangen.

12. Verfahren gemäß Anspruch 11, dadurch gekennzeichnet, daß man die Produkte der Formel (XI) mit trans-Konfiguration herstellt, indem man eine Base mit einem Produkt der Formel ($XI_1$)

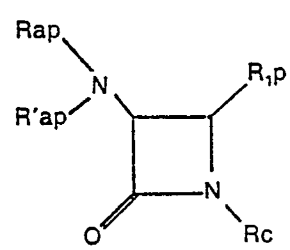

($XI_1$)

mit cis-Konfiguration, worin $R_1p$, Rap und R'ap die vorstehend angegebene Bedeutung besitzen und Rc für ein Wasserstoffatom oder eine Schutzgruppe steht, umsetzt, um ein Produkt der Formel ($XI_2$)

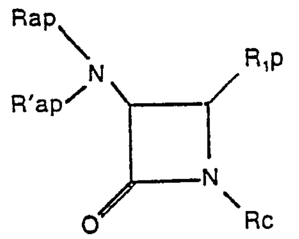

$(XI_2)$

mit trans-Konfiguration zu erhalten, in der $R_1p$, Rap, R'ap und Rc die vorstehend angegebene Bedeutung besitzen, welches man gegebenenfalls der Einwirkung eines Mittels zur Abspaltung der Schutzgruppe Rc unterzieht.

13. Als Arzneimittel die Produkte der Formel (I) gemäß Anspruch 1 und ihre pharmazeutisch verträglichen Salze.

14. Als Arzneimittel die Produkte der Formel (I') gemäß den Ansprüchen 2 bis 4 und deren pharmazeutisch verträgliche Salze.

15. Als Arzneimittel die in Anspruch 5 definierten Produkte.

16. Pharmazeutische Zusammensetzungen, enthaltend als Wirkstoff zumindest ein Arzneimittel gemäß einem der Ansprüche 13 bis 15.

17. Als neue, industrielle Produkte die Produkte der allgemeinen Formel (II)

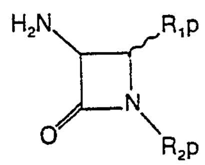

(II)

worin $R_1p$ und $R_2p$ die in Anspruch 6 angegebene Bedeutung besitzen.

18. Als neue, industrielle Produkte die Produkte der allgemeinen Formel (II) gemäß Anspruch 17, worin $R_1p$ ein Wasserstoffatom oder einen Alkylrest mit höchstens 12 Kohlenstoffatomen, der gegebenenfalls substituiert ist durch einen oder mehrere Reste, ausgewählt unter den Halogen-, Azido-, Hydroxyl-, Mercapto-, Phenyl-, Amino-, Nitril-, Alkylthio- mit 1 bis 4 Kohlenstoffatomen oder Phenylthio-, die gegebenenfalls oxidiert sind, Acetyl-, Propionyl-, Benzoyl-, Acetoxy-, Propionyloxy-, Benzoyloxy-, Acetylamino-, Benzylcarbonyl-, Carbamoyloxy-, Methyl- oder Dimethylcarbamoyloxy-Resten, bedeutet und $R_2p$ für einen gegebenenfalls geschützten Tetrazolylrest steht.

19. Als neue, industrielle Produkte die Produkte der allgemeinen Formel (II) gemäß Anspruch 17, worin $R_1p$ einen Fluormethylrest bedeutet und $R_2p$ für einen gegebenenfalls geschützten Tetrazolylrest steht.

**Patentansprüche für den Vertragsstaat: AT**

1. Verfahren zur Herstellung von Produkten der allgemeinen Formel (I)

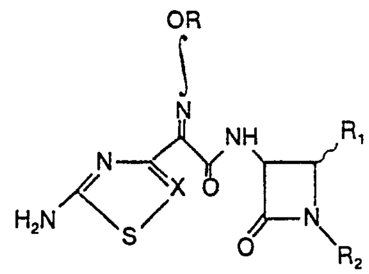

(I)

worin

R ein Wasserstoffatom, einen Alkyl-, Alkenyl- oder Alkinylrest mit höchstens 12 Kohlenstoffatomen, gegebenenfalls substituiert durch einen oder mehrere Reste, ausgewählt unter den gegebenenfalls der Salzbildung oder der Veresterung unterzogenen Carboxyresten, den Amino-, Methylamino-, Dimethylamino- und Diethylaminogruppen, dem Phenylrest, der gegebenenfalls substituiert ist durch einen oder mehrere Reste, ausgewählt unter den Hydroxy-, Methyl-, Methoxy-, Chlor-, Brom- und Fluorgruppen; den Fluor-, Chlor-, Brom- oder Jodresten, den Nitril-, $CONH_2$- oder CONH $SO_2R''$-Resten, worin R'' für einen Alkylrest mit 1 bis 4 Kohlenstoffatomen steht, einem Phenylrest, einer Amino-, Methyl- oder Dimethylaminogruppe, einer heterocyclischen Aminogruppe, ausgewählt unter den Piperidino-, Morpholino-, Piperazino- oder 4-Ethyl-2,3-dioxo-1-piperazinoresten;

einen Cycloalkylrest mit 3 bis 8 Kohlenstoffatomen oder einen Rest

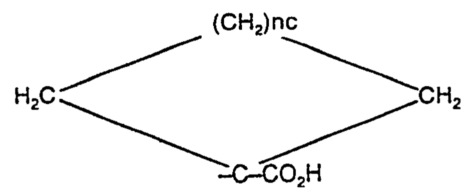

der der Salzbildung unterzogen oder verestert ist, worin nc eine ganze Zahl von 0 bis 5 darstellt,

einen Acetyl-, Propionyl- oder Benzoyl-, Carbamoyl-, Dimethylaminocarbonyl-, Phenyl- oder Benzylrest, gegebenenfalls substituiert durch Alkyl, Alkoxy oder Halogen, bedeutet;

$R_1$ ein Wasserstoffatom, einen Alkyl-, Alkenyl- oder Alkinyl- oder Thioalkylrest mit höchstens 12 Kohlenstoffatomen, gegebenenfalls substituiert durch einen oder mehrere Reste, ausgewählt unter den Azido-, Alkylthio- mit 1 bis 4 Kohlenstoffatomen oder Phenylthioresten, den gegebenenfalls der Salzbildung unterzogenen oder veresterten Carboxyresten, den Amino-, Methylamino-, Dimethylamino-, Diethylaminogruppen, dem Phenylrest, der gegebenenfalls substituiert ist durch einen oder mehrere Reste, ausgewählt unter den Hydroxy-, Halogen-, Trifluormethyl-, Amino-, Alkyl- oder Alkoxyresten mit 1 bis 4 Kohlenstoffatomen; den Fluor-, Chlor-, Brom- oder Jodresten, den Nitril-, $CONH_2$- oder CONH $SO_2R''$-Gruppen, worin R'' für einen Alkylrest mit 1 bis 4 Kohlenstoffatomen, einen Phenylrest, eine Amino-, Methyl- oder Dimethylaminogruppe, eine heterocyclische Aminogruppe, ausgewählt unter den Piperidino-, Morpholino-, Piperazino- oder 4-Ethyl-2,3-dioxo-1-piperazinoresten, steht; einem heterocyclischen Arylrest, ausgewählt unter den Thienyl-, Furyl-, Pyranyl-, Thiazolyl-, Thiadiazolyl-, Oxazolyl-, Oxadiazolyl-, Pyridinyl- oder Pyrimidinylresten; den Acetoxy-, Propionyloxy-, Benzoyloxy-, Acetylamino-, Benzylcarbonyl-, Carbamoyloxy-, Methylaminocarbonyloxy- oder Dimethylaminocarbonyloxy-, Acetyl-, Propionyl- oder Benzoylresten,

eine freie, veresterte oder der Salzbildung unterzogene Carboxygruppe,

eine Phenylgruppe, gegebenenfalls substituiert durch einen Alkyl-, Trifluormethyl-, Alkoxy-, Alkylthio-, Halogen-, Hydroxyl-, Amino-, Hydroxyalkylrest, einen heterocyclischen Rest, ausgewählt unter den Thienyl-, Furyl-, Pyrrolyl-, Thiazolyl-, Isothiazolyl-, Oxazolyl-, Isoxazolyl-, Imidazolinyl-, Imidazolyl-, Triazolyl-, Tetrazolyl-, Thiadiazolyl-, Oxadiazolyl-, Pyridinyl-, Pyrimidinyl-, Pyridazinyl-, Triazinylresten, gegebenenfalls substituiert durch einen Alkyl-, Carboxy-, Carboxyalkyl-, Aminoalkyl- oder Diaminoalkylrest;

einen Acetyl-, Propionyl-, n-Butyryl- oder Benzoylrest, gegebenenfalls substituiert durch Alkoxy oder Hydroxy;

einen Carbamoyl-, Methyl- oder Dimethylcarbamoylrest;

eine Azidogruppe

darstellt;

$R_2$ einen Stickstoff enthaltenden, heterocyclischen Rest, gegebenenfalls substituiert durch einen oder mehrere Reste, ausgewählt unter den Nitro-, Carboxy-, $CF_3$-, Nitril-, Halogen-, Sulfo-, Alkylsulfo-, $(CH_2)_nSO_3H$-, $(CH_2)_nNHSO_3H$-, $(CH_2)_nSO_2NH_2$-, $(CH_2)_nCO_2H$-Gruppen, worin n für eine ganze Zahl von 1 bis 4 steht, und zumindest ein saures Wasserstoffatom enthaltend, darstellt,

X für einen Rest CH oder ein Stickstoffatom steht,

die gewellten Linien anzeigen, daß sich der Rest OR in der syn- oder anti-Form befinden kann und daß die Produkte sich in der cis- oder trans-Form oder in der Form eines cis-trans-Gemisches befinden können, wobei die Produkte der Formel (I) in racemischer oder optisch aktiver Form vorliegen, sowie die Salze der Produkte der Formel (I) mit Basen und Säuren, dadurch gekennzeichnet, daß man ein Produkt der Formel (II)

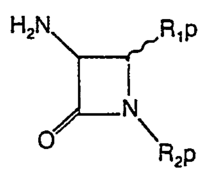

(II)

in der cis- oder trans-, racemischen oder optisch aktiven Form, worin entweder $R_1p$ die Bedeutung von $R_1$ besitzt, wobei $R_1$ die vorstehend angegebene Bedeutung aufweist, oder $R_1p$ den Substituenten $R_1$ darstellt, in dem die reaktiven Funktionen geschützt sind, und entweder $R_2p$ die Bedeutung von $R_2$ aufweist, wobei $R_2$ die vorstehend angegebene Bedeutung besitzt, oder $R_2p$ den Substituenten $R_2$ wiedergibt, in dem die reaktiven Funktionen geschützt sind, mit einem Produkt der Formel (III)

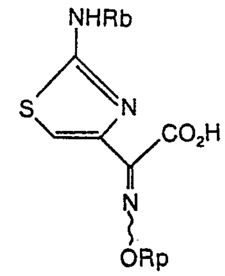

(III)

in der syn- oder anti-Form, worin Rb ein Wasserstoffatom oder eine Schutzgruppe des Aminorestes darstellt und Rp eine Schutzgruppe für die Hydroxylgruppe wiedergibt, oder Rp die Bedeutung von R besitzt, wobei R die vorstehend angegebene Bedeutung aufweist, oder Rp einen Rest R wiedergibt, in dem die reaktiven Funktionen geschützt sind, behandelt, um ein Produkt der Formel (IV)

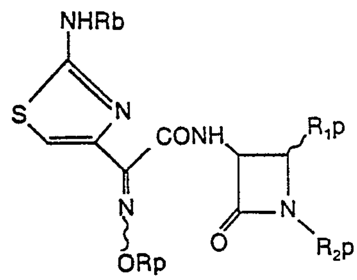

(IV)

in der syn- oder anti-, racemischen oder optisch aktiven Form zu erhalten, worin Rp, $R_1p$, $R_2p$ und Rb die vorstehende Bedeutung besitzen, welches man erforderlichenfalls und falls erwünscht irgendeiner der folgenden Reaktionen in beliebiger Reihenfolge unterzieht:

(a) Abspaltung durch Hydrolyse, Hydrogenolyse oder durch Einwirkung von Thioharnstoff der Schutzgruppe(n), die Rb und Rp darstellen können oder Rp, $R_1p$ und $R_2p$ enthalten können;

(b) Veresterung oder Salzbildung der Carboxy- oder Sulfogruppen, die die Reste Rp, $R_1p$ und $R_2p$ aufweisen können;

(c) Salzbildung mit einer Säure des Aminorestes oder der Aminoreste;

(d) Auftrennung des Moleküls, um ein optisch aktives Produkt zu erhalten.

2. Verfahren gemäß Anspruch 1 zur Herstellung von Produkten der Formel (I) gemäß Anspruch 1, entsprechend der Formel ($I_a$)

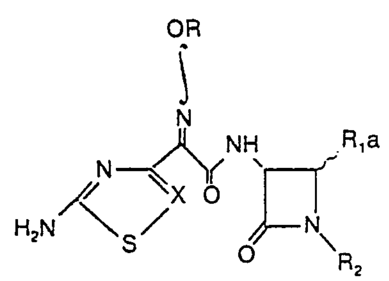

($I_a$)

worin R, $R_2$ und X die in Anspruch 1 angegebene Bedeutung besitzen und $R_1a$ die in Anspruch 1 für $R_1$ angegebene Bedeutung mit Ausnahme von Azido besitzt, dadurch gekennzeichnet, daß man von einem Produkt der Formel (II) ausgeht, worin $R_1p$ die in Anspruch 1 angegebenen Bedeutungen mit Ausnahme eines Azidorestes besitzt.

3. Verfahren gemäß Anspruch 2, dadurch gekennzeichnet, daß man von Verbindungen der Formel (II) und (III) ausgeht, die derart ausgewählt sind, daß man die Verbindungen der allgemeinen Formel (I') herstellt:

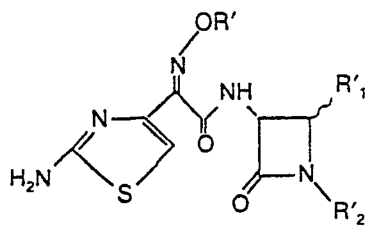

(I')

worin

R' ein Wasserstoffatom oder einen linearen oder verzweigten Alkylrest mit 1 bis 6 Kohlenstoffatomen, gegebenenfalls substituiert durch einen oder mehrere Reste, ausgewählt unter den freien, veresterten oder der Salzbildung unterzogenen Carboxygruppen, den Amino-, Mono- oder Dimethylamino-, Phenyl-, Halogen-, Nitril-, $CONHSO_2R''$-Gruppen, worin R'' für einen Alkylrest mit 1 bis 4 Kohlenstoffatomen, Phenyl oder Amino, Mono- oder Dimethylamino steht, bedeutet oder R' einen Acetyl-, Propionyl- oder Phenylcarbonyl- oder einen Phenylrest wiedergibt oder R' einen Cycloalkylrest mit 3 bis 8 Kohlenstoffatomen oder einen Rest

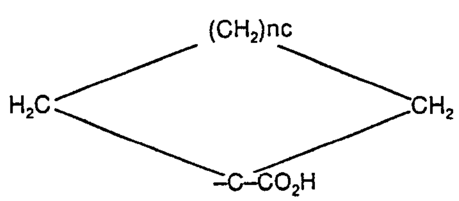

der der Salzbildung oder Veresterung unterzogen ist, worin nc eine ganze Zahl von 0 bis 5 darstellt, bedeutet;

$R'_1$ ein Wasserstoffatom oder einen Alkylrest mit höchstens 12 Kohlenstoffatomen, gegebenenfalls substituiert durch einen oder mehrere Reste, ausgewählt unter den Halogen-, Azido-, Hydroxyl-, Mercapto-, Phenyl-, Amino-, Nitril-, Alkylthio- mit 1 bis 4 Kohlenstoffatomen oder Phenylthioresten, die gegebenenfalls oxydiert sind, den Acetyl-, Propionyl-, Benzoyl-, Acetoxy-, Propionyloxy-, Benzoyloxy-, Acetylamino-, Benzylcarbonyl-, Carbamoyloxy-, Methyl- oder Dimethyl-carbamoyloxy-Resten, bedeutet, oder $R'_1$ einen Alkenyl- oder Alkinylrest mit höchstens 12 Kohlenstoffatomen, gegebenenfalls substituiert durch einen Phenylrest oder durch ein oder mehrere Halogenatome, darstellt, oder $R'_1$ für einen Thioalkylrest, gegebenenfalls substituiert durch Carbamoyl, steht, oder $R'_1$ einen Phenylrest, gegebenenfalls substituiert durch Halogen, $CF_3$, Amino, Hydroxy, Alkyl oder Alkoxy, wiedergibt, oder $R'_1$ für einen veresterten Carboxyrest, eine Carbamoylgruppe oder eine Azidogruppe steht;

$R'_2$ einen Tetrazolyl-, Triazolyl-, Imidazolyl-, Pyrazolyl- oder Pyrrolylrest, gegebenenfalls substituiert durch einen oder mehrere Reste, ausgewählt unter den Nitro-, Carboxy-, $CF_3$-, Nitril-, Halogen-, Sulfo-, Alkylsulfo-, $(CH_2)_nSO_3H$-, $(CH_2)_nNHSO_3H$-, $(CH_2)_nSO_2NH_2$- und $(CH_2)_nCO_2H$-Gruppen, worin n für eine ganze Zahl von 1 bis 4 steht, bedeutet, wobei die Produkte die syn-Isomerie besitzen,

die gewellte Linie anzeigt, daß die Produkte sich in der cis- oder trans-Form oder in Form eines cis-trans-Gemisches befinden können, wobei die Produkte der Formel (I') in racemischer oder optisch aktiver Form vorliegen,

ebenso wie die Salze der Produkte der Formel (I') mit Basen und Säuren.

4. Verfahren gemäß Anspruch 3, dadurch gekennzeichnet, daß man von Verbindungen der Formel (II) und (III) ausgeht, die derart ausgewählt sind, daß man die Verbindungen der allgemeinen Formel (I') herstellt, worin R' für ein Wasserstoffatom, einen Methyl-, Phenyl-, Difluormethyl-, 1-Methyl-1-carboxyethyl-, Cyanomethyl-, Carboxymethyl-(methylsulfonyl)-carbamoylmethylrest oder einen Rest

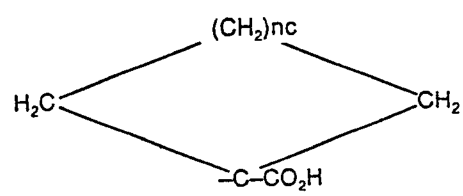

in Form des Salzes oder in veresterter Form, worin nc eine ganze Zahl von 0 bis 5 bedeutet, steht, $R'_1$ ein Wasserstoffatom, einen Methyl-, Fluormethyl-, Trifluormethyl-, Ethoxycarbonyl- oder Carbamoylrest darstellt, $R'_2$ einen 1H-Tetrazol-5-yl- oder 1,3,4-Triazol-2-yl-Rest, der gegebenenfalls durch einen Trifluormethyl- oder Carboxymethylrest substituiert ist, wiedergibt.

5. Verfahren gemäß Anspruch 2 zur Herstellung der Produkte der Formel (I) gemäß Anspruch 1, dadurch gekennzeichnet, daß man zur Durchführung des Verfahrens von einem Produkt der Formel (II), worin $R_1p$ ein Wasserstoffatom, einen Methyl-, Fluormethyl-, Trifluormethyl-, Ethoxycarbonyl- oder Carbamoylrest bedeutet und $R_2p$ für einen 1H-Tetrazol-5-yl- oder 1,3,4-Triazol-2-yl-Rest, der gegebenenfalls durch einen Trifluormethyl- oder Carboxymethylrest substituiert ist, steht, und einem Produkt der Formel (III) ausgeht, worin Rb für eine Schutzgruppe der Aminogruppe steht und Rp eine Schutzgruppe für die Hydroxylgruppe, einen Methyl-, Phenyl-, Difluormethyl-, 1-Methyl-1-carboxyethyl-, Cyanomethyl-, Carboxymethyl-(methylsulfonyl)-carbamoylmethylrest oder einen Rest

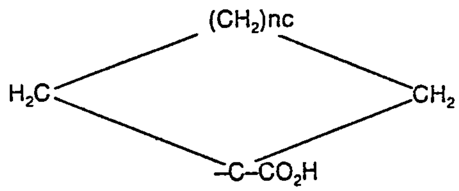

in Form des Salzes oder in veresterter Form, in dem nc eine ganze Zahl von 0 bis 5 ist, wiedergibt.

6. Verfahren gemäß Anspruch 3 zur Herstellung der Produkte der Formel (I'), worin R' einen Rest

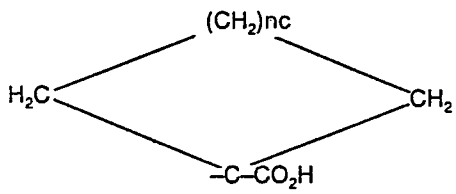

in Form des Esters oder des Salzes wiedergibt, in dem nc eine ganze Zahl von 0 bis 5 ist und $R'_1$ für einen Trifluormethylrest steht.

7. Verfahren gemäß Anspruch 1, dadurch gekennzeichnet, daß man von Verbindungen der Formel (II) und (III) ausgeht, die derart ausgewählt sind, daß man die Verbindungen der Formel (I) mit den folgenden Bezeichnungen herstellt:

3-[[2-(2-Aminothiazol-4-yl)-2-methoxyimino-acetyl]-amino]4-methyl-1-(1H-tetrazol-5-yl)-2-azetidinon in cis- oder trans-, syn-isomerer, racemischer oder optisch aktiver Form;

3-[[2-(2-Aminothiazol-4-yl)-2-carboxy-methoxyiminoacetyl]-amino]-4-methyl-1-(1H-tetrazol-5-yl)-2-azetidinon in cis- oder trans-, syn-isomerer, racemischer oder optisch aktiver Form;

3-[[2-(2-Aminothiazol-4-yl)-2-(1-carboxy-1-methyl)-ethoxyiminoacetyl]amino]-4-methyl-1-(1H-tetrazol-5-yl)-2-azetidinon in cis- oder trans-, syn-isomerer, racemischer oder optisch aktiver Form;

3-[[2-(2-Aminothiazol-4-yl)-2-methoxy-iminoacetyl]-amino]-4-fluormethyl-1-(1H-tetrazol-5-yl)-2-azetidinon in cis- oder trans-, syn-isomerer, racemischer oder optisch aktiver Form;

3-[[-2(2-Aminothiazol-4-yl)-2-fluor-methoxyiminoacetyl]-amino]-4-methyl-1-(1H-tetrazol-5-yl)-2-azetidinon in cis- oder trans-, syn-isomerer, racemischer oder optisch aktiver Form.

6. Verfahren gemäß Anspruch 1 zur Herstellung von Produkten der Formel (I), dadurch gekennzeichnet, daß die Produkte der Formel (II)

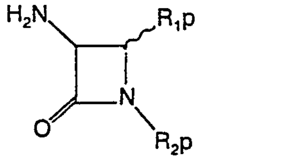

(II)

hergestellt werden, indem man ein Produkt der Formel (V)

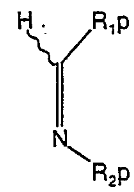

(V)

worin $R_1p$ und $R_2p$ die in Anspruch 1 gegebene Bedeutung haben, in Gegenwart einer starken Base mit einem Produkt der Formel (VI)

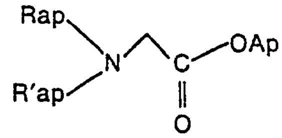

(VI)

worin Ap für ein Wasserstoffatom oder eine Estergruppe steht, Rap und R'ap derart sind, daß entweder Rap und R'ap jeweils ein Wasserstoffatom bedeuten oder einer der beiden Reste ein Wasserstoffatom und der andere eine Schutzgruppe für die Aminogruppe darstellt oder Rap und R'ap gemeinsam eine zweiwertige Schutzgruppe für die Aminogruppe bilden, umsetzt, um ein Produkt der Formel (VII)

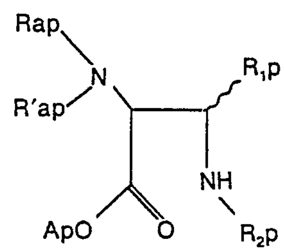

(VII)

zu erhalten, in dem Ap, $R_1p$, $R_2p$, Rap und R'ap die vorstehende Bedeutung besitzen und die gewellte Linie anzeigt, daß sich der Substituent $R_1p$ in α- oder β-Stellung befinden kann, das Produkt der Formel (VII) gewünschtenfalls der einen oder anderen der folgenden Reaktionen in beliebiger Reihenfolge unterzieht:

(a) der Trennung der beiden Isomeren;

(b) dem Schutz der $NH_2$-Gruppe, wenn Rap und R'ap jeweils ein Wasserstoffatom wiedergeben, das Produkt der Formel (VII) in Form eines einzigen Isomeren oder in Form eines Gemisches von Isomeren, wenn Ap eine Estergruppe bedeutet, einem Verseifungsmittel unterzieht, hiernach einem Mittel für die β-Lactamisierung, um ein Produkt der Formel (VIII)

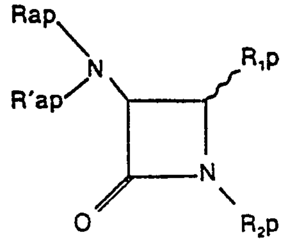

(VIII)

zu erhalten, das man erforderlichenfalls und wenn erwünscht einer der folgenden Reaktionen in beliebiger Reihenfolge unterzieht:

(a) der Trennung der Isomeren;

(b) der Abspaltung durch Hydrolyse, Hydrogenolyse oder durch Einwirken von Thioharnstoff eines der Reste Rap und R'ap oder dieser beiden Reste, wenn einer eine Schutzgruppe wiedergibt oder die beiden gemeinsam eine Schutzgruppe darstellen, um das gewünschte Produkt der Formel (II) zu erhalten.

9. Verfahren gemäß Anspruch 1 zur Herstellung der Produkte der Formel (I), dadurch gekennzeichnet, daß man die Produkte der Formel (II)

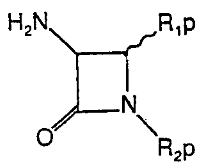
(II)

herstellt, indem man ein β-Lacton der Formel (IX)

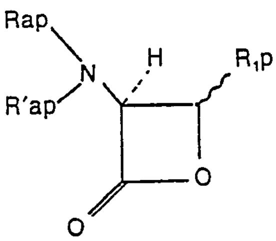
(IX)

worin $R_1p$, Rap und R'ap wie in Anspruch 7 definiert sind, mit einem Produkt der Formel (A):

$H_2N-R_2p$ (A)

worin $R_2p$ wie in Anspruch 8 definiert ist, umsetzt, um ein Produkt der Formel (X)

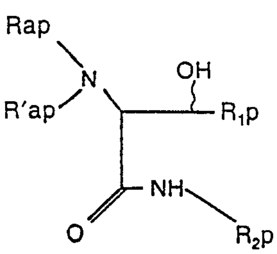
(X)

zu erhalten, das Produkt der Formel (X) gewünschtenfalls einer der folgenden Reaktionen in beliebiger Reihenfolge unterzieht:

(a) der Trennung der Isomeren, wenn $R_1p$ kein Wasserstoffatom bedeutet;

(b) dem Schutz der $NH_2$-Gruppe, wenn Rap und R'ap jeweils für ein Wasserstoffatom stehen, oder der Modifizierung der Schutzgruppe, für die einer der Reste Rap und R'ap steht oder die gemeinsam durch Rap und R'ap gebildet wird, und das Produkt der Formel (X) in Form eines isomeren oder eines Gemisches von Isomeren einem Cyclisierungsreagens unterzieht, um zu einem Produkt der Formel (VIII)

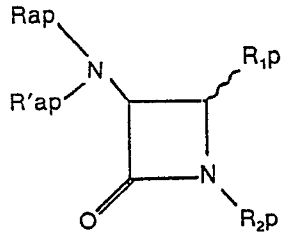
(VIII)

zu gelangen, das Produkt der Formel (VIII), das man in seine Isomeren trennen kann und das man, wenn Rap oder R'ap für eine Schutzgruppe der Aminogruppe steht oder wenn Rap und R'ap gemeinsam eine zweiwertige Schutzgruppe für die Aminogruppe wiedergeben, der Einwirkung eines Abspaltungsreagens durch Hydrolyse, Hydrogenolyse oder Einwirkung von Thioharnstoff unterzieht, um zu einem erwarteten Produkt der Formel (II) zu gelangen.

10. Verfahren gemäß Anspruch 9, dadurch gekennzeichnet, daß man zur Herstellung eines Produkts der Formel (II), worin der Substituent $R_1p$ für ein Wasserstoffatom oder einen Methylrest steht, das Verfahren gemäß Anspruch 8 durchführt, indem man von einem Produkt der Formel (IX) ausgeht, worin $R_1p$ ein Wasserstoffatom oder einen Methylrest wiedergibt.

11. Verfahren gemäß Anspruch 1 zur Herstellung der Produkte der Formel (I), dadurch gekennzeichnet, daß man die Produkte der Formel (II)

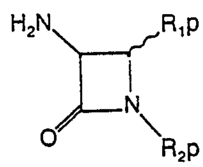
(II)

herstellt, indem man ein Produkt der Formel

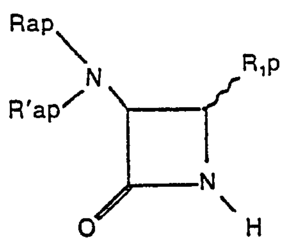
(XI)

worin $R_1p$, Rap und R'ap wie vorstehend definiert sind, mit einem Produkt der Formel

$Y-R_2p$ (XII)

in dem $R_2p$ wie vorstehend definiert ist und Y eine aus dem Kern austretende Gruppe wiedergibt, in Gegenwart einer Base umsetzt, um zu einem vorstehend definierten Produkt der Formel (VIII) zu gelangen, das man in seine Isomeren auftrennen kann und das man, wenn Rap und R'ap für eine Schutzgruppe der Aminogruppe steht oder wenn Rap und R'ap gemeinsam eine zweiwertige Schutzgruppe für die Aminogruppe wiedergeben, der Einwirkung eines Reagens für die Spaltung

durch Hydrolyse, Hydrogenolyse oder der Einwirkung von Thioharnstoff unterzieht, um zu einem erwarteten Produkt der Formel (II) zu gelangen.

12. Verfahren gemäß Anspruch 11, dadurch gekennzeichnet, daß man die Produkte der Formel (XI) mit trans-Konfiguration herstellt, indem man eine Base mit einem Produkt der Formel $(XI_1)$

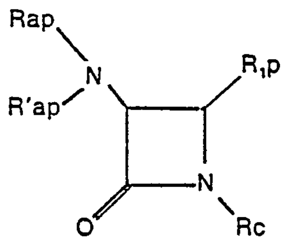

$(XI_1)$

mit cis-Konfiguration, worin $R_1p$, Rap und R'ap die vorstehend angegebene Bedeutung besitzen und Rc ein Wasserstoffatom oder eine Schutzgruppe wiedergibt, umsetzt, um ein Produkt der Formel $(XI_2)$

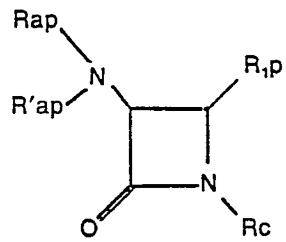

$(XI_2)$

mit trans-Konfiguration zu erhalten, in dem $R_1p$, Rap, R'ap und Rc die vorstehend angegebene Bedeutung besitzen, welches man gegebenenfalls der Einwirkung eines Mittels zur Abspaltung der Schutzgruppe Rc unterzieht.